(19) Europäisches Patentamt / European Patent Office / Office européen des brevets

(11) EP 3 915 992 A1

(12) **EUROPEAN PATENT APPLICATION**
published in accordance with Art. 153(4) EPC

(43) Date of publication:
**01.12.2021 Bulletin 2021/48**

(21) Application number: **20744765.7**

(22) Date of filing: **20.01.2020**

(51) Int Cl.:
***C07D 487/04*** *(2006.01)* ***A61K 31/4375*** *(2006.01)*
***A61P 25/18*** *(2006.01)* ***A61P 25/28*** *(2006.01)*

(86) International application number:
**PCT/CN2020/073128**

(87) International publication number:
**WO 2020/151636 (30.07.2020 Gazette 2020/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.01.2019 CN 201910063192**
**07.03.2019 CN 201910172132**
**26.03.2019 CN 201910230953**
**27.09.2019 CN 201910929917**

(71) Applicant: **Transthera Sciences (Nanjing), Inc.**
**Jiangbei New Area Nanjing**
**Jiangsu 210032 (CN)**

(72) Inventor: **WU, Frank**
**Nanjing, Jiangsu 210032 (CN)**

(74) Representative: **De Vries & Metman**
**Overschiestraat 180**
**1062 XK Amsterdam (NL)**

(54) **PDE9 INHIBITOR AND USE THEREOF**

(57) The present invention relates to the technical field of pharmaceuticals, and particularly to a PDE9 inhibitor compound of formula (I) or a pharmaceutically acceptable salt, an isomer, a deuterated compound, a metabolite or a prodrug thereof. The present invention also relates to pharmaceutical formulations, pharmaceutical compositions and use thereof. $X_1$, $X_2$, $X_3$, $X_4$, $R_1$, $R_2$, ring A, L and m are as defined in the specification. The compound of the present invention can be used in the manufacture of a medicament for treating or preventing a PDE9-mediated related disease.

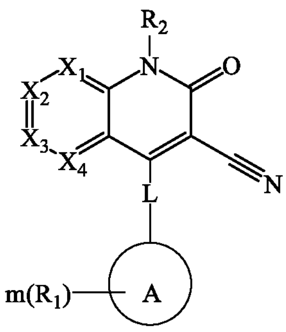

(I)

EP 3 915 992 A1

## Description

### TECHNICAL FIELD

The present invention relates to the technical field of pharmaceuticals, and particularly to a phosphodiesterase 9 inhibitor compound of formula (I) or a pharmaceutically acceptable salt, an isomer, a deuterated compound, a metabolite or a prodrug thereof, and use thereof.

### BACKGROUND

Phosphodiesterases (PDEs) are a class of proteases that selectively degrade important second messengers cGMP (cyclic guanosine monophosphate) and cAMP (cyclic adenosine monophosphate) in the body and thus participate in important physiological processes in the body. PDEs can be divided into 11 members (PDE1-PDE11) based on their sequence homology of genes and selectivity for cGMP or cAMP. Among them, PDE9A is an important member of the PDE family, and it is widely expressed in testis, brain, small intestine, skeletal muscle, heart, lung, thymus and pancreas. With the deepening research in recent years, it has been reported in several articles and proved by clinical data that PDE9A inhibitors are useful in the treatment of diseases associated with cognitive impairment caused by central nervous system disorders, such as senile dementia, schizophrenia, and neurodegenerative diseases of the brain. The two nucleotides, cAMP and cGMP, are important second messengers and play a central role in cell signaling. They mainly activate protein kinases, where the one activated by cAMP is called protein kinase A (PKA) and the one activated by cGMP is called protein kinase G (PKG). Activated PKA and PKG can phosphorylate many cellular effector proteins, such as ion channels, G-protein coupled receptors, structural proteins and transduction factors. Thus, cAMP and cGMP may control most physiological processes in many organs in this way. Meanwhile, cAMP and cGMP can also directly act on effector proteins, thereby playing the same role as described above. It is well known that cGMP can act directly on ion receptors, thereby affecting the ion concentration in cells. PDEs hydrolyze cyclic monophosphates cAMP and cGMP and thus convert them to inactive monophosphates AMP and GMP.

Human PDE9 was first cloned and sequenced in 1998 and is the PDE having the highest selectivity for cGMP reported to date. PDE9 has a binding constant (Km) of 170 nM for cGMP, while it has a binding constant of up to 230,000 nM for cAMP with a selectivity over 1000 times. Compared with PDE2A and PDE5A, PDE9 inhibitors may increase baseline cGMP concentration because PDE9 has no cGMP binding region and thus the catalytic activity of PDE9 is not enhanced by cGMP.

Conventional PDE inhibitors cannot inhibit human PDE9, and therefore the medicaments IBMX, dipyridamole, SKF94120, rolipram and vinpocetine have no inhibitory activity or low inhibitory activity against PDE9.

Currently, no PDE9 inhibitor medicament is available on the market, and only some PDE9 inhibitors are in clinical development phases, such as PF-04447943 by Pfizer (WO2008139293A1, Example 111) and BI-409306 by BI (WO2009121919 A1, Example 51).

In addition, Merck also reports compounds having PDE9-inhibiting activity in patents WO2017019723A1, WO2017019726A1 and WO2017019724A1. Compound 1-8, as described in WO2017019723A1, has the following structure:

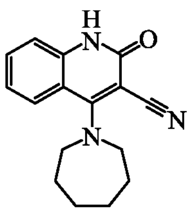

(I-8)

### SUMMARY

One purpose of the present invention is to provide a class of compounds or pharmaceutically acceptable salts, isomers or deuterated compounds thereof used as PDE9 protease inhibitors. The compounds disclosed herein have good inhibitory activity against PDE9 protease, selectivity and druggability (e.g., good pharmacokinetic properties *in vivo* and *in vitro,* higher stability in liver microsomes), can treat or prevent PDE9-mediated related diseases, and can play an important role in treating diseases associated with cognitive impairment caused by central nervous system disorders.

The technical solution of the present invention is as follows:

**A compound of general formula (I) or a pharmaceutically acceptable salt, an isomer, a deuterated compound,**

**a metabolite or a prodrug thereof:**

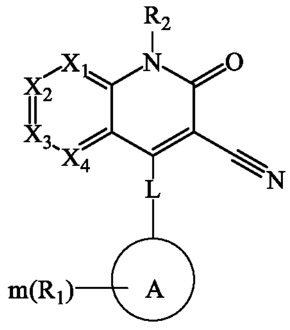

(I)

wherein $X_1$, $X_2$ and $X_4$ are each independently selected from CR' and N; $X_3$ is selected from $CR_3$ and N, and at least one of $X_1$, $X_2$, $X_3$ and $X_4$ is N, wherein the N heteroatom may be optionally oxidized to

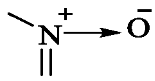

;

R' and $R_3$, at each occurrence, are each independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino, $C_{1-6}$ alkylsulfonylamino, $C_{1-6}$ alkylcarbonyloxy, $C_{3-6}$ cycloalkyl, $C_{3-6}$ cycloalkylcarbonyloxy, $C_{2-8}$ alkynyl, halogenated $C_{1-6}$ alkyl, $C_{2-8}$ alkenyl, halogenated $C_{1-6}$ alkoxy,

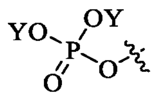

,

4-6 membered heterocyclyl unsubstituted or optionally substituted with one or more independent substituents, and heteroaryl unsubstituted or optionally substituted with one or more independent substituents;

the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-6}$ alkyl and $C_{1-6}$ alkoxy;

Y is selected from metal ions and organic ammonium ions, and is preferably $Na^+$, $K^+$ or $NH_4^+$;

L is a bond or -NH-($CH_2$)t-, wherein t is 0, 1, 2 or 3;

ring A is 3-12 membered heterocyclyl, 5-10 membered heteroaryl, 3-12 membered cycloalkyl or 3-12 membered cycloalkenyl, wherein the 3-12 membered heterocyclyl has heteroatoms selected from one of or any combinations of O, S and N, the S atom may be optionally oxidized to S(O) or S(O)2, the C atom may be optionally oxidized to C(O), the N heteroatom may be optionally oxidized to

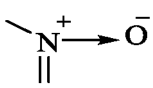

,

and the 5-10 membered heteroaryl has heteroatoms selected from one of or any combinations of O, S and N;

each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl,

$C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino and $C_{1-6}$ alkylsulfonylamino;

m is 0, 1, 2 or 3; and

$R_2$ is selected from hydrogen, $C_{1-6}$ alkyl, $C_{2-8}$ alkenyl, $C_{2-8}$ alkynyl and halogenated $C_{1-6}$ alkyl;

with the proviso that

(1) when $X_2$ is N, $X_1$ is CH, $X_3$ is $CR_3$, and $X_4$ is CH, ring A is

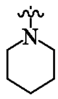 or 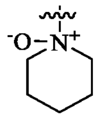 ;

(i) when

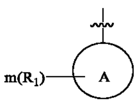 is 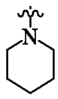 , 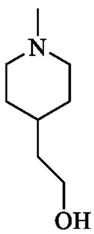 , 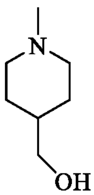 , 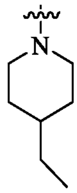 , 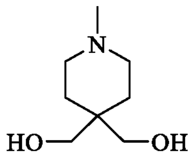 or 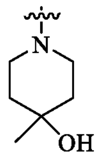 ,

$R_3$ is not H;

(ii) when

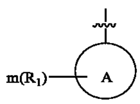 is 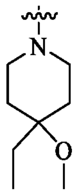 ,

$R_3$ is not Cl;

(iii) when

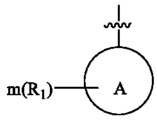 is 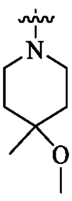 ,

$R_3$ is not H,

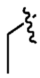, 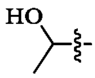, 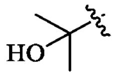, 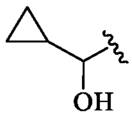,

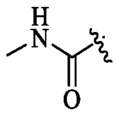, 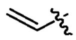, 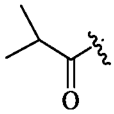, 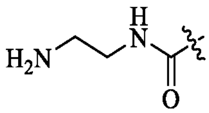, 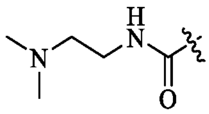, 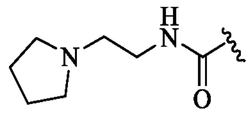,

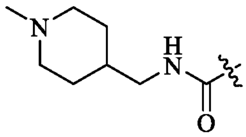, 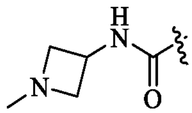, 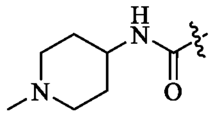, 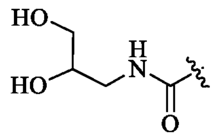, 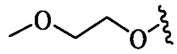,

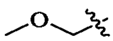, 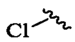, 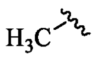, 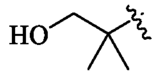 or 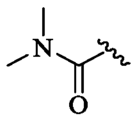;

(2) when $X_2$ and $X_4$ are each N, $X_1$ is CH, $X_3$ is $CR_3$, ring A is not methylthio;

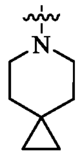,

and m is 0, $R_3$ is

(3) when $X_4$ is N, $X_1$ and $X_2$ are each CH, $X_3$ is $CR_3$, ring A is

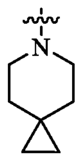 or 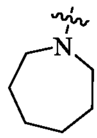,

and m is 0, $R_3$ is not hydrogen; and

(4) when $X_1$ is N, $X_2$ and $X_4$ are CR', $X_3$ is $CR_3$, ring A is pyrrolidinyl or

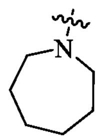,

and m is 0, $R_2$ is not H or $C_{1-6}$ alkyl.

Preferably, when $X_2$ is N, $X_1$ is CH, $X_3$ is $CR_3$, $X_4$ is CH, and

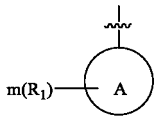 is 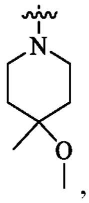,

$R_3$ is selected from isopropyl, cyclopropyl, hydroxymethyl,

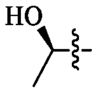, 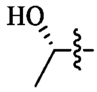,

$C_{1-6}$ alkylcarbonyl, $C_{1-6}$ alkylcarbonyloxy $C_{1-6}$ alkyl, $C_{1-6}$ alkyl substituted with

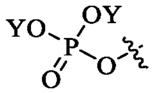,

$C_{3-6}$ cycloalkylcarbonyloxy $C_{1-6}$ alkyl and deuterated $C_{1-6}$ alkyl; - preferably, when $X_2$ is N, $X_1$ is CH, $X_3$ is $CR_3$, and $X_4$ is CH, ring A is

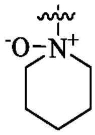.

In a preferred embodiment, $X_2$ is N, $X_1$ and $X_4$ are each independently CR', and $X_3$ is $CR_3$.

In another preferred embodiment, $X_4$ is N, $X_1$ and $X_2$ are each independently CR' and $X_3$ is $CR_3$.

In another preferred embodiment, $X_2$ and $X_4$ are each independently N, $X_1$ is CR', and $X_3$ is $CR_3$.

**Some embodiments of the present invention relate to a compound of formula (II) or a pharmaceutically acceptable salt, an isomer, a deuterated compound, a metabolite or a prodrug thereof:**

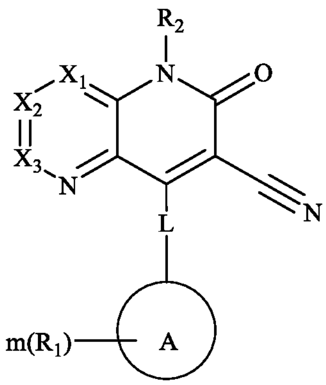

(II)

wherein, $X_1$ and $X_2$ are each independently selected from CR' and N, $X_3$ is selected from $CR_3$ and N, and the N heteroatom may be optionally oxidized to

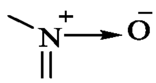;

R' and $R_3$, at each occurrence, are each independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino, $C_{1-6}$ alkylsulfonylamino, $C_{1-6}$ alkylcarbonyloxy, $C_{3-6}$ cycloalkyl, $C_{3-6}$ cycloalkylcarbonyloxy, $C_{2-8}$ alkynyl, halogenated $C_{1-6}$ alkyl, $C_{2-8}$ alkenyl, halogenated $C_{1-6}$ alkoxy, 4-6 membered heterocyclyl unsubstituted or optionally substituted with one or more independent substituents, and heteroaryl unsubstituted or optionally substituted with one or more independent substituents;

the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-6}$ alkyl and $C_{1-6}$ alkoxy;

L is a bond or -NH-($CH_2$)t-, wherein t is 0, 1, 2 or 3;

ring A is 3-12 membered heterocyclyl, 5-10 membered heteroaryl, 3-12 membered cycloalkyl or 3-12 membered cycloalkenyl, wherein the 3-12 membered heterocyclyl has heteroatoms selected from one of or any combinations of O, S and N, the S atom may be optionally oxidized to S(O) or S(O)2, the C atom may be optionally oxidized to C(O), the N heteroatom may be optionally oxidized to

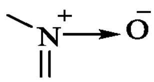,

and the 5-10 membered heteroaryl has heteroatoms selected from one of or any combinations of O, S and N;

each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, ($C_{1-6}$alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino and $C_{1-6}$ alkylsulfonylamino;

m is 0, 1, 2 or 3; and

$R_2$ is selected from hydrogen, $C_{1-6}$ alkyl, $C_{2-8}$ alkenyl, $C_{2-8}$ alkynyl and halogenated $C_{1-6}$ alkyl;

with the proviso that

(1) when $X_2$ is N, $X_1$ is CH, $X_3$ is $CR_3$, ring A is

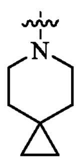,

and m is 0, $R_3$ is not methylthio; and

(2) when $X_1$ and $X_2$ are each CH, $X_3$ is $CR_3$, ring A is

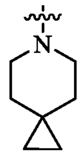 or 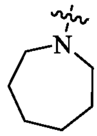,

and m is 0, $R_3$ is not hydrogen.

In another preferred embodiment, $X_1$ and $X_2$ are each independently CR', and $X_3$ is $CR_3$.

In another preferred embodiment, $X_2$ is N, $X_1$ is CR', and $X_3$ is $CR_3$.

**Some embodiments of the present invention relate to a compound of general formula (III) or a pharmaceutically acceptable salt, an isomer, a deuterated compound, a metabolite or a prodrug thereof:**

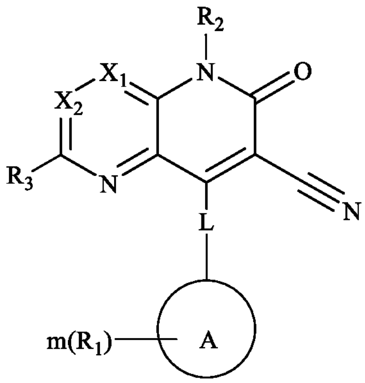

(III)

wherein $X_1$ and $X_2$ are each independently selected from CR' and N, and the N heteroatom may be optionally oxidized to

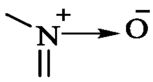;

R' and $R_3$, at each occurrence, are each independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, $(C_{1-6}$ alkyl$)_2$amino, halogenated $C_{1-6}$ alkyl, halogenated $C_{1-6}$ alkoxy, $C_{2-8}$ alkenyl, C2-8 alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, $(C_{1-6}$ alkyl$)_2$aminocarbonyl, 4-6 membered heterocyclylcarbonyl and 5-6 membered heteroaryl-oxy, wherein the $C_{1-6}$ alkyl, $C_{1-6}$ alkoxy, $C_{1-6}$ alkylamino, $(C_{1-6}$ alkyl$)_2$amino, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, $(C_{1-6}$ alkyl$)_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, $(C_{1-6}$ alkyl$)_2$amino, $C_{1-6}$ alkylcarbonylamino, $C_{1-6}$ alkylsulfonylamino, $C_{1-6}$ alkylcarbonyloxy, $C_{3-6}$ cycloalkyl, $C_{3-6}$ cycloalkylcarbonyloxy, $C_{2-8}$ alkynyl, halogenated $C_{1-6}$ alkyl, $C_{2-8}$ alkenyl, halogenated $C_{1-6}$ alkoxy, 4-6 membered heterocyclyl unsubstituted or optionally substituted with one or more independent substituents, and heteroaryl unsubstituted or optionally substituted with one or more independent substituents;

the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-6}$ alkyl and $C_{1-6}$ alkoxy;

L is a bond or $-NH-(CH_2)t-$, wherein t is 0, 1, 2 or 3;

ring A is 3-12 membered heterocyclyl, 5-10 membered heteroaryl, 3-12 membered cycloalkyl or 3-12 membered cycloalkenyl, wherein the 3-12 membered heterocyclyl has heteroatoms selected from one of or any combinations of O, S and N, the S atom may be optionally oxidized to S(O) or $S(O)_2$, the C atom may be optionally oxidized to C(O), the N heteroatom may be optionally oxidized to

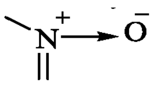

, and the 5-10 membered heteroaryl has heteroatoms selected from one of or any combinations of O, S and N; each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino and $C_{1-6}$ alkylsulfonylamino;
m is 0, 1, 2 or 3; and
$R_2$ is selected from hydrogen, $C_{1-6}$ alkyl, $C_{2-8}$ alkenyl, $C_{2-8}$ alkynyl and halogenated $C_{1-6}$ alkyl;

with the proviso that

(1) when $X_2$ is N, $X_1$ is CH, ring A is

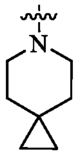

,

and m is 0, $R_3$ is not methylthio; and

(2) when $X_1$ and $X_2$ are each CH, ring A is

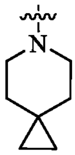

or

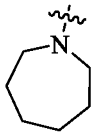

,

and m is 0, $R_3$ is not hydrogen.

In another preferred embodiment, $X_1$ and $X_2$ are each independently CR'.

In another preferred embodiment, $X_2$ is N and $X_1$ is CR'.

**Some embodiments of the present invention relate to a compound of formula (I), (II) or (III), or a pharmaceutically acceptable salt, an isomer, a deuterated compound, a metabolite or a prodrug thereof,**

wherein $X_1$ and $X_2$ are each independently selected from CR' and N, and the N heteroatom may be optionally oxidized to

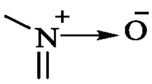

;

R' and $R_3$, at each occurrence, are each independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy, wherein the $C_{1-6}$ alkyl,

$C_{1-6}$ alkoxy, $C_{1-6}$ alkylamino, $(C_{1-6}$ alkyl$)_2$amino, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, $(C_{1-6}$ alkyl$)_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, $(C_{1-6}$ alkyl$)_2$amino, $C_{1-6}$ alkylcarbonylamino, $C_{1-6}$ alkylsulfonylamino, $C_{1-6}$ alkylcarbonyloxy, $C_{3-6}$ cycloalkyl, $C_{3-6}$ cycloalkylcarbonyloxy, $C_{2-8}$ alkynyl, halogenated $C_{1-6}$ alkyl, $C_{2-8}$ alkenyl, halogenated $C_{1-6}$ alkoxy, 4-6 membered heterocyclyl unsubstituted or optionally substituted with one or more independent substituents, and heteroaryl unsubstituted or optionally substituted with one or more independent substituents;

the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-6}$ alkyl and $C_{1-6}$ alkoxy;

L is a bond;

ring A is 4-7 membered monocyclic heterocyclyl, wherein the 4-7 membered monocyclic heterocyclyl has heteroatoms selected from one of or combinations of two of O, S and N, and contains at least one N, ring A is connected to L via the N atom, the S atom may be optionally oxidized to S(O)2, the C atom may be optionally oxidized to C(O), and the N heteroatom may be optionally oxidized to

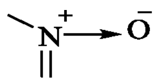 ;

preferably, ring A is 4-7-membered saturated nitrogen-containing monocyclic heterocyclyl; more preferably, ring A is

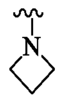, 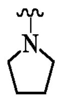, 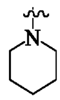, 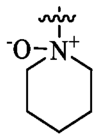, 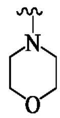, 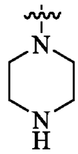, 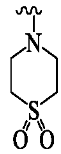 or 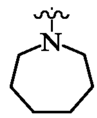 ;

and further more preferably, ring A is

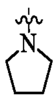, 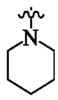, 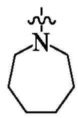 or 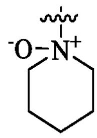 ;

each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, $(C_{1-6}$ alkyl$)_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, $(C_{1-6}$alkyl$)_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with one or more groups selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, $(C_{1-6}$ alkyl$)_2$amino, $C_{1-6}$ alkylcarbonylamino and $C_{1-6}$ alkylsulfonylamino;

$R_2$ is selected from hydrogen, $C_{1-6}$ alkyl, $C_{2-8}$ alkenyl, $C_{2-8}$ alkynyl and halogenated $C_{1-6}$ alkyl; and

m is 0, 1 or 2;

with the proviso that

when $X_1$ and $X_2$ are each CH, ring A is

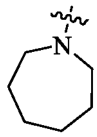,

and m is 0, $R_3$ is not hydrogen.

In another preferred embodiment, $X_1$ and $X_2$ are each independently CR'.

In another preferred embodiment, $X_2$ is N and $X_1$ is CR'.

**Some embodiments of the present invention relate to a compound of formula (I), (II) or (III), or a pharmaceutically acceptable salt, an isomer or a deuterated compound thereof,**

wherein $X_1$ and $X_2$ are each independently selected from CR' and N, and the N heteroatom may be optionally oxidized to

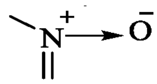;

;

R' and $R_3$, at each occurrence, are each independently selected from hydrogen, deuterium, halogen, $C_{1-4}$ alkyl, $C_{3-6}$ cycloalkyl, 5-6 membered heteroaryl, aryl, $C_{1-4}$ alkoxy, $C_{2-6}$ alkenyl, $C_{1-4}$ alkylaminocarbonyl, $C_{1-4}$ alkylcarbonyl, $(C_{1-4}$ alkyl$)_2$aminocarbonyl and aminocarbonyl, wherein the $C_{1-4}$ alkyl, $C_{3-6}$ cycloalkyl, 5-6 membered heteroaryl, aryl, $C_{1-4}$ alkoxy, $C_{2-6}$ alkenyl, $C_{1-4}$ alkylaminocarbonyl, $C_{1-4}$ alkylcarbonyl, ($C_{1-4}$ alkyl)2aminocarbonyl and aminocarbonyl are unsubstituted or optionally substituted with one or more groups independently selected from hydroxy, amino, $C_{1-4}$ alkyl, $C_{1-4}$ alkoxy, $C_{3-6}$ cycloalkyl, $C_{1-6}$ alkylcarbonyloxy, $C_{3-6}$ cycloalkylcarbonyloxy, $C_{1-4}$ alkylamino, ($C_{1-4}$ alkyl)2 amino and 4-6 membered heterocyclyl unsubstituted or substituted with $C_{1-4}$ alkyl;

L is a bond;

ring A is

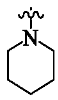 or 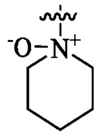;

each $R_1$ is independently selected from hydrogen, deuterium, $C_{1-4}$ alkyl and $C_{1-4}$ alkoxy;

m is 0, 1 or 2; and

$R_2$ is selected from hydrogen and $C_{1-4}$ alkyl

In another preferred embodiment, $X_1$ and $X_2$ are each independently CR'.

In another preferred embodiment, $X_2$ is N and $X_1$ is CR'.

**Some embodiments of the present invention relate to a compound of formula (I) or a pharmaceutically acceptable salt, an isomer, a deuterated compound, a metabolite or a prodrug thereof,**

wherein $X_2$ is N, $X_3$ is $CR_3$, $X_1$ and $X_4$ are each independently CR', and the N heteroatom may be optionally oxidized to

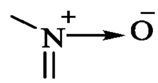;

R' is selected from hydrogen, deuterium and $C_{1-4}$ alkyl;

$R_3$ is selected from isopropyl, cyclopropyl, hydroxymethyl,

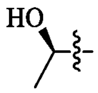, 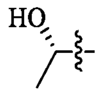,

$C_{1-6}$ alkylcarbonyl, $C_{1-6}$ alkylcarbonyloxy $C_{1-6}$ alkyl, $C_{1-6}$ alkyl substituted with

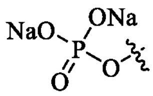

,

C3-$_6$ cycloalkylcarbonyloxy $C_{1-6}$ alkyl and deuterated $C_{1-6}$ alkyl;
L is a bond;
ring A is

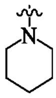 or 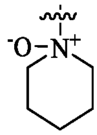 ;

each $R_1$ is independently selected from hydrogen, deuterium, $C_{1-4}$ alkyl and $C_{1-4}$ alkoxy;
$R_2$ is selected from hydrogen and $C_{1-4}$ alkyl; and
m is 0, 1 or 2.

**Some embodiments of the present invention relate to a compound of formula (I) or a pharmaceutically acceptable salt, an isomer, a deuterated compound, a metabolite or a prodrug thereof,**

wherein $X_2$ is N, $X_3$ is $CR_3$, $X_1$ and $X_4$ are each independently CR', and the N heteroatom may be optionally oxidized to

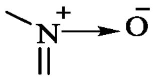

;

R' is selected from hydrogen, deuterium and $C_{1-4}$ alkyl;
$R_3$ is selected from $C_{1-4}$ alkyl; preferably, $R_3$ is selected from methyl and ethyl;
L is a bond;
ring A is

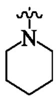 or 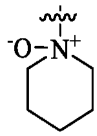 ;

each $R_1$ is independently selected from hydrogen, deuterium, hydroxy and $C_{1-4}$ alkyl;
$R_2$ is selected from hydrogen and $C_{1-4}$ alkyl; and
m is 0, 1 or 2.

**Some embodiments of the present invention relate to a compound of formula (I), (II) or (III), or a pharmaceutically acceptable salt, an isomer, a deuterated compound, a metabolite or a prodrug thereof,**

wherein $X_1$ and $X_2$ are each independently selected from CR' and N, and the N heteroatom may be optionally oxidized to

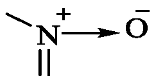

;

R' and $R_3$, at each occurrence, are each independently selected from hydrogen, deuterium, amino, carboxyl, cyano, halogen, $C_{1-6}$ alkyl, $C_{1-4}$ alkoxy, $C_{1-4}$ alkylamino, ($C_{1-4}$ alkyl)$_2$amino, $C_{2-4}$ alkenyl, $C_{2-4}$ alkynyl, $C_{1-4}$ alkylsulfonyl, $C_{1-4}$alkylthio, $C_{3-6}$ cycloalkyl, 4-6 membered nitrogen-containing heterocyclyl, 5-6 membered heteroaryl, aryl, $C_{1-4}$

alkylcarbonyl, $C_{1-4}$ alkylaminocarbonyl, ($C_{1-4}$ alkyl)$_2$aminocarbonyl and aminocarbonyl, wherein the $C_{1-4}$ alkyl, $C_{1-4}$ alkoxy, $C_{1-4}$ alkylamino, ($C_{1-4}$ alkyl)$_2$amino, $C_{2-6}$ alkenyl, $C_{2-6}$ alkynyl, $C_{1-4}$ alkylsulfonyl, $C_{1-4}$ alkylthio, $C_{3-6}$ cycloalkyl, 4-6 membered nitrogen-containing heterocyclyl, 5-6 membered heteroaryl, aryl, $C_{1-4}$ alkylcarbonyl, $C_{1-4}$ alkylaminocarbonyl, ($C_{1-4}$ alkyl)$_2$aminocarbonyl and aminocarbonyl are unsubstituted or optionally substituted with one or more groups independently selected from hydroxy, amino, cyano, halogen, $C_{1-4}$ alkyl, $C_{1-4}$ alkoxy, $C_{1-4}$ alkylamino, ($C_{1-4}$ alkyl)$_2$amino, $C_{1-4}$ alkylcarbonyloxy, $C_{3-6}$ cycloalkylcarbonyloxy, $C_{3-6}$ cycloalkyl, and 4-6 membered heterocyclyl unsubstituted or substituted with one or more independent $C_{1-4}$ alkyl groups;

L is a bond;

ring A is

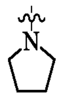;

$R_2$ is selected from hydrogen and $C_{1-4}$ alkyl; and

each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino and $C_{1-6}$ alkylsulfonylamino;

**Some embodiments of the present invention relate to a compound of formula (I), (II) or (III), or a pharmaceutically acceptable salt, an isomer, a deuterated compound, a metabolite or a prodrug thereof,**

wherein $X_1$ and $X_2$ are each independently selected from CR' and N, and the N heteroatom may be optionally oxidized to

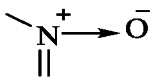;

L is a bond;

R' and $R_3$, at each occurrence, are each independently selected from hydrogen, deuterium, hydroxy, amino, carboxyl, cyano, nitro, halogen, $C_{1-4}$ alkyl, $C_{1-4}$ alkoxy, $C_{1-4}$ alkylamino, ($C_{1-4}$ alkyl)$_2$amino, halogenated $C_{1-4}$ alkyl, halogenated $C_{1-4}$ alkoxy, $C_{2-6}$ alkenyl, $C_{2-6}$ alkynyl, $C_{1-4}$ alkylsulfonyl, $C_{1-4}$ alkylthio, $C_{3-6}$ cycloalkyl, 4-6 membered heterocyclyl, 5-6 membered heteroaryl, aryl, $C_{1-4}$ alkylcarbonyl, aminocarbonyl, $C_{1-4}$ alkylaminocarbonyl, ($C_{1-4}$ alkyl)$_2$aminocarbonyl, 4-6 membered heterocyclylcarbonyl and 5-6 membered heteroaryl-oxy, wherein the $C_{1-4}$ alkyl, $C_{1-4}$ alkoxy, $C_{1-4}$ alkylamino, ($C_{1-4}$ alkyl)$_2$amino, halogenated $C_{1-4}$ alkoxy, $C_{2-6}$ alkenyl, $C_{2-6}$ alkynyl, $C_{1-4}$ alkylsulfonyl, $C_{1-4}$ alkylthio, $C_{3-6}$ cycloalkyl, 4-6 membered heterocyclyl, 5-6 membered heteroaryl, aryl, $C_{1-4}$ alkylcarbonyl, aminocarbonyl, $C_{1-4}$ alkylaminocarbonyl, ($C_{1-4}$ alkyl)$_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-4}$ alkyl, $C_{1-4}$ alkoxy, $C_{1-4}$ alkoxy $C_{1-4}$ alkoxy, $C_{1-4}$ alkylamino, ($C_{1-4}$ alkyl)$_2$amino, $C_{1-4}$ alkylcarbonylamino, $C_{1-4}$ alkylsulfonylamino, $C_{1-4}$ alkylcarbonyloxy, $C_{3-6}$ cycloalkylcarbonyloxy, $C_{3-6}$ cycloalkyl, $C_{2-6}$ alkynyl, halogenated $C_{1-4}$ alkyl, $C_{2-6}$ alkenyl, halogenated $C_{1-4}$ alkoxy, 4-6 membered heterocyclyl unsubstituted or optionally substituted with one or more independent substituents, and heteroaryl unsubstituted or optionally substituted with one or more independent substituents;

the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-4}$ alkyl and $C_{1-4}$ alkoxy;

ring A is 7-12 membered spiro-heterocyclyl, wherein the spiro-heterocyclyl has heteroatoms selected from one of or combinations of two of O, S and N, and contains at least one N, ring A is connected to L via the N atom, the S atom may be optionally oxidized to S(O)2, the C atom may be optionally oxidized to C(O), and the N heteroatom may be optionally oxidized to

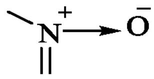 ;

preferably, the 7-12 membered spiro-heterocyclyl is 7-12 membered saturated nitrogen-containing spiro-heterocyclyl; more preferably, the 7-12 membered saturated nitrogen-containing spiro-heterocyclyl is selected from the following groups:

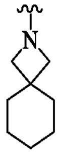, 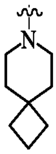, 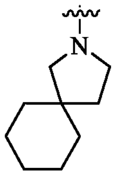, 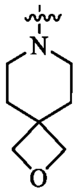, 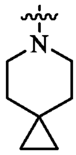, 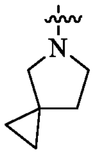 and 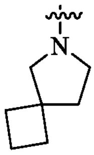;

in some embodiments, ring A is selected from

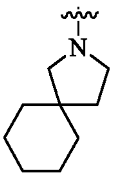, 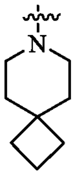, 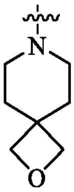, 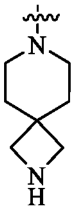,

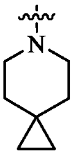, 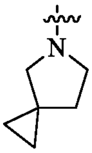, 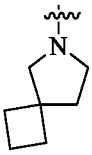, 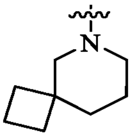, 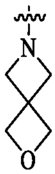, 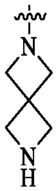 and 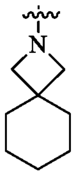;

further preferably, ring A is selected from

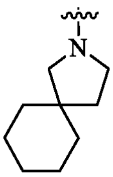, 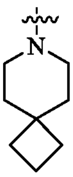, 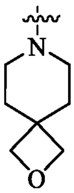, 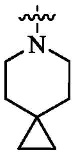, 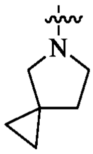,

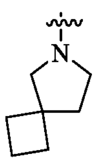 and 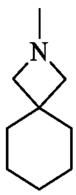;

each $R_1$ is independently selected from hydrogen, deuterium, hydroxy, amino, carboxyl, cyano, nitro, halogen, $C_{1-4}$ alkyl, $C_{1-4}$ alkoxy, $C_{1-4}$ alkylamino, $(C_{1-4}$ alkyl$)_2$amino, halogenated $C_{1-4}$ alkyl, halogenated $C_{1-4}$ alkoxy, $C_{2-6}$ alkenyl, $C_{2-6}$ alkynyl, $C_{1-4}$ alkylsulfonyl, $C_{1-4}$ alkylthio, 3-12 membered cycloalkyl, 3-12 membered cycloalkenyl, 3-12 membered heterocyclyl, aryl and 5-10 membered heteroaryl, wherein the $C_{1-4}$ alkyl, $C_{1-4}$ alkoxy, $C_{1-4}$ alkylamino, $(C_{1-4}$alkyl$)_2$amino, halogenated $C_{1-4}$ alkyl, halogenated $C_{1-4}$ alkoxy, C2-6 alkenyl, $C_{2-6}$ alkynyl, $C_{1-4}$ alkylsulfonyl,

$C_{1-4}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-4}$ alkyl, $C_{1-4}$ alkoxy, $C_{1-4}$ alkoxy $C_{1-4}$ alkoxy, $C_{1-4}$ alkylamino, $(C_{1-4}alkyl)_2$amino, $C_{1-4}$alkylcarbonylamino and $C_{1-4}$alkylsulfonylamino;
$R_2$ is selected from hydrogen, $C_{1-4}$ alkyl, $C_{2-6}$ alkenyl, $C_{2-6}$ alkynyl and halogenated $C_{1-4}$ alkyl; and
m is 0, 1 or 2;

with the proviso that

(1) when $X_2$ is N, $X_1$ is CH, ring A is

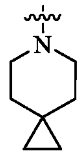,

and m is 0, $R_3$ is not methylthio; and

(2) when $X_1$ and $X_2$ are each CH, ring A is

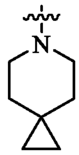,

and m is 0, $R_3$ is not hydrogen. In another preferred embodiment, $X_1$ and $X_2$ are each independently CR'.

In another preferred embodiment, $X_2$ is N and $X_1$ is CR'.
**Some embodiments of the present invention relate to a compound of formula (I), (II) or (III), or a pharmaceutically acceptable salt, an isomer, a deuterated compound, a metabolite or a prodrug thereof,** wherein,

$X_2$ and $X_4$ are each independently selected from CR' and N, $X_3$ is selected from $CR_3$ and N, and the N heteroatom may be optionally oxidized to

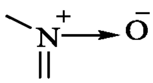;

R' and $R_3$, at each occurrence, are each independently selected from hydrogen, deuterium, cyano, amino, halogen, carboxyl, $C_{1-4}$ alkyl, $C_{1-4}$ alkoxy, $C_{2-6}$ alkenyl, $C_{1-4}$ alkylcarbonyl, $C_{2-6}$ alkynyl, $C_{1-4}$ alkylamino, $(C_{1-4}$ alkyl$)_2$amino, $C_{1-4}$ alkylaminocarbonyl, $C_{1-4}$ alkylcarbonyl, $C_{1-4}$ alkylthio, $C_{1-4}$ alkylsulfonyl, $C_{3-6}$ cycloalkyl, 4-6 membered heterocyclyl, 5-6 membered heteroaryl and aryl, wherein the $C_{1-4}$ alkyl, $C_{1-4}$ alkoxy, $C_{2-6}$ alkenyl, $C_{1-4}$ alkylcarbonyl, $C_{2-6}$ alkynyl, $C_{1-4}$ alkylamino, $(C_{1-4}$ alkyl$)_2$amino, $C_{1-4}$ alkylaminocarbonyl, $C_{1-4}$ alkylthio, $C_{1-4}$ alkylsulfonyl, $C_{3-6}$ cycloalkyl, 4-6 membered heterocyclyl, 5-6 membered heteroaryl and aryl are unsubstituted or optionally substituted with one or more groups independently selected from hydroxy, amino, cyano, halogen, $C_{1-4}$ alkyl, $C_{1-4}$ alkoxy, $C_{1-4}$ alkylamino, $(C_{1-4}$ alkyl$)_2$amino, $C_{3-6}$ cycloalkyl, $C_{1-6}$ alkylcarbonyloxy, $C_{3-6}$ cycloalkylcarbonyloxy and $C_{1-4}$ alkylcarbonyloxy;
L is a bond;
$R_2$ is selected from hydrogen and $C_{1-4}$ alkyl; and
ring A is selected from

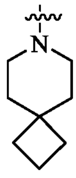, 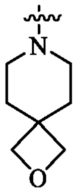 and 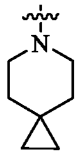 ;

m is 0, 1 or 2; and

each $R_1$ is independently selected from hydrogen, deuterium, hydroxy, amino, carboxyl, cyano, nitro, halogen, $C_{1-4}$ alkyl, $C_{1-4}$ alkoxy, $C_{1-4}$ alkylamino, $(C_{1-4}$ alkyl$)_2$amino, halogenated $C_{1-4}$ alkyl, halogenated $C_{1-4}$ alkoxy, $C_{2-6}$ alkenyl, $C_{2-6}$ alkynyl, $C_{1-4}$ alkylsulfonyl, $C_{1-4}$ alkylthio, 3-12 membered cycloalkyl, 3-12 membered cycloalkenyl, 3-12 membered heterocyclyl, aryl and 5-10 membered heteroaryl, wherein the $C_{1-4}$ alkyl, $C_{1-4}$ alkoxy, $C_{1-4}$ alkylamino, $(C_{1-4}$alkyl$)_2$amino, halogenated $C_{1-4}$ alkyl, halogenated $C_{1-4}$ alkoxy, C2-6 alkenyl, $C_{2-6}$ alkynyl, $C_{1-4}$ alkylsulfonyl, $C_{1-4}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-4}$ alkyl, $C_{1-4}$ alkoxy, $C_{1-4}$ alkoxy $C_{1-4}$ alkoxy, $C_{1-4}$ alkylamino, $(C_{1-4}$ alkyl$)_2$amino, $C_{1-4}$ alkylcarbonylamino and $C_{1-4}$ alkylsulfonylamino;

with the proviso that

(1) when $X_2$ is N, $X_1$ is CH, ring A is

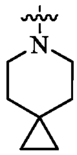 ,

and m is 0, $R_3$ is not methylthio; and

(2) when $X_1$ and $X_2$ are each CH, ring A is

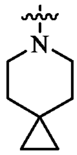 ,

and m is 0, $R_3$ is not hydrogen.

In another preferred embodiment, $X_1$ and $X_2$ are each independently CR'.

In another preferred embodiment, $X_2$ is N and $X_1$ is CR'.

**Some embodiments of the present invention relate to a compound of formula (I) or a pharmaceutically acceptable salt, an isomer, a deuterated compound, a metabolite or a prodrug thereof,** wherein,

$X_1$, $X_2$ and $X_4$ are each independently selected from CR' and N; $X_3$ is selected from $CR_3$ and N, and at least one of $X_1$, $X_2$, $X_3$ and $X_4$ is N, wherein the N heteroatom may be optionally oxidized to

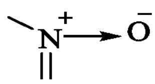 ;

R' and $R_3$, at each occurrence, are each independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, $(C_{1-6}$alkyl$)_2$amino, halogenated $C_{1-6}$ alkyl, halogenated $C_{1-6}$ alkoxy, $C_{2-8}$ alkenyl, C2-8 alkynyl, $C_{1-6}$ alkylsulfonyl, $C_{1-6}$ alkylthio, $C_{3-6}$ cycloalkyl, 4-6 membered heterocyclyl,

5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino, $C_{1-6}$ alkylsulfonylamino, $C_{1-6}$ alkylcarbonyloxy, $C_{3-6}$ cycloalkyl, $C_{3-6}$ cycloalkylcarbonyloxy, $C_{2-8}$ alkynyl, halogenated $C_{1-6}$ alkyl,

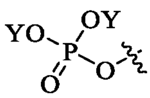,

C2-8 alkenyl, halogenated $C_{1-6}$ alkoxy, 4-6 membered heterocyclyl unsubstituted or optionally substituted with one or more independent substituents, and heteroaryl unsubstituted or optionally substituted with one or more independent substituents;

the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-6}$ alkyl and $C_{1-6}$ alkoxy;

Y is selected from metal ions and organic ammonium ions, and is preferably $Na^+$, $K^+$ or $NH_4^+$;

L is a bond or -NH-($CH_2$)t-, wherein t is 0, 1, 2 or 3;

each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyL cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino and $C_{1-6}$ alkylsulfonylamino;

$R_2$ is selected from hydrogen, $C_{1-6}$ alkyl, $C_{2-8}$ alkenyl, $C_{2-8}$ alkynyl and halogenated $C_{1-6}$ alkyl;

m is 0, 1 or 2; and

ring A is selected from the following groups:

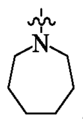, 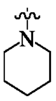, 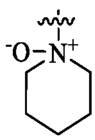, 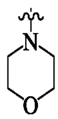, 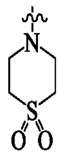, 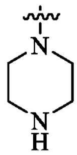, 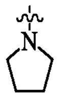, 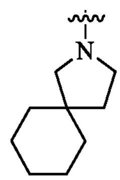, 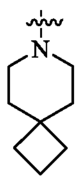, 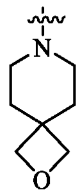, 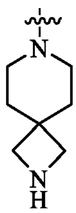,

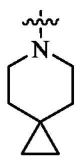, 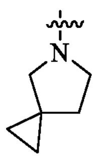, 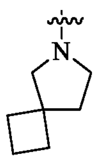, 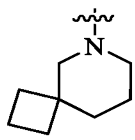, 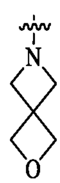, 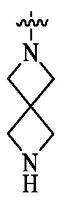 and 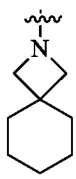;

preferably, ring A is selected from

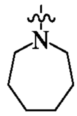, 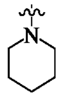, 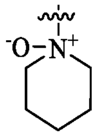, 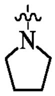, 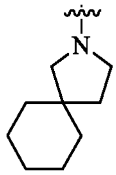, 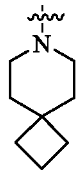,

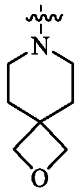, 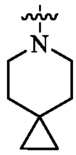, 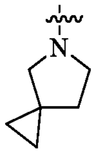, 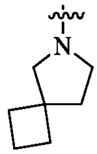, and 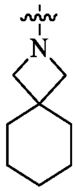;

with the proviso that

(1) when $X_2$ is N, $X_1$ is CH, $X_3$ is $CR_3$, and $X_4$ is CH, ring A is

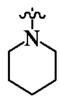;

(i) when $R_1$ is independently methyl and methoxy, $R_3$ is selected from isopropyl, cyclopropyl, hydroxymethyl,

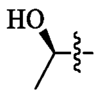 and 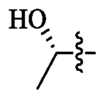;

and

(ii) when $R_1$ is independently hydrogen, methyl and hydroxy, $R_3$ is not H;

(2) when $X_2$ and $X_4$ are each N, $X_1$ is CH, $X_3$ is $CR_3$, ring A is

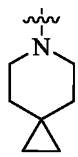,

and m is 0, $R_3$ is not methylthio;

(3) when $X_4$ is N, $X_1$ and $X_2$ are each CH, $X_3$ is $CR_3$, ring A is

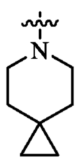 or 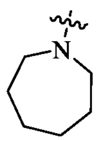,

and m is 0, $R_3$ is not hydrogen; and

(4) when $X_1$ is N, $X_2$ and $X_4$ are CR', $X_3$ is $CR_3$, ring A is pyrrolidinyl, and m is 0, $R_2$ is not $C_{1-6}$ alkyl

In a preferred embodiment, $X_2$ is N, $X_1$ and $X_4$ are each independently CR', and $X_3$ is $CR_3$.

In another preferred embodiment, $X_4$ is N, $X_1$ and $X_2$ are each independently CR' and $X_3$ is $CR_3$.

In another preferred embodiment, $X_2$ and $X_4$ are each independently N, $X_1$ is CR', and $X_3$ is $CR_3$.

**Some embodiments of the present invention relate to a compound of formula (IV) or a pharmaceutically acceptable salt, an isomer, a deuterated compound, a metabolite or a prodrug thereof:**

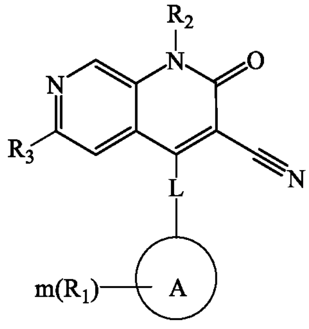

(IV)

wherein, N at position $X_2$ may be oxidized to

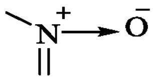

;

$R_3$ is selected from hydrogen, deuterium, amino, carboxyl, cyano, halogen, $C_{1-6}$ alkyl, $C_{1-4}$ alkoxy, $C_{1-4}$ alkylamino, ($C_{1-4}$ alkyl)$_2$amino, $C_{2-6}$ alkenyl, $C_{2-6}$ alkynyl, $C_{1-4}$ alkylsulfonyl, $C_{1-4}$ alkylthio, $C_{3-6}$ cycloalkyl, 4-6 membered nitrogen-containing heterocyclyl, 5-6 membered heteroaryl, aryl, $C_{1-4}$ alkylcarbonyl, $C_{1-4}$ alkylaminocarbonyl, ($C_{1-4}$ alkyl)$_2$aminocarbonyl and aminocarbonyl, wherein the $C_{1-4}$ alkyl, $C_{1-4}$ alkoxy, $C_{1-4}$ alkylamino, ($C_{1-4}$ alkyl)$_2$amino, $C_{2-4}$ alkenyl, $C_{2-4}$ alkynyl, $C_{1-4}$ alkylsulfonyl, $C_{1-4}$ alkylthio, $C_{3-6}$ cycloalkyl, 4-6 membered nitrogen-containing heterocyclyl, 5-6 membered heteroaryl, aryl, $C_{1-4}$ alkylcarbonyl, $C_{1-4}$ alkylaminocarbonyl, ($C_{1-4}$ alkyl)$_2$aminocarbonyl and aminocarbonyl are unsubstituted or optionally substituted with one or more groups independently selected from hydroxy, amino, cyano, halogen, $C_{1-4}$ alkyl, $C_{1-4}$ alkoxy, $C_{1-4}$ alkylamino, ($C_{1-4}$ alkyl)$_2$amino, $C_{1-4}$ alkylcarbonyloxy,

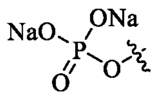

,

$C_{3-6}$ cycloalkylcarbonyloxy, $C_{3-6}$ cycloalkyl, and 4-6 membered heterocyclyl unsubstituted or substituted with one or more independent $C_{1-4}$ alkyl groups;

L is a bond or -NH-($CH_2$)t-, wherein t is 0, 1, 2 or 3;

ring A is

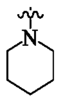

or

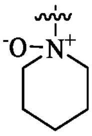

;

m is 0, 1 or 2;

each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, C2-8 alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino and $C_{1-6}$ alkylsulfonylamino; and

$R_2$ is selected from hydrogen, $C_{1-6}$ alkyl, $C_{2-8}$ alkenyl, $C_{2-8}$ alkynyl and halogenated $C_{1-6}$ alkyl;

with the proviso that

(i) when

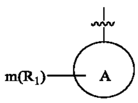 is 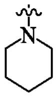, 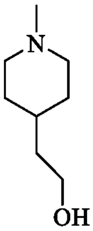, 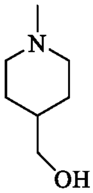, 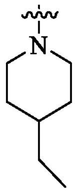, 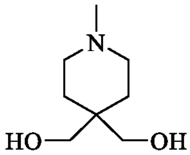 or 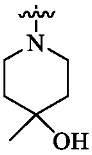,

$R_3$ is not H;

(ii) when

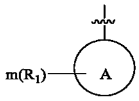 is 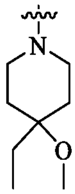,

$R_3$ is not Cl;

(iii) when

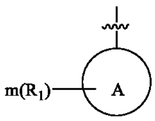 is 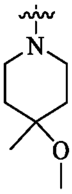, $R_3$ is not H, 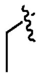, 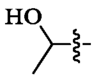, 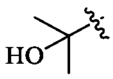, 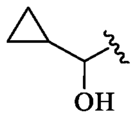,

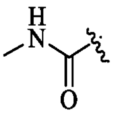, 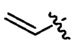, 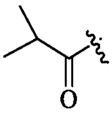, 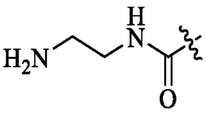, 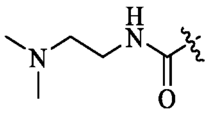, 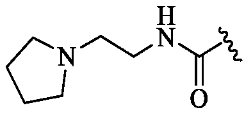,

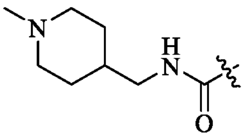, 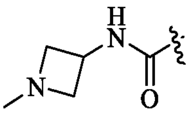, 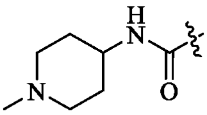, 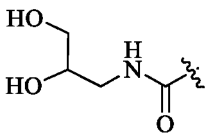, 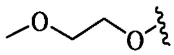,

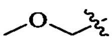, 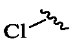, 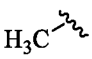, 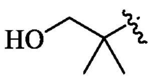 or 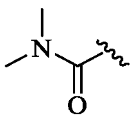;

preferably, when

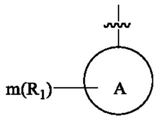 is 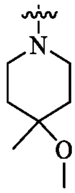,

$R_3$ is selected from isopropyl, cyclopropyl, hydroxymethyl,

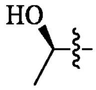, 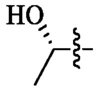,

, , $C_{1-6}$ alkylcarbonyl, $C_{1-6}$ alkylcarbonyloxy $C_{1-6}$ alkyl, $C_{1-6}$ alkyl substituted with

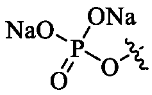,

, $C_{3-6}$ cycloalkylcarbonyloxy $C_{1-6}$ alkyl and deuterated $C_{1-6}$ alkyl; and

preferably, ring A is

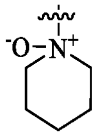.

**Some embodiments of the present invention relate to a compound of formula (I) or a pharmaceutically acceptable salt, an isomer, a deuterated compound, a metabolite or a prodrug thereof,**
wherein,

$X_1$, $X_2$ and $X_4$ are each independently selected from CR' and N; $X_3$ is selected from $CR_3$ and N, and at least one of $X_1$, $X_2$, $X_3$ and $X_4$ is N, wherein the N heteroatom may be optionally oxidized to

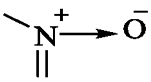;

R' and $R_3$, at each occurrence, are each independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, C2-8 alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino, $C_{1-6}$ alkylsulfonylamino, $C_{1-6}$ alkylcarbonyloxy, $C_{3-6}$ cycloalkyl, $C_{3-6}$ cycloalkylcarbonyloxy, $C_{2-8}$ alkynyl, halogenated $C_{1-6}$ alkyl, $C_{2-8}$ alkenyl, halogenated $C_{1-6}$ alkoxy, 4-6 membered heterocyclyl unsubstituted or optionally substituted with one or more independent substituents, and heteroaryl unsubstituted or optionally substituted with one or more independent substituents;
the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-6}$ alkyl and $C_{1-6}$ alkoxy; L is a bond or -NH-$(CH_2)$t-, wherein t is 0, 1, 2 or 3;

ring A is 5-10 membered heteroaryl, wherein the 5-10 membered heteroaryl has heteroatoms selected from one of or any combinations of O, S and N;

each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, $(C_{1-6}$ alkyl$)_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, $(C_{1-6}$ alkyl$)_2$amino, halogenated $C_{1-6}$ alkyl, halogenated $C_{1-6}$ alkoxy, C2-8 alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, $(C_{1-6}$ alkylamino, $C_{1-6}$ alkylcarbonylamino and $C_{1-6}$ alkylsulfonylamino;

m is 0, 1, 2 or 3;

$R_2$ is selected from hydrogen, $C_{1-6}$ alkyl, $C_{2-8}$ alkenyl, $C_{2-8}$ alkynyl and halogenated $C_{1-6}$ alkyl;

more preferably, ring A is 9-10 membered nitrogen-containing heteroaryl; and

most preferably, ring A is selected from

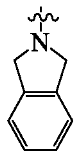 and 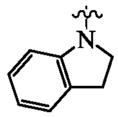 .

**In one embodiment of the present invention, the isomer of the compound of general formula (I), (II), (III) or (IV) refers to a stereoisomer or a tautomer.**

**In one embodiment of the present invention, when $R_2$ in the structure shown as the general formula (I) is hydrogen, the tautomer of general formula (I') is provided:**

the tautomer of

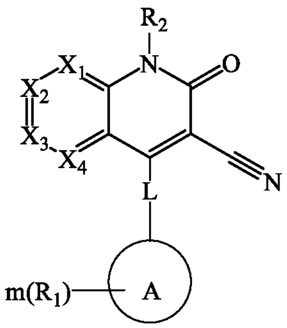 (I) is 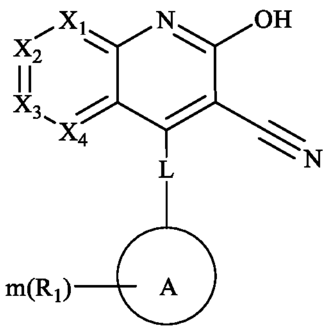 (I').

In one embodiment of the present invention, compounds of the aforementioned formulas (I), (II), (III) and (IV), and pharmaceutically acceptable salts, isomers, deuterated compounds, metabolites and prodrugs thereof are shown in Table 1:

Table 1

| Serial number | Structure | Serial number | Structure |
|---|---|---|---|
| 1 | 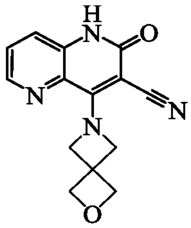 | 2 | 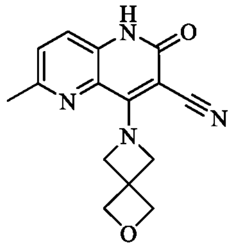 |
| 3 | 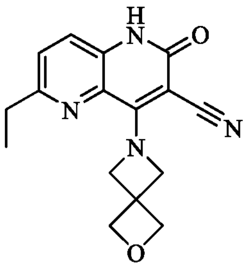 | 4 | 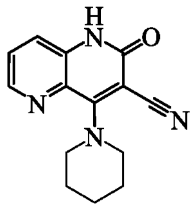 |
| 5 | 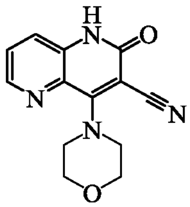 | 6 | 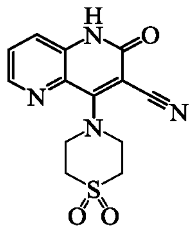 |
| 7 | 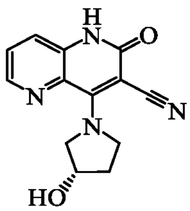 | 8 | 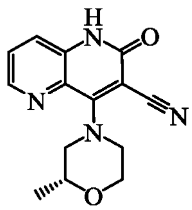 |
| 9 | 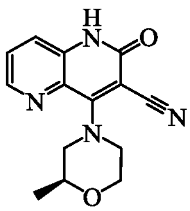 | 10 | 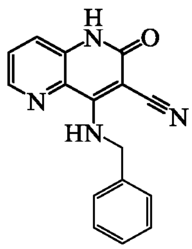 |
| 11 | 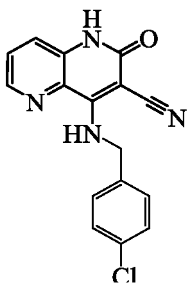 | 12 | 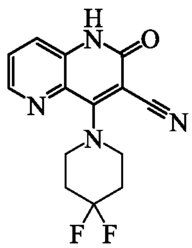 |

(continued)

| Serial number | Structure | Serial number | Structure |
|---|---|---|---|
| 13 | 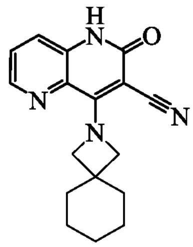 | 14 | 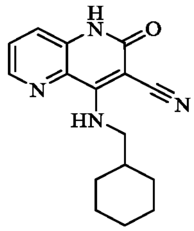 |
| 15 | 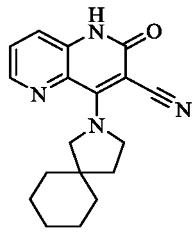 | 16 | 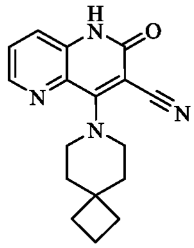 |
| 17 | 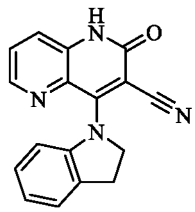 | 18 | 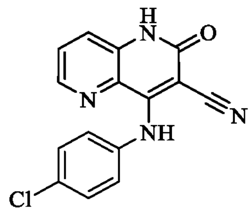 |
| 19 | 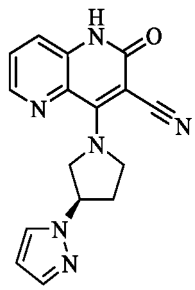 | 20 | 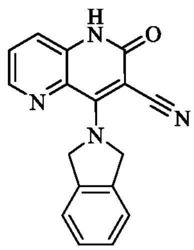 |
| 21 | 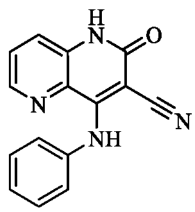 | 22 | 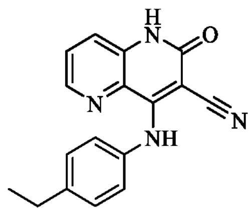 |
| 23 | 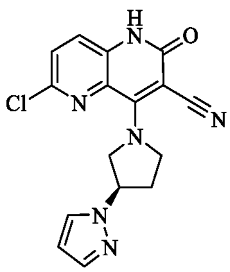 | 24 | 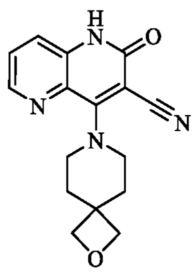 |

(continued)

| Serial number | Structure | Serial number | Structure |
|---|---|---|---|
| 25 | 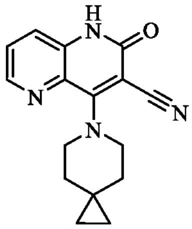 | 26 | 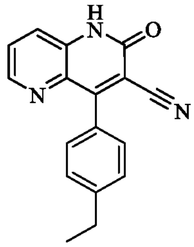 |
| 27 | 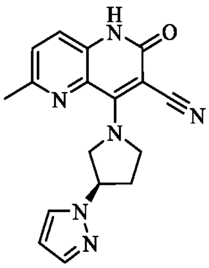 | 28 | 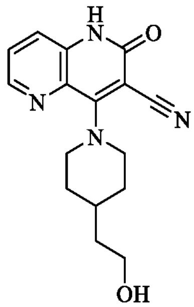 |
| 29 | 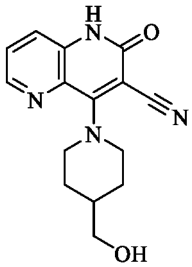 | 30 | 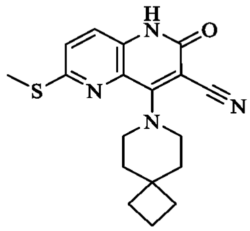 |
| 31 | 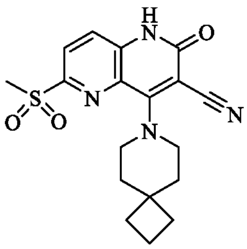 | 32 | 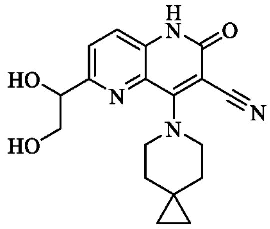 |
| 33 | 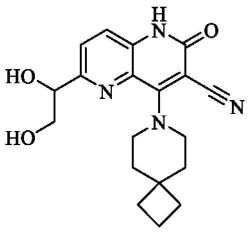 | 34 | 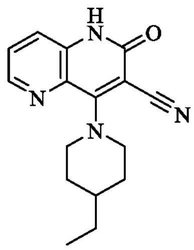 |
| 35 | 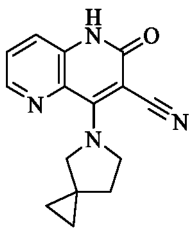 | 36 | 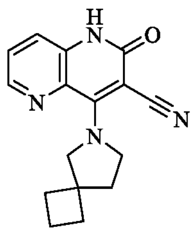 |

(continued)

| Serial number | Structure | Serial number | Structure |
|---|---|---|---|
| 37 | 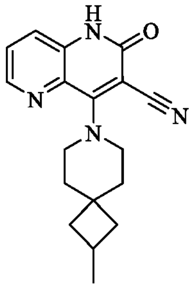 | 38 | 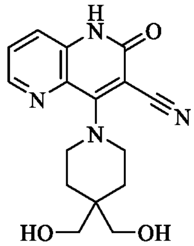 |
| 39 | 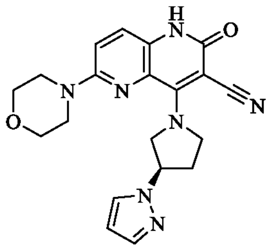 | 40 | 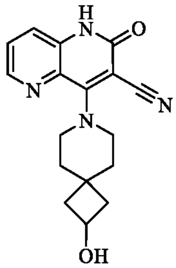 |
| 41 | 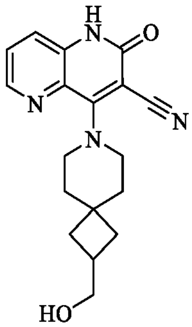 | 42 | 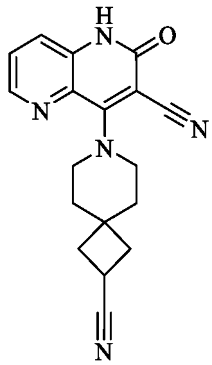 |
| 43 | 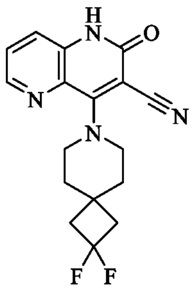 | 44 | 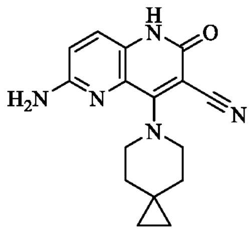 |
| 45 | 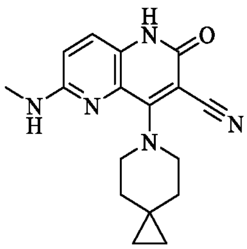 | 46 | 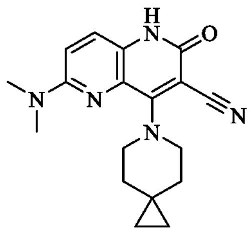 |

(continued)

| Serial number | Structure | Serial number | Structure |
|---|---|---|---|
| 47 | 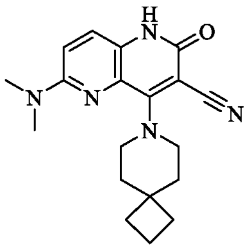 | 48 | 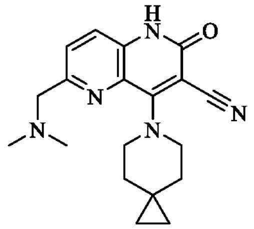 |
| 49 | 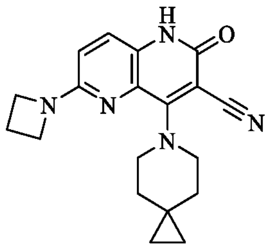 | 50 | 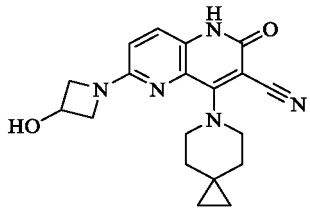 |
| 51 | 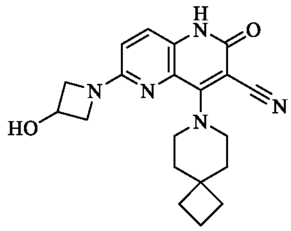 | 52 | 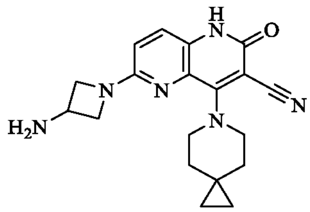 |
| 53 | 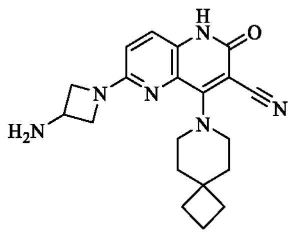 | 54 | 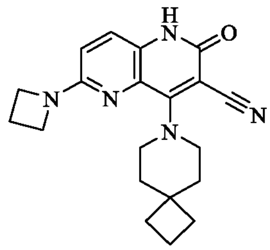 |
| 55 | 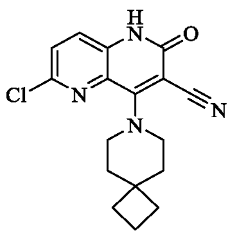 | 56 | 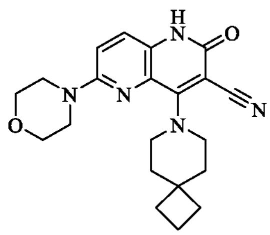 |
| 57 | 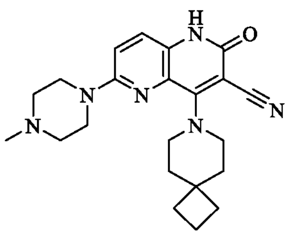 | 58 | 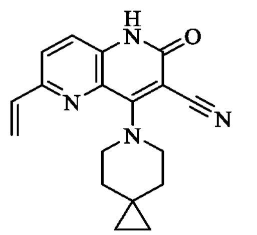 |

(continued)

| Serial number | Structure | Serial number | Structure |
|---|---|---|---|
| 59 | 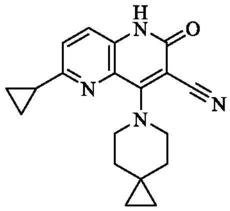 | 60 | 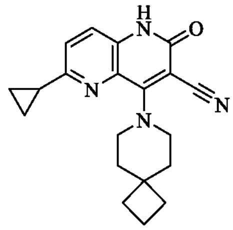 |
| 61 | 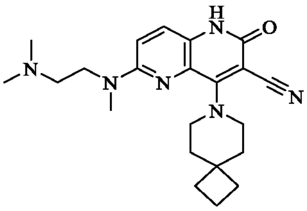 | 62 | 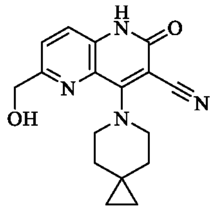 |
| 63 | 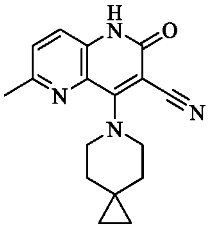 | 64 | 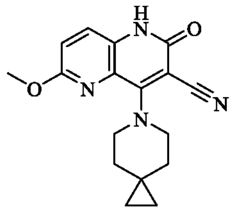 |
| 65 | 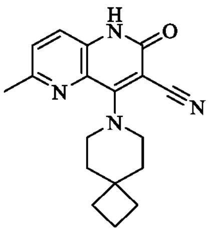 | 66 | 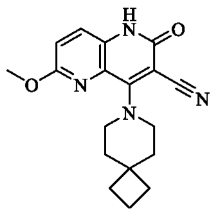 |
| 67 | 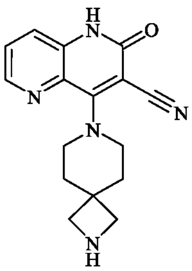 | 68 | 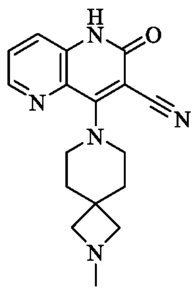 |
| 69 | 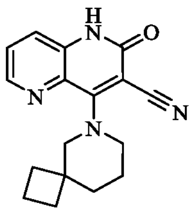 | 70 | 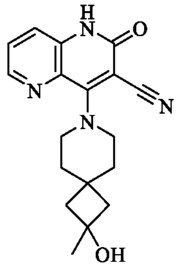 |

(continued)

| Serial number | Structure | Serial number | Structure |
|---|---|---|---|
| 71 | 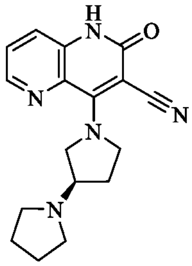 | 72 | 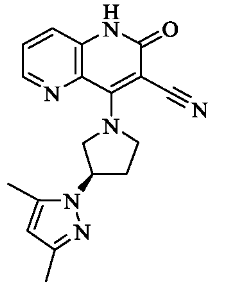 |
| 73 | 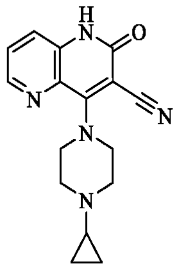 | 74 | 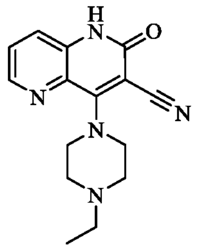 |
| 75 | 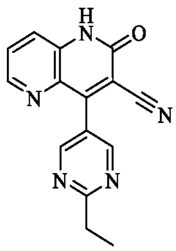 | 76 | 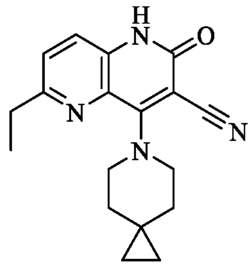 |
| 77 | 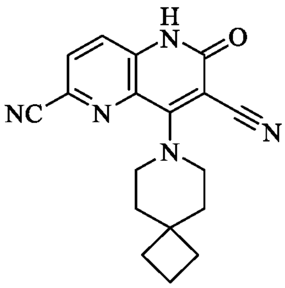 | 78 | 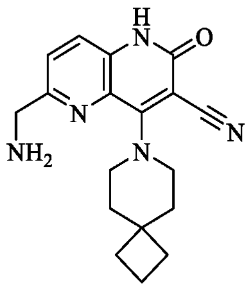 |
| 79 | 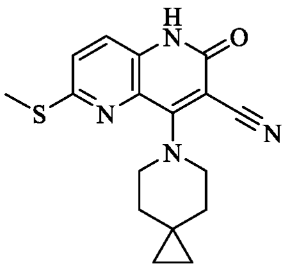 | 80 | 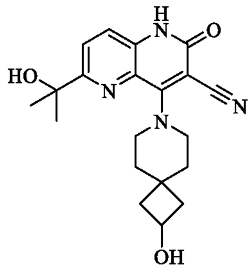 |

(continued)

| Serial number | Structure | Serial number | Structure |
|---|---|---|---|
| 81 | 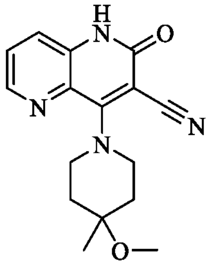 | 82 | 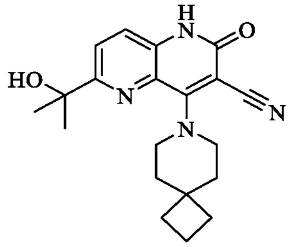 |
| 83 | 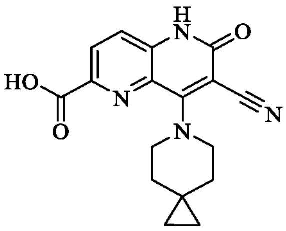 | 84 | 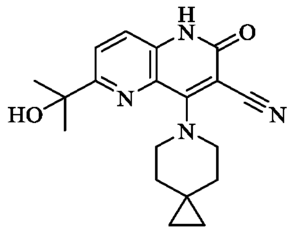 |
| 85 | 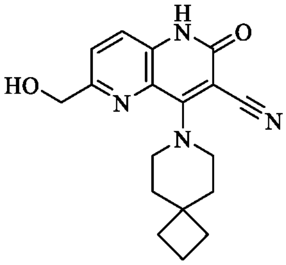 | 86 | 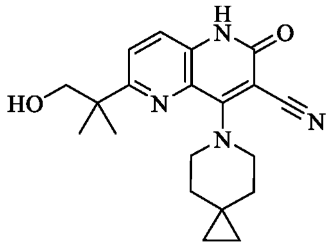 |
| 87 | 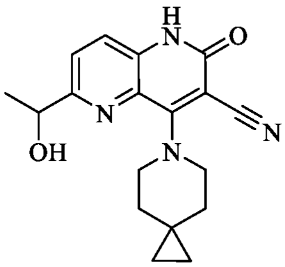 | 88 | 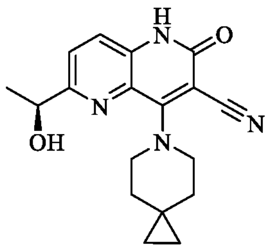 |
| 89 | 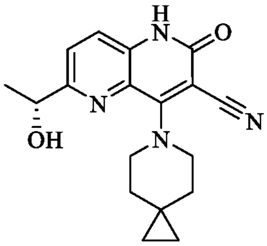 | 90 | 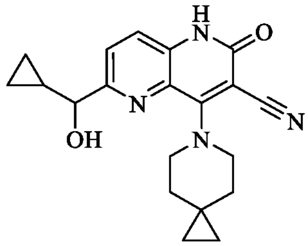 |
| 91 | 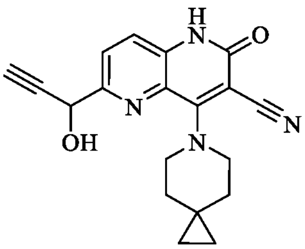 | 92 | 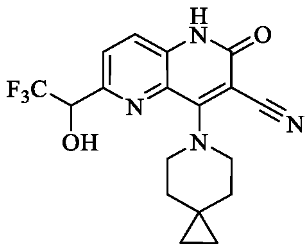 |

(continued)

| Serial number | Structure | Serial number | Structure |
|---|---|---|---|
| 93 | 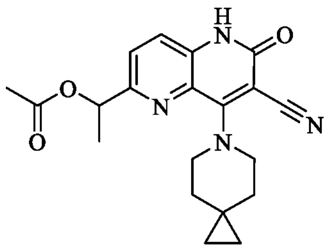 | 94 | 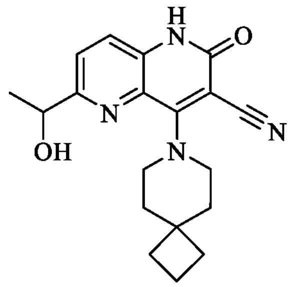 |
| 95 | 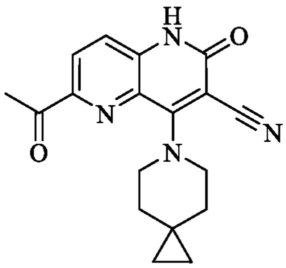 | 96 | 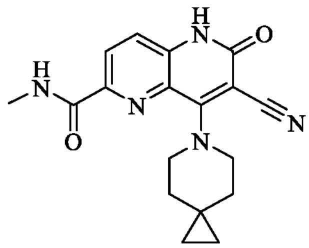 |
| 97 | 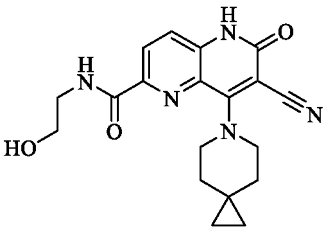 | 98 | 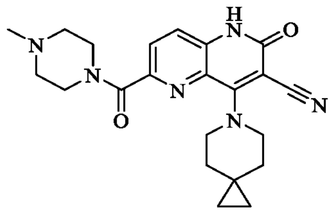 |
| 99 | 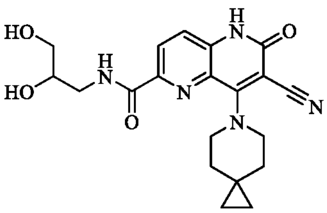 | 100 | 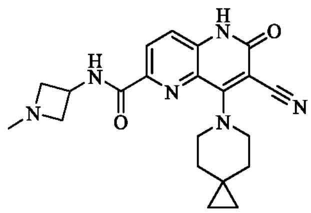 |
| 101 | 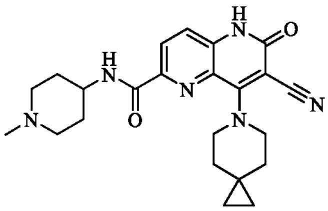 | 102 | 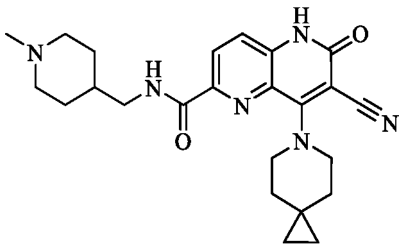 |
| 103 | 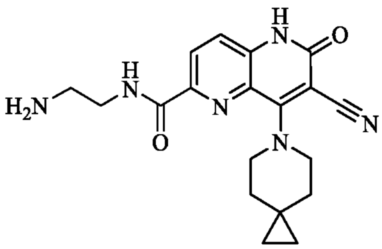 | 104 | 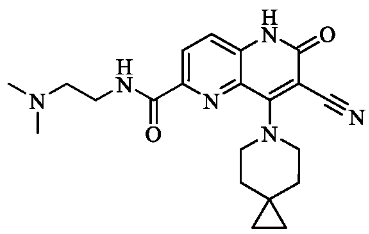 |

(continued)

| Serial number | Structure | Serial number | Structure |
|---|---|---|---|
| 105 | 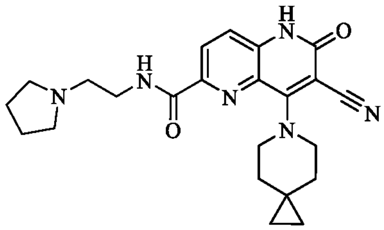 | 106 | 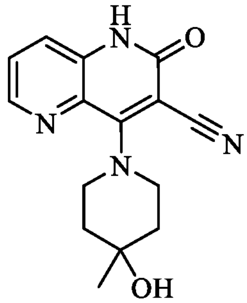 |
| 107 | 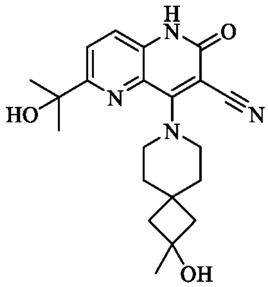 | 108 | 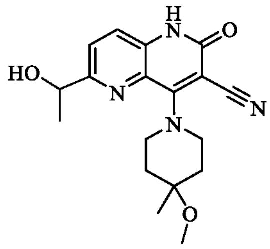 |
| 109 | 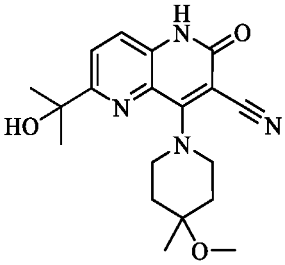 | 110 | 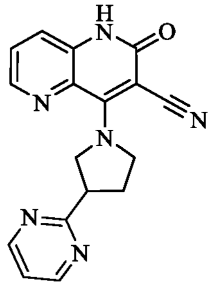 |
| 111 | 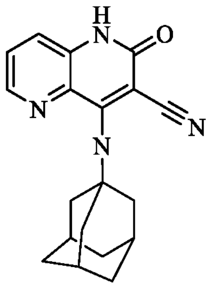 | 112 | 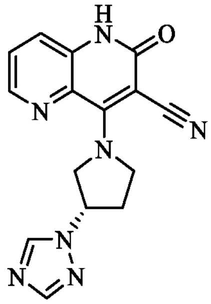 |
| 113 | 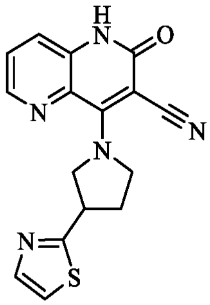 | 114 | 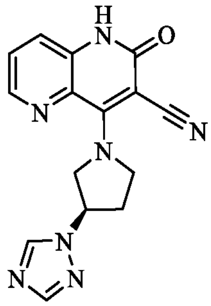 |

(continued)

| Serial number | Structure | Serial number | Structure |
|---|---|---|---|
| 115 | 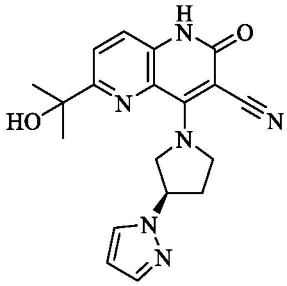 | 116 | 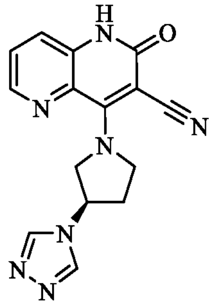 |
| 117 | 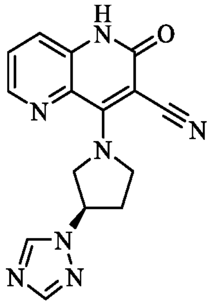 | 118 | 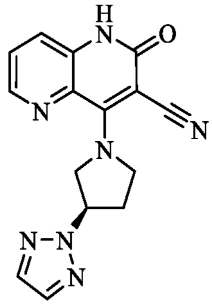 |
| 119 | 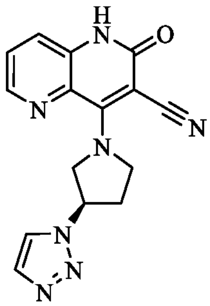 | 120 | 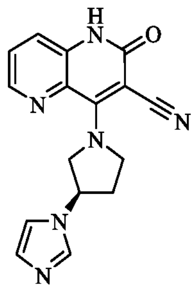 |
| 121 | 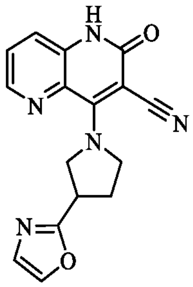 | 122 | 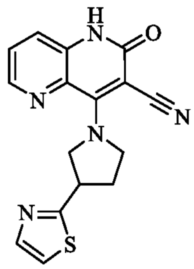 |
| 123 | 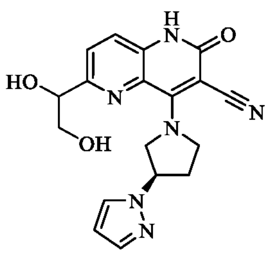 | 124 | 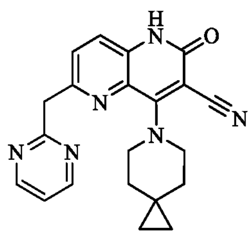 |

(continued)

| Serial number | Structure | Serial number | Structure |
|---|---|---|---|
| 125 | 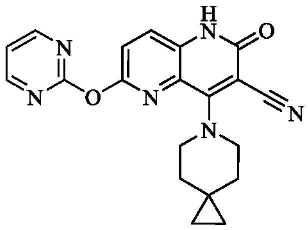 | 126 | 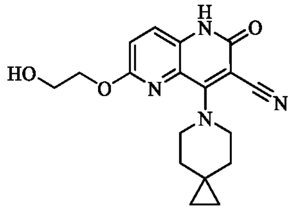 |
| 127 | 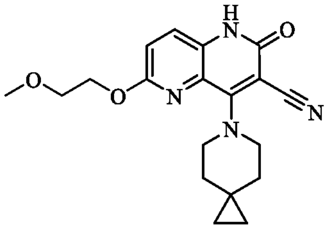 | 128 | 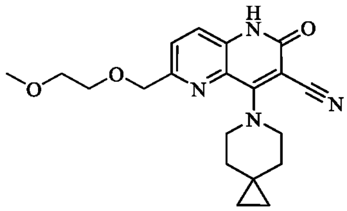 |
| 129 | 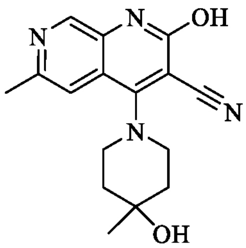 | 130 | 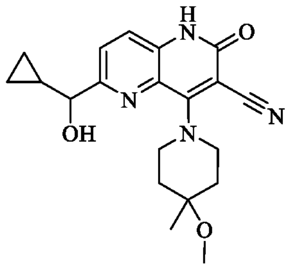 |
| 131 | 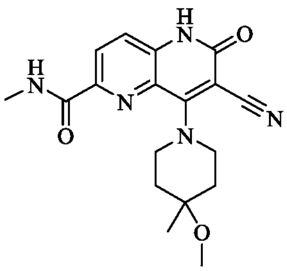 | 132 | 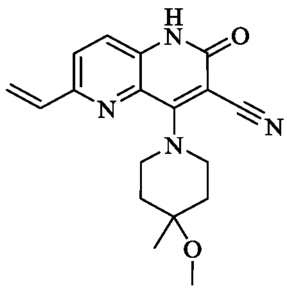 |
| 133 | 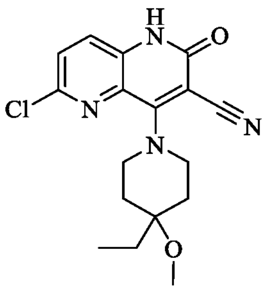 | 134 | 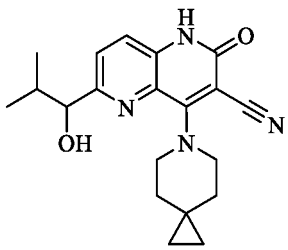 |

(continued)

| Serial number | Structure | Serial number | Structure |
|---|---|---|---|
| 135 | 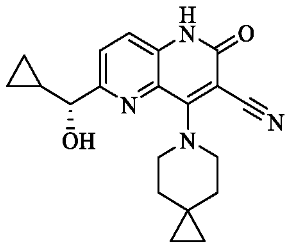 | 136 | 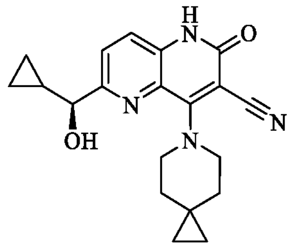 |
| 137 | 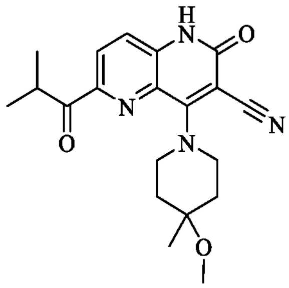 | 138 | 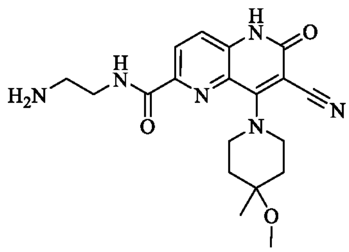 |
| 139 | 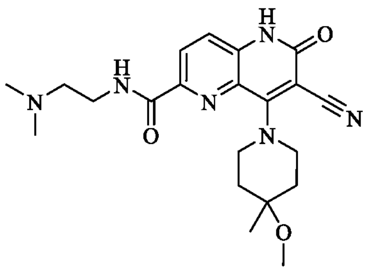 | 140 | 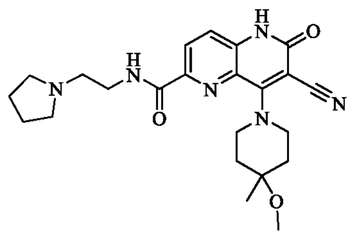 |
| 141 | 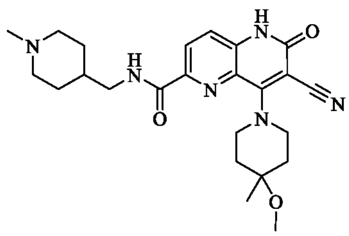 | 142 | 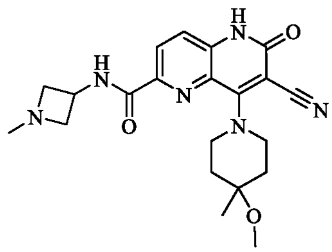 |
| 143 | 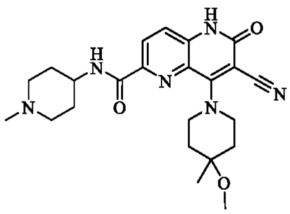 | 144 | 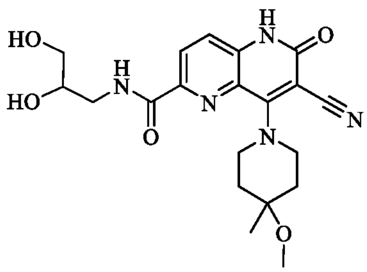 |

(continued)

| Serial number | Structure | Serial number | Structure |
|---|---|---|---|
| 145 | 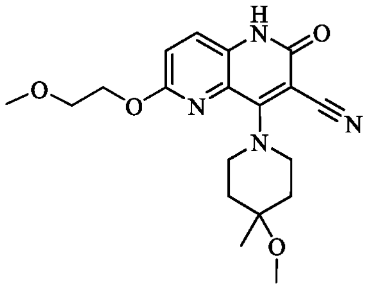 | 146 | 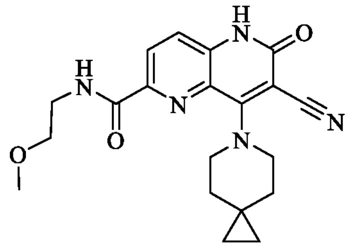 |
| 147 | 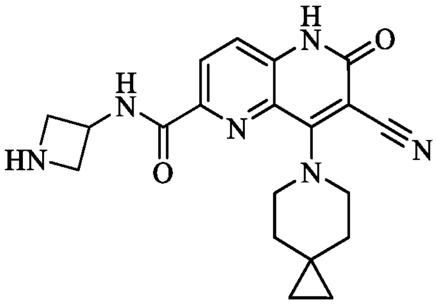 | 148 | 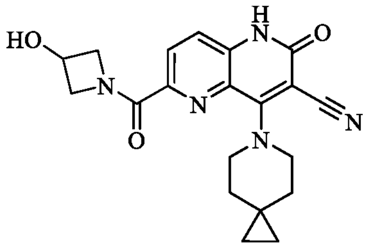 |
| 149 | 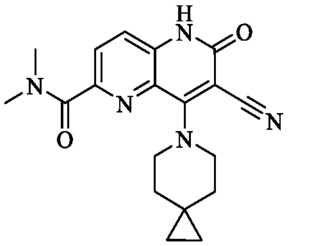 | 150 | 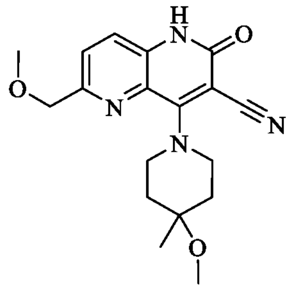 |
| 151 | 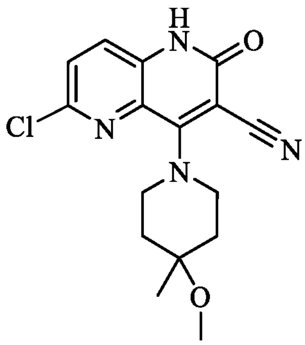 | 152 | 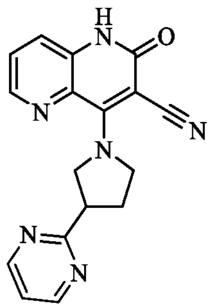 |
| 153 | 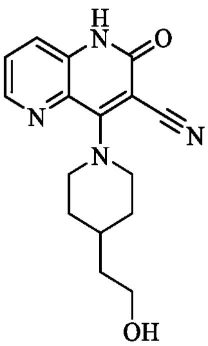 | 154 | 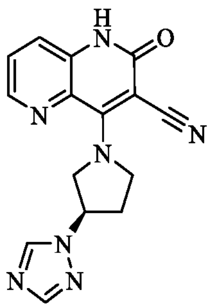 |

(continued)

| Serial number | Structure | Serial number | Structure |
|---|---|---|---|
| 155 | 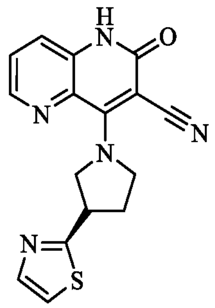 | 156 | 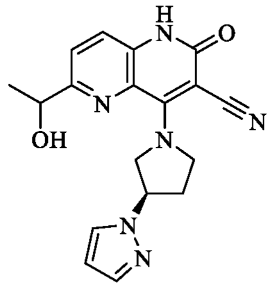 |
| 157 | 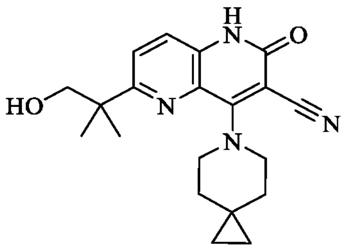 | 158 | 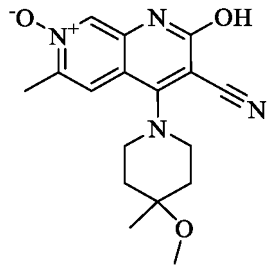 |
| 159 | 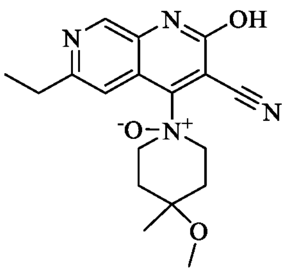 | 160 | 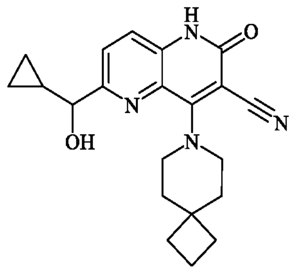 |
| 161 | 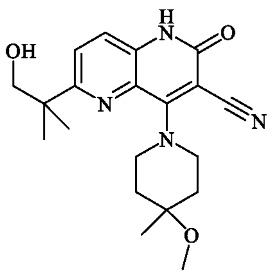 | 162 | 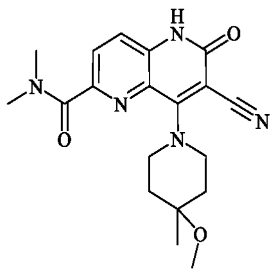 |
| 163 | 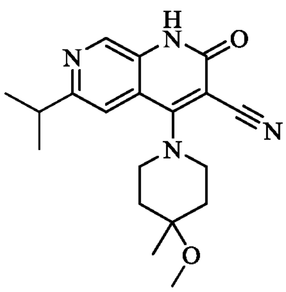 | 164 | 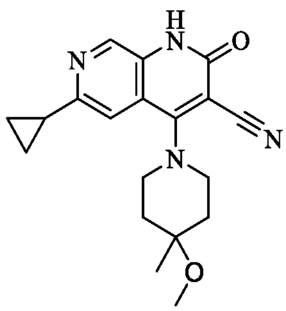 |

(continued)

| Serial number | Structure | Serial number | Structure |
|---|---|---|---|
| 165 | 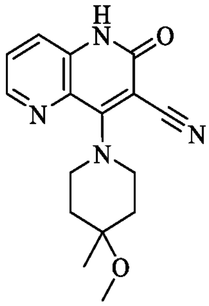 | 166 | 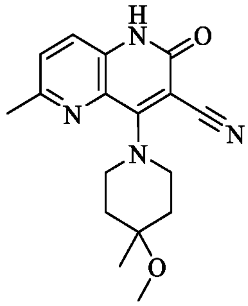 |
| 167 | 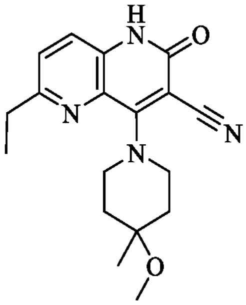 | 168 | 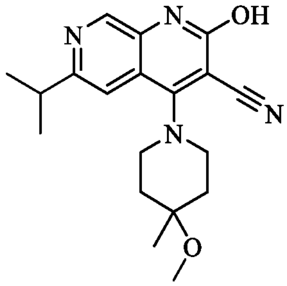 |
| 169 | 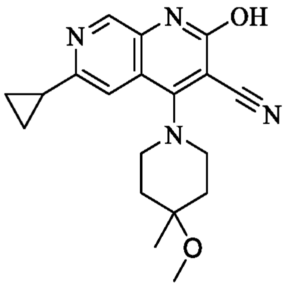 | 170 | 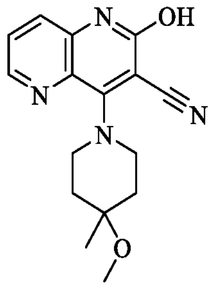 |
| 171 | 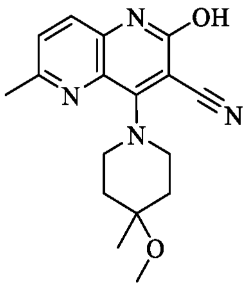 | 172 | 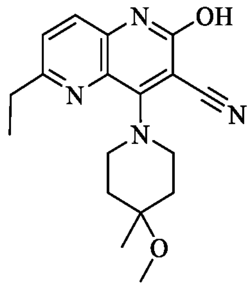 |
| 173 | 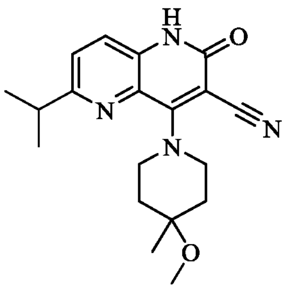 | 174 | 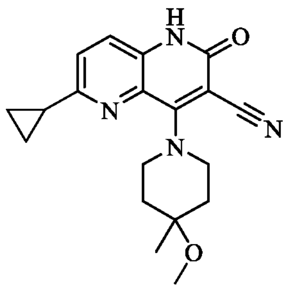 |

(continued)

| Serial number | Structure | Serial number | Structure |
|---|---|---|---|
| 175 | 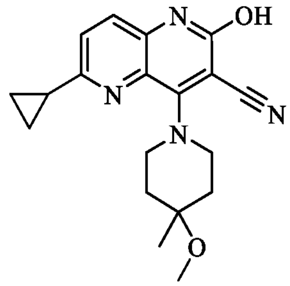 | 176 | 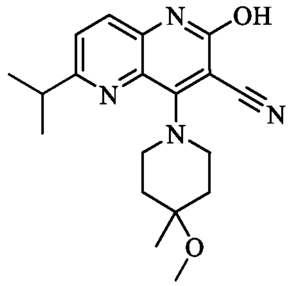 |
| 177 | 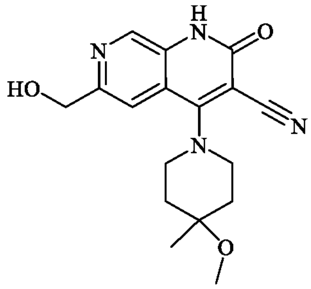 | 178 | 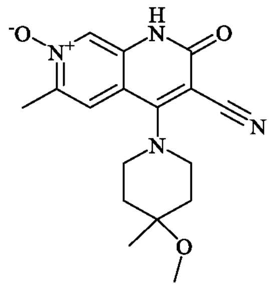 |
| 179 | 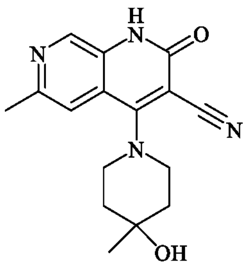 | 180 | 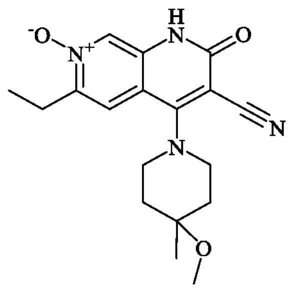 |
| 181 | 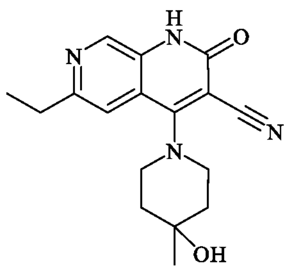 | 182 | 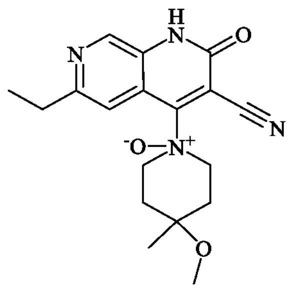 |
| 183 | 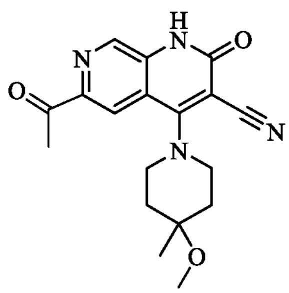 | 184 | 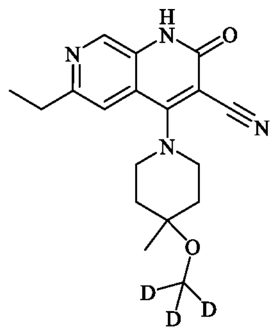 |

(continued)

| Serial number | Structure | Serial number | Structure |
|---|---|---|---|
| 185 | 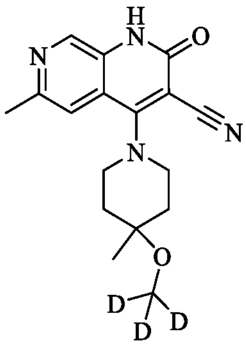 | 186 | 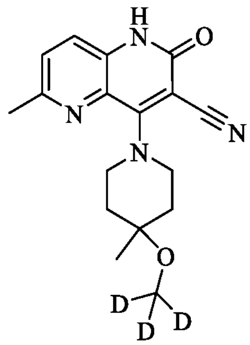 |
| 187 | 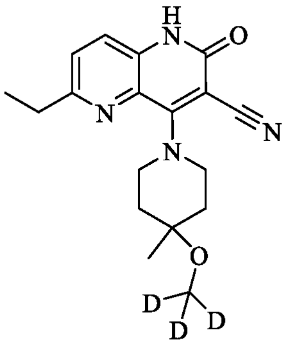 | A and B | 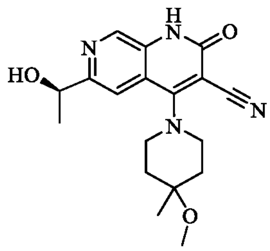 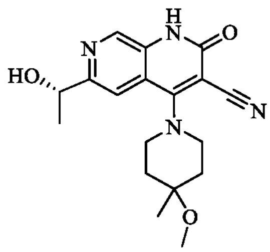 |
| 189 | 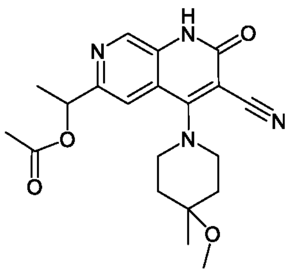 | 190 | 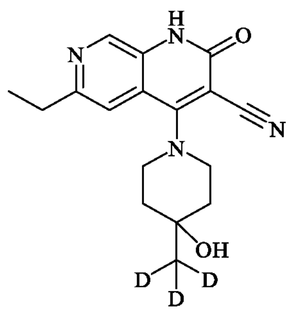 |
| 191 | 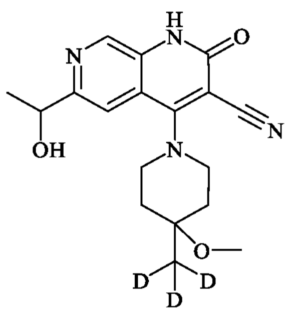 | 192 | 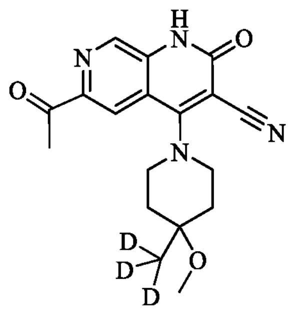 |

(continued)

| Serial number | Structure | Serial number | Structure |
|---|---|---|---|
| 193 | 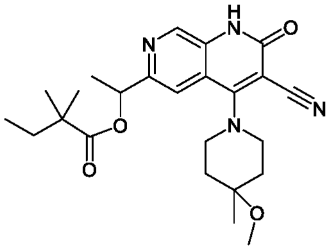 | 194 | 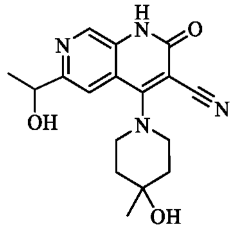 |
| 195 | 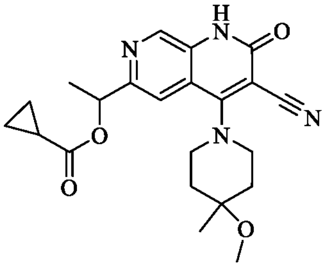 | 196 | 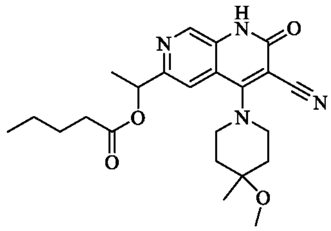 |
| 197 | 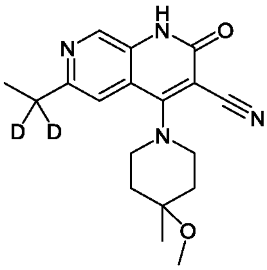 | 198 | 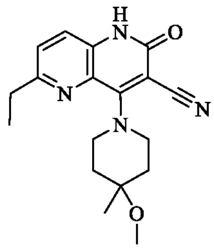 |
| 199 | 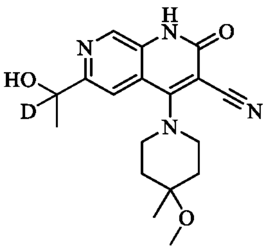 | 200 | 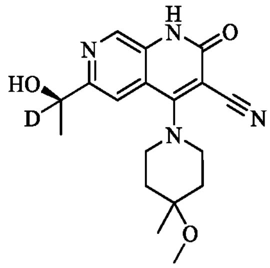 |
| 201 | 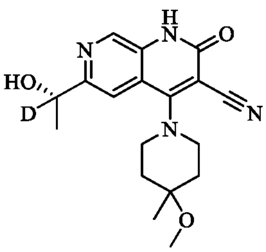 | 202 | 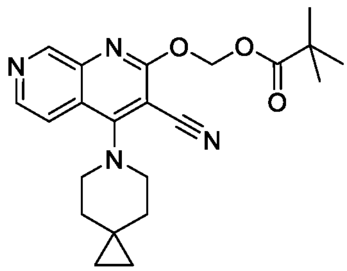 |

(continued)

| Serial number | Structure | Serial number | Structure |
|---|---|---|---|
| 203 | 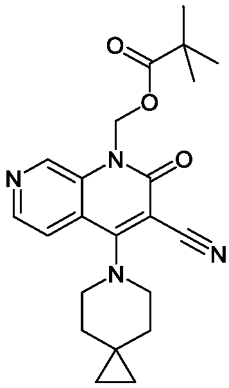 | 204 | 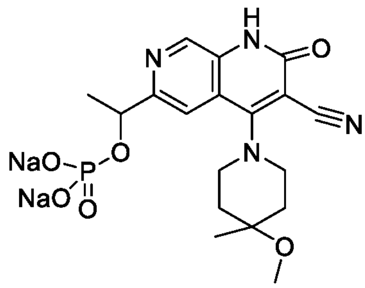 |

In one embodiment of the present invention, the hydrogen atom in the deuterated compound of the compound of formula (I) can be optionally deuterated by one or more deuterium atoms.

In one embodiment of the present invention, the deuterated compound of the compound of formula (I), (II), (III) or (IV) is selected from the following structures:

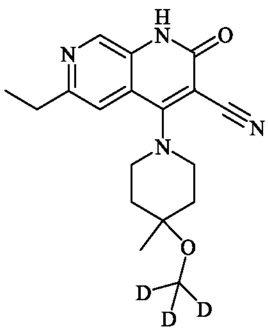
,
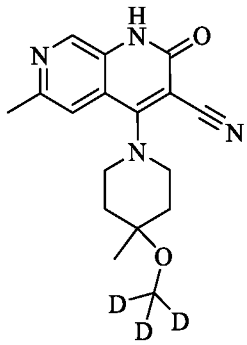
,
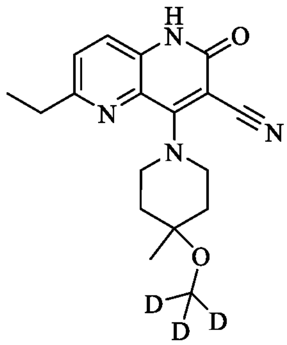
,
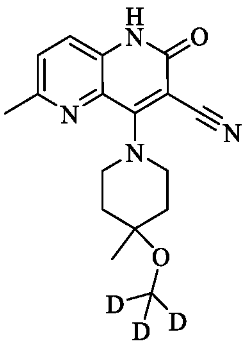
,
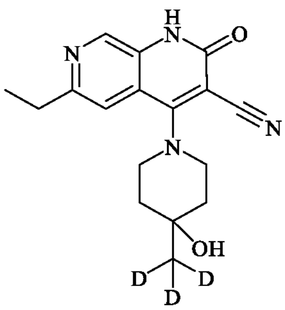
,
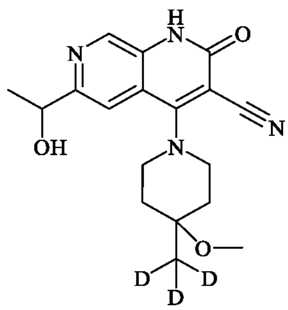
,

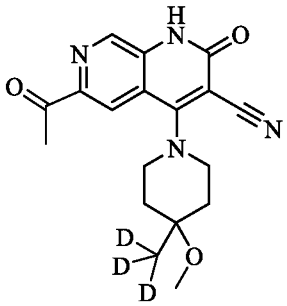,
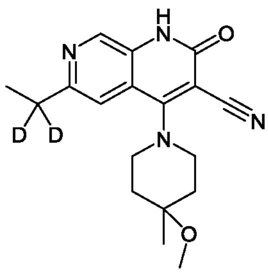,
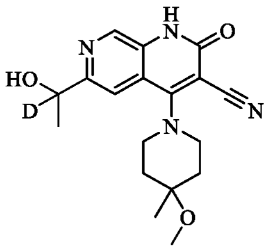,
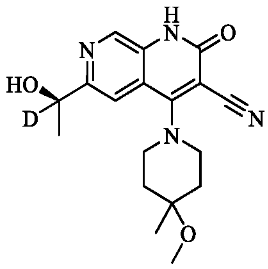

and

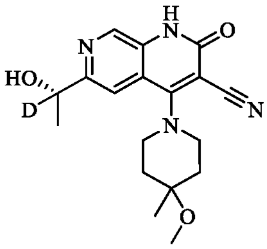.

The present invention further provides a pharmaceutical composition comprising the aforementioned compound of formula (I), (II), (III) or (IV) or the pharmaceutically acceptable salt, the isomer, the deuterated compound, the metabolite or the prodrug thereof, and one or more second therapeutic active agents.

The second therapeutically active agent is selected from acetylcholinesterase inhibitors, amyloid-β (or fragments thereof), antibodies of amyloid-β (or fragments thereof), amyloid-lowering or -inhibiting agents, α-adrenoceptor antagonists, β-adrenoceptor blockers, anticholinergics, anticonvulsants, tranquilizers, calcium channel blockers, catechol-*O*-methyltransferase inhibitors, central nervous system stimulators, corticosteroids, dopamine receptor agonists, dopamine receptor antagonists, dopamine reuptake inhibitors, γ-aminobutyric acid receptor agonists, immunomodulators, immunosuppressants, interferons, levodopa, *N*-methyl-D-aspartate receptor antagonists, monoamine oxidase inhibitors, muscarinic receptor agonists, nicotinic receptor agonists, neuroprotective agents, norepinephrine reuptake inhibitors, other PDE9 inhibitors, other phosphodiesterase inhibitors, β-secretase inhibitors, γ-secretase inhibitors, serotonin (5-hydroxytryptamine)1A (5-$HT_{1A}$) receptor antagonists, serotonin (5-hydroxytryptamine)6 (5-HT6) receptor antagonists, serotonin (5-HT) reuptake inhibitors and trophic factors.

In one embodiment of the present invention, the composition may be used by administering a "therapeutically effective amount" of the aforementioned compound of formula (I), (II), (III) or (IV) or the pharmaceutically acceptable salt, the isomer, the deuterated compound, the metabolite or the prodrug thereof and one or more second therapeutically active agents in combination, for example, sequential administration, simultaneous administration, or administration in a form of a combination formulation comprising the compound or the pharmaceutically acceptable salts, the isomers or the deuterated compounds thereof provided herein and second therapeutically active agents.

The present invention further provides a pharmaceutical formulation comprising the aforementioned compound of formula (I), (II), (III) or (IV) or the pharmaceutically acceptable salt, the isomer, the deuterated compound, the metabolite or the prodrug thereof.

In some embodiments of the present invention, the pharmaceutical formulation may comprise one or more pharmaceutically carriers.

The pharmaceutical carrier described herein may be one or more solid or liquid fillers or gel materials suitable for administration in human. The pharmaceutical carrier has sufficient purity and sufficiently low toxicity, and is compatible with the compound or the pharmaceutically acceptable salt or the isomer thereof provided herein without significantly decreasing its efficacy. For example, the pharmaceutical carrier may be a filler, a binder, a disintegrant, a lubricant, an aqueous solvent, a nonaqueous solvent, and the like.

The pharmaceutical formulation disclosed herein may be formulated into any pharmaceutically acceptable dosage form, and can be administered to a patient or a subject in need of such treatment in any suitable route of administration, such as oral, parenteral, rectal or pulmonary administration. For oral administration, it can be formulated into tablets, capsules, pills, granules, and the like. Forparenteral administration, it can be formulated into injections, sterile powders for injection, and the like.

The present invention further provides use of the aforementioned compound of formula (I), (II), (III) or (IV) or the pharmaceutically acceptable salt, the isomer, the deuterated compound, the metabolite or the prodrug thereof, the aforementioned pharmaceutical formulation or the aforementioned pharmaceutical composition in the manufacture of a medicament for treating or preventing a PDE9-mediated related disease; specifically, the PDE9-mediated related disease is cognitive impairment caused by central nervous system disorders; and more specifically, the cognitive impairment includes impairments of perception, concentration, memory and learning, including but not limited to senile dementia, schizophrenia, age-related memory loss, vascular dementia, craniocerebral trauma, stroke, post-stroke dementia, post-traumatic dementia, general concentration impairment, concentration impairments in children with learning and memory problems, Alzheimer's disease, Lewy body dementia, dementia with degeneration of the frontal lobes, dementia with corticobasal degeneration, amyotrophic lateral sclerosis, Huntington's disease, multiple sclerosis, thalamic degeneration, Creutzfeldt-Jakob dementia, HIV dementia, schizophrenia, Korsakoff's psychosis, and/or depression or bipolar disorder.

The present invention further provides use of the aforementioned compound of formula (I), (II), (III) or (IV) or the pharmaceutically acceptable salt, the isomer, the deuterated compound, the metabolite or the prodrug thereof, the aforementioned pharmaceutical formulation or the aforementioned pharmaceutical composition in treating or preventing diseases.

The present invention further provides use of the aforementioned compound of formula (I), (II), (III) or (IV) or the pharmaceutically acceptable salt, the isomer, the deuterated compound, the metabolite or the prodrug thereof, the aforementioned pharmaceutical formulation or the aforementioned pharmaceutical composition in treating or preventing a PDE9-mediated related disease; specifically, the PDE9-mediated related disease is cognitive impairment caused by central nervous system disorders; and more specifically, the cognitive impairment includes impairments of perception, concentration, memory and learning, including but not limited to senile dementia, schizophrenia, age-related memory loss, vascular dementia, craniocerebral trauma, stroke, post-stroke dementia, post-traumatic dementia, general concentration impairment, concentration impairments in children with learning and memory problems, Alzheimer's disease, Lewy body dementia, dementia with degeneration of the frontal lobes, dementia with corticobasal degeneration, amyotrophic lateral sclerosis, Huntington's disease, multiple sclerosis, thalamic degeneration, Creutzfeldt-Jakob dementia, HIV dementia, schizophrenia, Korsakoff's psychosis, and/or depression or bipolar disorder.

The present invention further provides a method for treating or preventing a disease, comprising administering to a patient in need thereof a therapeutically effective amount of the aforementioned compound of formula (I), (II), (III) or (IV) or the pharmaceutically acceptable salt, the isomer, the deuterated compound, the metabolite or the prodrug thereof, the aforementioned pharmaceutical formulation or the aforementioned pharmaceutical composition, wherein the disease is a PDE9-mediated related disease; specifically, the PDE9-mediated related disease is cognitive impairment caused by central nervous system disorders; and more specifically, the cognitive impairment includes impairments of perception, concentration, memory and learning, including but not limited to senile dementia, schizophrenia, age-related memory loss, vascular dementia, craniocerebral trauma, stroke, post-stroke dementia, post-traumatic dementia, general concentration impairment, concentration impairments in children with learning and memory problems, Alzheimer's disease, Lewy body dementia, dementia with degeneration of the frontal lobes, dementia with corticobasal degeneration, amyotrophic lateral sclerosis, Huntington's disease, multiple sclerosis, thalamic degeneration, Creutzfeldt-Jakob dementia, HIV dementia, schizophrenia, Korsakoff's psychosis, and/or depression or bipolar disorder.

## DETAILED DESCRIPTION OF THE INVENTION

The "halogen" described herein refers to fluorine, chlorine, bromine, iodine, and the like, and preferably fluorine and chlorine.

The "halogenated" described herein means that any hydrogen atom in a substituent can be substituted with one or more identical or different halogen atoms. "Halogen" is defined as above.

The "$C_{1-6}$ alkyl" described herein refers to linear or branched alkyl derived by removing one hydrogen atom from a hydrocarbon moiety containing 1 to 6 carbon atoms, such as methyl, ethyl, *n*-propyl, isopropyl, *n*-butyl, isobutyl, *sec*-butyl, *tert*-butyl, *n*-pentyl, isopentyl, 2-methylbutyl, neopentyl, 1-ethylpropyl, *n*-hexyl, isohexyl, 4-methylpentyl, 3-methylpentyl, 2-methylpentyl, 1-methylpentyl, 3,3-dimethylbutyl, 2,2-diniethylbutyl, 1,1-dimethylbutyl, 1,2-dimethylbutyl, 1,3-dimethylbutyl, 2,3-dimethylbutyl, 2-ethylbutyl and 1-methyl-2-methylpropyl. The "$C_{1-4}$ alkyl" refers to the aforementioned examples containing 1 to 4 carbon atoms.

The "$C_{2-8}$ alkenyl" described herein refers to linear, branched or cyclic alkenyl derived by removing one hydrogen atom from an alkene moiety containing 2 to 8 carbon atoms and a carbon-carbon double bond, such as vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl and 1,4-hexadienyl

The "$C_{2-8}$ alkynyl" described herein refers to linear or branched alkynyl derived by removing one hydrogen atom from an alkyne moiety containing 2 to 8 carbon atoms and a carbon-carbon triple bond, such as ethynyl, propynyl,

2-butynyl, 2-pentynyl, 3-pentynyl, 4-methyl-2-pentynyl, 2-hexynyl and 3-hexynyl.

The "$C_{1-6}$ alkoxy" described herein refers to a group in which the "$C_{1-6}$ alkyl" defined above is linked to a parent molecule via an oxygen atom, i.e., a "$C_{1-6}$ alkyl-O-" group, such as methoxy, ethoxy, *n*-propoxy, isopropoxy, *n*-butoxy, tert-butoxy, *n*-pentyloxy, neopentyloxy and n-hexyloxy. The "$C_{1-4}$ alkoxy" refers to the aforementioned examples containing 1 to 4 carbon atoms, ie., a "$C_{1-4}$ alkyl-O-" group.

The "$C_{1-6}$ alkylamino", "($C_{1-6}$ alkyl)$_2$amino", "$C_{1-6}$ alkylcarbonylamino", "$C_{1-6}$ alkylsulfonylamino", "$C_{1-6}$ alkylaminocarbonyl", "($C_{1-6}$ alkyl)$_2$aminocarbonyl", "$C_{1-6}$ alkoxycarbonyl", "$C_{1-6}$ alkylsulfonyl", "$C_{1-6}$ alkylthio" and "$C_{1-6}$ alkylcarbonyl" refer to $C_{1-6}$ alkyl-NH-, ($C_{1-6}$ alkyl)($C_{1-6}$ alkyl)N-, $C_{1-6}$ alkyl-C(O)-NH-, $C_{1-6}$ alkylS(O)$_2$-NH$_2$-, $C_{1-6}$ alkyl-NH-C(O)-, ($C_{1-6}$ alkyl)($C_{1-6}$ alkyl)N-C(O)-, $C_{1-6}$ alkyl-O-C(O)-, $C_{1-6}$ alkyl-S(O)$_2$-, $C_{1-6}$ alkyl-S- and $C_{1-6}$ alkyl-C(O)-, respectively, wherein the "$C_{1-6}$ alkyl" is defined as above, and is preferably "$C_{1-4}$ alkyl".

The "polycyclic ring" described herein refers to a multi-ring system structure formed by two or more ring structures connected by an ortho-fused, spiro- or bridged linkage. The ortho-fused ring refers to a polycyclic structure formed by two or more ring structures sharing two adjacent ring atoms (i.e., sharing a bond) with each other. The bridged ring refers to a polycyclic structure formed by two or more ring structures sharing two non-adjacent ring atoms with each other. The spiro-ring refers to a polycyclic structure formed by two or more ring structures sharing a ring atom with each other.

Unless otherwise specified, the "3-12 membered cycloalkenyl" described herein includes all possibly formed monocyclic and polycyclic (including fused in the form of ortho-, spiro- or bridged) cases, such as 3-8 membered monocyclic cycloalkenyl, 7-11 membered spiro-cycloalkenyl, 7-11 membered ortho-fused cycloalkenyl and 6-11 membered bridged cycloalkenyl.

The cycloalkyl described herein includes all possibly formed monocyclic and polycyclic (including fused in the form of ortho-, spiro- or bridged) cases. For example, "3-12 membered cycloalkyl" can be a monocyclic, bicyclic or polycyclic cycloalkyl system (also referred to as a polycyclic ring system). Unless otherwise specified, the monocyclic ring system is a cyclic hydrocarbon group containing 3 to 8 carbon atoms. Examples of 3-8 membered cycloalkyl include, but are not limited to: cyclopropyl, cyclobutyl, cyclopentyl, cyclohexyl, cycloheptyl, cyclooctyl, and the like. Polycyclic cycloalkyl includes ortho-fused cycloalkyl, bridged cycloalkyl and spiro-cycloalkyl Ortho-fused cycloalkyl may be 6-11 membered ortho-fused cycloalkyl or 7-10 membered ortho-fused cycloalkyl, and the representative examples thereof include, but are not limited to, bicyclo[3.1.1]heptane, bicyclo[2.2.1]heptane, bicyclo[2.2.2]octane, bicyclo[3.2.2]nonane, bicyclo[3.3.1]nonane and bicyclo[4.2.1]nonane. The spiro-cycloalkyl may be 7-12 membered spiro-cycloalkyl or 7-11 membered spiro-cycloalkyl, and the examples thereof include, but are not limited to:

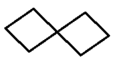, 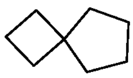, 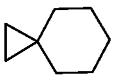, 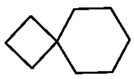, 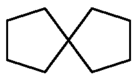, 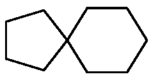 and

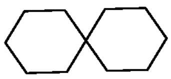.

The bridged cycloalkyl may be 6-11 membered bridged cycloalkyl or 7-10 membered bridged cycloalkyl, and the examples thereof include, but are not limited to:

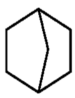, 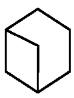, 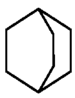, 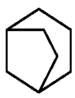, 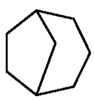 and 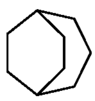.

The "heterocyclyl" described herein refers to a 3-12 membered non-aromatic cyclic group in which at least one ring carbon atom is replaced with a heteroatom selected from O, S and N, and preferably 1 to 3 heteroatoms are present, wherein a carbon atom, a nitrogen atom and a sulfur atom may be oxidized.

"3-12 membered heterocyclyl" refers to a monocyclic heterocyclyl, bicyclic heterocyclyl, or polycyclic heterocyclyl system (also referred to as a fused ring system), including saturated and partially saturated heterocyclyl groups, but excluding aromatic rings. Unless otherwise specified, all possibly formed monocyclic, polycyclic (including fused in the form of ortho-, spiro- or bridged), saturated and partially saturated cases are included.

The monocyclic heterocyclyl may be 3-8 membered heterocyclyl, 3-8 membered saturated heterocyclyl, 3-6 membered heterocyclyl, 4-7 membered heterocyclyl, 5-7 membered heterocyclyl, 5-6 membered heterocyclyl, 5-6 membered oxygen-containing heterocyclyl, 3-8 membered nitrogen-containing heterocyclyl, 5-6 membered nitrogen-containing heterocyclyl, 5-6 membered saturated heterocyclyl, or the like. Examples of the "3-8 membered saturated heterocyclyl"

include, but are not limited to, aziridinyl, oxiranyl, thiiranyl, azetidinyl, oxetanyl, thietanyl, tetrahydrofuranyl, pyrrolidinyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2*H*-pyranyL, tetrahydro-2*H*-thiapyranyl, piperidinyl, piperazinyl, morpholinyl, 1,4-dioxanyl and 1,4-oxathianyl Examples of the "3-8 membered partially saturated heterocyclyl" include, but are not limited to, 4,5-dihydroisoxazolyl, 4,5-dihydrooxazolyl, 2,5-dihydrooxazolyl, 2,3-dihydrooxazolyl, 3,4-dihydro-2*H*-pyrrolyl, 2,3-dihydro-1*H*-pyrrolyl, 2,5-dihydro-1*H*-imidazolyl, 4,5-dihydro-1*H*-imidazolyl, 4,5-dihydro- 1H-pyrazolyl, 4,5-dihydro-3*H*-pyrazolyl, 4,5-dihydrothiazolyl, 2,5-dihydrothiazolyL, 2*H*-pyranyl, 4*H*-pyranyL 2*H*-thiapyranyl, 4*H*-thiapyranyl, 2,3,4,5-tetrahydropyridinyl, 1,2-isoxazinyl, 1,4-isoxazinyl, 6*H*-1,3-oxazinyl and the like. Polycyclic heterocyclyl includes ortho-fused heterocyclyl, spiro-heterocyclyl and bridged heterocyclyl, which may be saturated, partially saturated or unsaturated, but non-aromatic. Polycyclic heterocyclyl may be 5-6 membered monocyclic heterocyclyl ring which is fused to a benzene ring, 5-6 membered monocyclic cycloalkyl, 5-6 membered monocyclic cycloalkenyl, 5-6 membered monocyclic heterocyclyl or 5-6 membered monocyclic heteroaryl. The ortho-fused heterocyclyl may be 6-12 membered ortho-fused heterocyclyl, 7-10 membered ortho-fused heterocyclyl, 6-10 membered ortho-fused heterocyclyl or 6-12 membered saturated ortho-fused heterocyclyl, and representative examples include, but are not limited to: 3-azabicyclo[3.1.0]hexyl, 3,6-diazabicyclo[3.2.0]heptyl, 3,8-diazabicyclo[4.2.0]octyl, 3,7-diazabicyclo[4.2.0]octyl, octahydropyrrolo[3,4-*c*]pyrrolyl, octahydropyrrolo[3,4-*b*] pyrrolyl, octahydropyrrolo[3,4-*b*][1,4]oxazinyl, octahydro-1*H*-pyrrolo[3,4-*c*]pyridinyl, 2,3-dihydrobenzofuran-2-yl, 2,3-dihydrobenzofuran-3-yl, indolin-1-yl, indolin-2-yl, indolin-3-yl, 2,3-dihydrobenzothiophen-2-yl, octahydro-1*H*-indolyl and octahydrobenzofuranyl. The spiro-heterocyclyl may be 6-12 membered spiro-heterocyclyl, 7-11 membered spiro-heterocyclyl or 6-12 membered saturated spiro-heterocyclyl, and examples thereof include, but are not limited to:

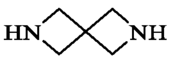 , 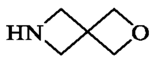 , 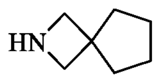 , 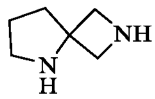 ,

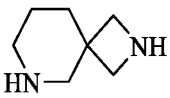 , 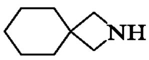 , 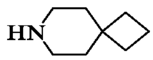 , 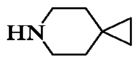 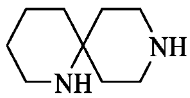 , 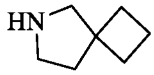 ,

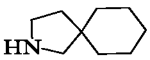 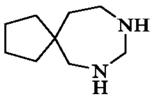 , 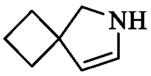 , 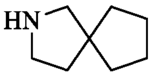 , 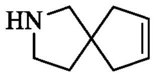 , 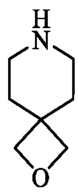 , 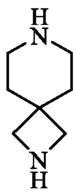 ,

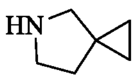 and 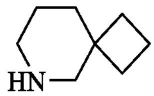 .

The bridged heterocyclyl may be 6-12 membered bridged heterocyclyl, 7-11 membered bridged heterocyclyl or 6-12 membered saturated bridged heterocyclyl, and examples thereof include, but are not limited to:

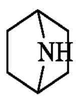 , 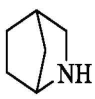 , 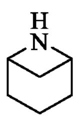 , 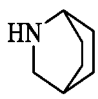 , 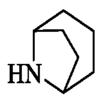 , 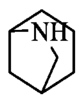 ,

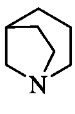 , 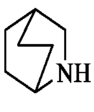 , 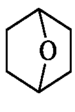 , 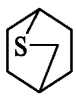 , 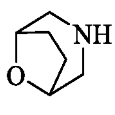 and 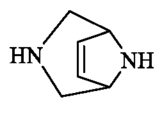 .

The "aryl" described herein refers to a cyclic aromatic group containing 6 to 14 carbon atoms, including phenyl, naphthalene, phenanthrene, and the like.

The heteroaryl described herein includes all possibly formed monocyclic, polycyclic, fully aromatic and partially aromatic cases. For example, "5-10 membered heteroaryl" refers to an aromatic cyclic group in which at least one ring carbon atom is replaced with a heteroatom selected from O, S and N, and preferably 1 to 3 heteroatoms are present. Moreover, the case where carbon atoms or sulfur atoms are oxidized is included. For example, carbon atoms are replaced with C(O), sulfur atoms are replaced with S(O) or $S(O)_2$, and nitrogen atoms

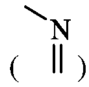

may be replaced with

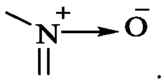

Unless otherwise specified, heteroaryl includes monocyclic heteroaryl and polycyclic heteroaryl. Monocyclic heteroaryl may be 5-7 membered heteroaryl or 5-6 membered heteroaryl, and examples thereof include, but are not limited to, furanyl, imidazolyl, isoxazolyl, thiazolyl, isothiazolyl, oxadiazolyl, oxazolyl, pyridinyl, pyridazinyl, pyrimidinyl, pyrazinyl, pyrazolyl, pyrrolyl, tetrazolyl, thiadiazolyl, thienyl, triazolyl and triazinyl. In certain examples, polycyclic heteroaryl refers to a group in which a monocyclic heteroaromatic ring is fused to phenyl, cycloalkenyl, heteroaryl, cycloalkyl or heterocyclyl. Polycyclic heteroaryl may be 8-12 membered ortho-fused heteroaryl or 9-10 membered ortho-fused heteroaryl, and examples include, but are not limited to, benzimidazolyl, benzofuranyl, benzothienyl, benzoxadiazolyl, benzothiadiazolyl, benzothiazolyl, cinnolinyl, 5,6-dihydroquinolin-2-yl, 5,6-dihydroisoquinolin-1-yl, furopyridinyl, indazolyl, indolyl, isoindolyl, isoquinolinyl, naphthyridinyl, purinyl, quinolinyl, 5,6,7,8-tetrahydroquinolin- 2-yl, 5,6,7,8-tetrahydroquinolinyl, 5,6,7,8-tetrahydroquinolin-4-yl, 5,6,7,8-tetrahydroisoquinolin -1-yl, thienopyridinyl, 4,5,6,7-tetrahydro[*c*][1,2,5]oxadiazolyl and 6,7-dihydro[*c*][1,2,5] oxadiazol-4(5*H*)keto.

The "pharmaceutically acceptable salt" described herein refers to a pharmaceutically acceptable addition salt of acid and base or a solvate thereof. Such pharmaceutically acceptable salts include salts of the following acids: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid, HOOC-(CH2)n-COOH (wherein n is 0-4)), and the like. The following salts of bases are also included: sodium salt, potassium salt, calcium salt, ammonium salt, and the like. Those skilled in the art know a variety of pharmaceutically acceptable non-toxic addition salts.

The "isomer" described herein refers to a stereoisomer and a tautomer.

The stereoisomer refers to an enantiomer in the case that atoms are asymmetric in a compound, and a cis-trans isomer in the case that a double bond or a cyclic structure exists in a compound. All enantiomers, diastereomers, racemic isomers, cis-trans isomers, geometric isomers, epimers and mixtures thereof of the compound of formula (I) are included in the scope of the present invention.

The "tautomer" refers to a functional group isomer that is produced due to the rapid shifting of a certain atom between two positions in a molecule, and the tautomer is a special functional group isomer. Examples include tautomerization of a carbonyl compound containing $\alpha$-H, specifically as follows:

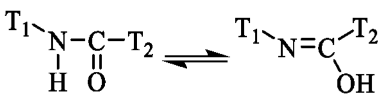

and

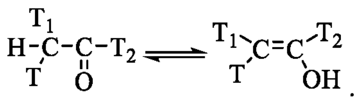

The tautomerization may also be, for example, other prototropic tautomerizations, specifically such as phenol-keto tautomerization, nitroso-oximino tautomerization and imine-enamine tautomerization.

T, T1 and T2 are each independently any group that accords with the bonding rule of a compound.

The compound disclosed herein contains a lactam structure and involves the following tautomerization:

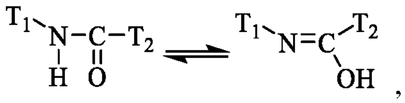

and therefore when referring to the compound disclosed herein, it means that the tautomers of the compound are also referred to. If any one tautomer type is obtained in the synthesis example of the present invention, it means that another tautomeric configuration is obtained at the same time; they can be rapidly converted to each other and are in dynamic equilibrium.

The "deuterated" described herein refers to the replacement of one or more hydrogen atoms with deuterium atoms in a compound or group.

The "therapeutically effective amount" described herein refers to an amount of the aforementioned compound or the pharmaceutically acceptable salt or isomer thereof, the composition or the pharmaceutical formulation thereof, that, when administered to a patient, is at least capable of alleviating symptoms of the patient's condition. An actual amount comprising the "therapeutically effective amount" will vary depending on a variety of circumstances, including, but not limited to, the particular condition being treated, the severity of the condition, the physique and health of the patient, and the route of administration. The appropriate amount can be readily determined by skilled medical practitioners using methods known in the medical field.

The "N atom" described herein may be replaced with

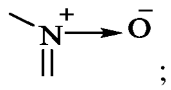

the "C atom" may be replaced with C(O); the "S atom" may be replaced with S(O) or $S(O)_2$.

**Compound preparation**

The compound disclosed herein can be prepared by a variety of methods including standard chemical methods. Unless otherwise stated, any variable defined above will continue to have the meaning defined above. Exemplary general synthesis methods are elaborated in the following schemes, and can be easily modified to prepare other compounds disclosed herein. The specific compounds disclosed herein were prepared in examples.

In some examples, the compound of formula (I) can be prepared by metal-catalyzed coupling, aromatic nucleophilic substitution or other reactions of a compound of formula (I-d) with a compound of formula (I-e).

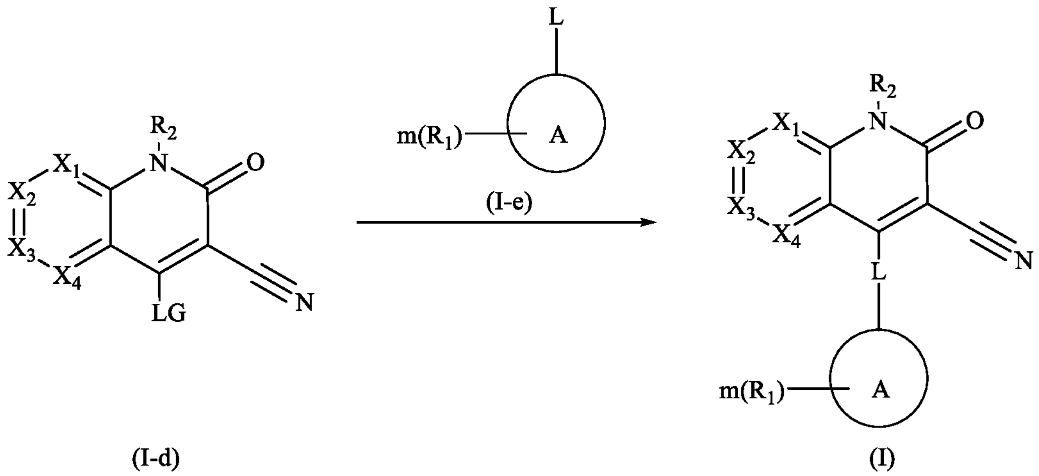

In some examples, the compound of formula (I-d) can be prepared by the reaction of a compound of formula (I-c) with a halogenating agent, or substituted or unsubstituted sulfonyl chloride or sulfonic anhydride.

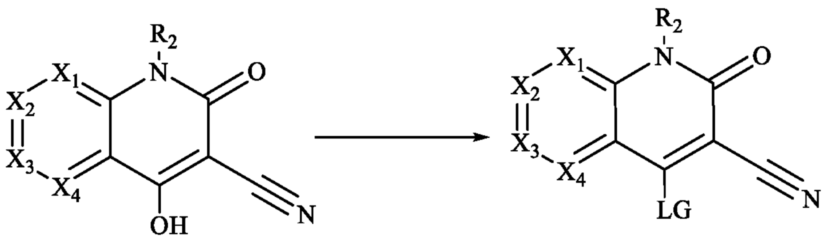

(I-c) (I-d)

In some examples, the compound of formula (I-c) can be prepared by cyclization of a compound of formula (I-b) in the presence of a suitable base.

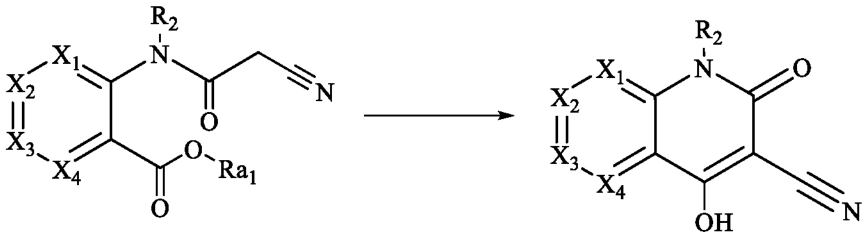

(I-b) (I-c)

In some examples, the compound of formula (I-b) can be prepared by the reaction of a compound of formula (I-a) with cyanoacetic acid in the presence of a suitable peptide coupling agent.

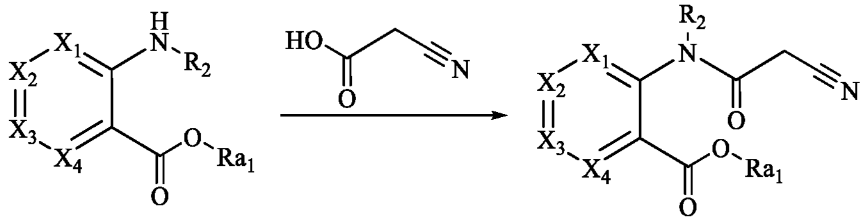

(I-a) (I-b)

In the above examples, $X_1$, $X_2$, $X_3$, $X_4$, $R_1$, $R_2$, A, L and m are defined as above, $Ra_1$ is selected from $C_{1-6}$ alkyl, and LG is selected from halogen, substituted or unsubstituted benzene sulfonate, $C_{1-6}$ alkyl sulfonate, triflate and the like.

In some examples, the compound of formula (I) can be prepared by metal-catalyzed coupling reaction of a compound of formula (I-f2) with a suitable reagent comprising an $R_2$ group, or by metal-catalyzed coupling reaction of a compound of formula (I-f2) with a suitable reagent followed by one or more conventional chemical transformations (such as oxidation, reduction, addition, substitution, hydrogenation, chlorination and amination).

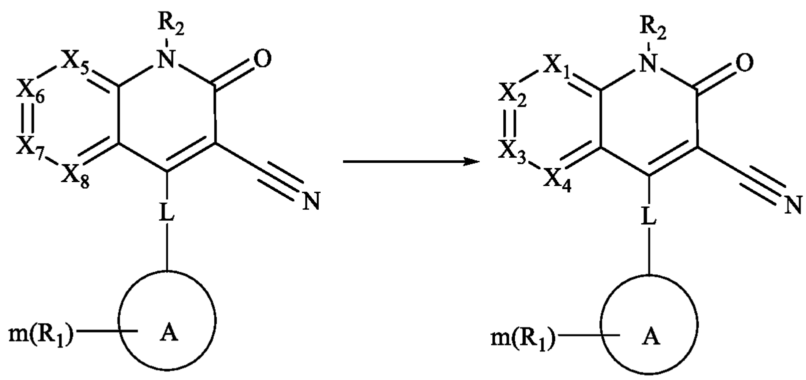

(I-f2) (I)

In some examples, the compound of (I-f2) can be prepared by aromatic nucleophilic substitution or other reactions of a compound of formula (I-d2) with the compound of formula (I-e).

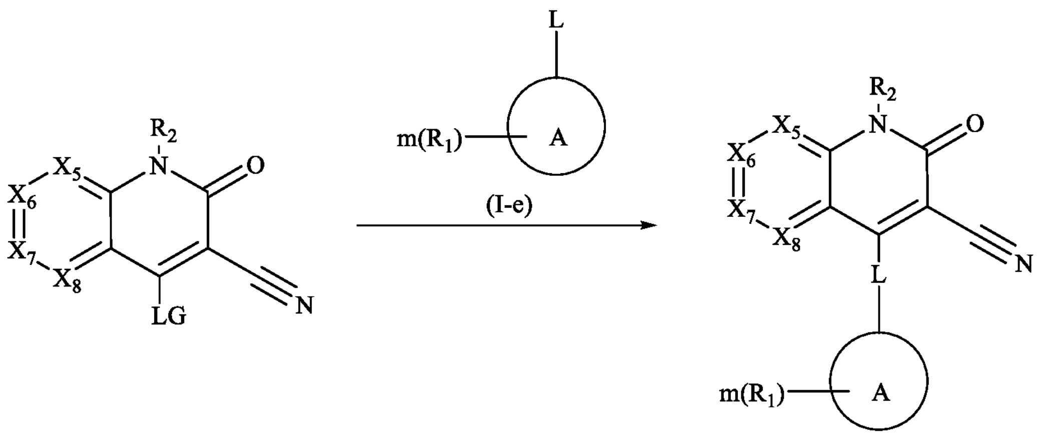

(I-d2) (I-f2)

In some examples, the compound of formula (I-d2) can be prepared by the reaction of a compound of formula (I-c2) with a halogenating agent, or substituted or unsubstituted sulfonyl chloride or sulfonic anhydride.

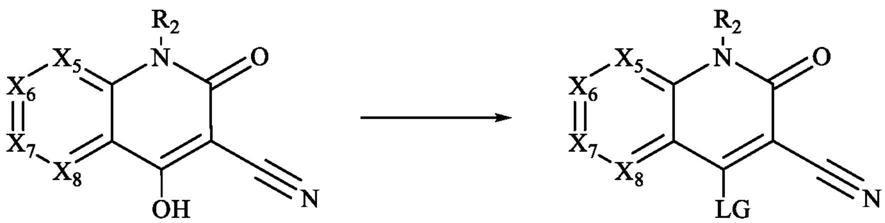

(I-c2) (I-d2)

In some examples, the compound of formula (I-c2) can be prepared by cyclization of a compound of formula (I-b2) in the presence of a suitable base.

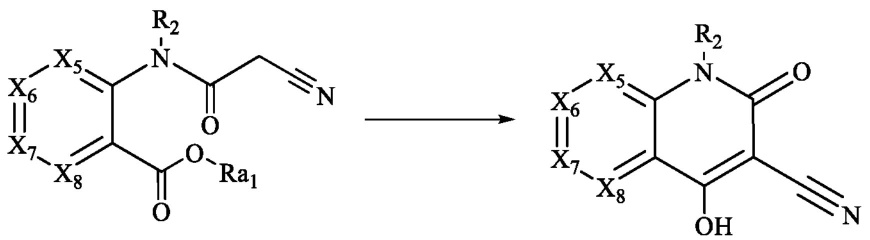

(I-b2) (I-c2)

In some examples, the compound of formula (I-b2) can be prepared by the reaction of a compound of formula (I-a2) with cyanoacetic acid in the presence of a suitable peptide coupling agent.

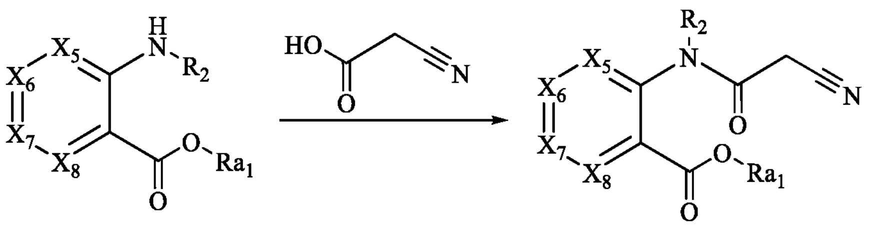

(I-a2) (I-b2)

In the above examples, $X_1$, $X_2$, $X_3$, $X_4$, $R_1$, $R_2$, A, L and m are defined as above, $Ra_1$ is selected from $C_{1-6}$ alkyl, and LG is selected from halogen, substituted or unsubstituted benzene sulfonate, $C_{1-6}$ alkyl sulfonate, triflate and the like. $X_5$, $X_6$, $X_7$ and $X_8$ are independently selected from N and C-Xa, wherein Xa is bromine or iodine, and at least one of $X_5$, $X_6$, $X_7$ and $X_8$ is N.

The halogenating agent refers to reagents used in the halogenation reaction, including but not limited to *N*-bromosuccinimide, *N*-chlorosuccinimide, *N*-iodosuccinimide, dibromohydantoin, phosphorus tribromide, phosphine trichloride, thionyl chloride, phosphorus oxychloride, phosphorus pentachloride, or phosphorus oxybromide.

The suitable base includes organic and inorganic bases. The organic bases include, but are not limited to, sodium *tert*-butoxide, potassium *tert*-butoxide, sodium ethoxide, sodium methoxide, LiHMDS, *N,N*-diisopropylethylamine, triethylamine, lithium diisopropylamide, and the like. The inorganic bases include, but are not limited to, sodium hydride, sodium carbonate, potassium carbonate, cesium carbonate, potassium phosphate, potassium hydroxide, sodium hydroxide, magnesium hydroxide, calcium hydroxide, and the like.

The substituted or unsubstituted sulfonyl chloride refers to $Ra_2$-$SO_2Cl$, and

the substituted or unsubstituted sulfonic anhydride refers to $(Ra_2\text{-}SO_2)_2$-O,
wherein $Ra_2$ is selected from $C_{1-6}$ alkyl, halogenated $C_{1-6}$ alkyl, substituted or unsubstituted aryl, and the like.

The peptide coupling agent refers to agents capable of activating carboxylic acid to form amides with amines, and includes, but is not limited to, 1-(3-dirrethylaminopropyl) - 3-ethylcarbodiimide hydrochloride, 2-(7-azabenzotriazol)-N,N,A",A"-tetramethyluronium hexafluoropho sphate, *O*-benzotriazol-tetramethyluro nium hexafluorophosphate, *N,N*-carbonyldiimidazole, benzotriazol-1-yloxytris(dimethylamino)phosphonium hexafluorophosphate, propylphosphoric anhydride, carbodiimide, and the like.

## Examples

For the abbreviations used herein, "DIPEA" refers to *N*,*N*-diisopropylethylamine, "EA" refers to ethyl acetate, "PE" refers to petroleum ether, "DCM" refers to dichloromethane, and "THF" refers to tetrahydrofuran.

**Preparation example 1: synthesis of intermediate 4-methoxy-4-methylpiperidine - trifluoroacetate**

Step 1: synthesis of *tert*-butyl 4-hydroxy-4-methylpiperidine-1-carboxylate

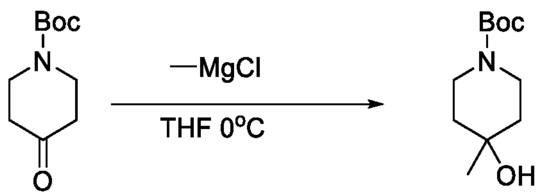


The starting material *tert-butyl* 4-oxopiperidine-1-carboxylate (5.0 g, 0.025 mol, 1.0 eq) was dissolved in tetrahydrofuran (25 mL), and then the reagent methylmagnesium chloride (9 mL, 0.0055 mol, 1.1 eq) was added at 0 °C under nitrogen atmosphere. Then the reaction mixture was reacted for 2 h. After the reaction completion as detected by TLC, the reaction mixture was adjusted to pH = 4 with diluted hydrochloric acid, added with water (30 mL), and then extracted with ethyl acetate (30 mL × 3). The organic phase was dried, filtered and concentrated under reduced pressure to give a crude product, which was purified by silica gel column chromatography (PE:EA = 5:1) to give *tert*-butyl 4-hydroxy-4-methylpiperidine-1-carboxylate (5.2 g, yield: 96%) in the form of a yellow liquid.

Step 2: synthesis of *tert-butyl* 4-methoxy-4-methylpiperidine-1-carboxylate

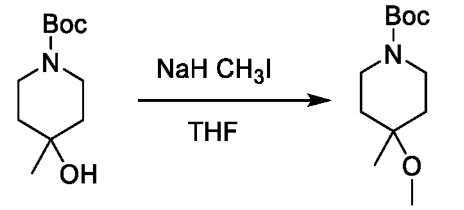


The starting material *tert*-butyl 4-hydroxy-4-methylpiperidine-1-carboxylate (500 mg, 2.32 mmol, 1.0 eq) was dissolved in tetrahydrofuran (5 mL), and sodium hydride (186 mg, 4.64 mmol, 2.0 eq) was added. After reaction for 1 h, the reaction mixture was added with iodomethane (659 mg, 4.64 mmol, 2.0 eq) and reacted for 8 h. After the reaction completion as detected by TLC, the reaction flask was added with water (10 mL), and the reaction mixture was extracted with ethyl acetate (20 mL × 3). The organic phase was dried, filtered and concentrated under reduced pressure to give a crude product, which was purified by silica gel column chromatography (PE:EA = 20:1) to give *tert*-butyl 4-methoxy-4-methylpiperidine-1-carboxylate (306 mg, yield: 57%) in the form of a yellow liquid.

Step 3: synthesis of 4-methoxy-4-methylpiperidine-trifluoroacetate

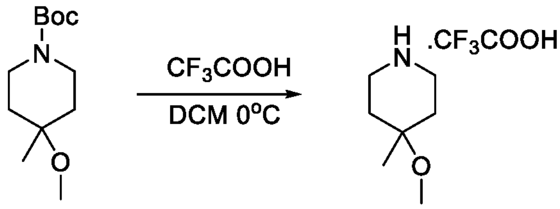


The starting material *tert*-butyl 4-methoxy-4-methylpiperidine-1-carboxylate (500 mg, 2.18 mmol, 1.0 eq) was dissolved in dichloromethane (4 mL) and trifluoroacetic acid (3 mL) was added at 0 °C, and the reaction mixture was reacted for 1 h. After the reaction completion as detected by TLC, the reaction mixture was directly concentrated under reduced pressure to give 4-methoxy-4-methylpiperidine-trifluoroacetate (530 mg, yield: 100%) in the form of a yellow liquid.

Preparation Example 2: synthesis of intermediate *tert*-butyl 4-hydroxy-4-(methyl-$d_3$)piperidine-1-carboxylate

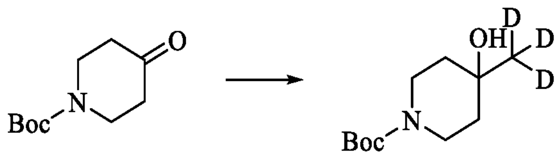

Mg (4.40 g, 181.1 mmol, 1.9 eq), $I_2$(0.20 g, 0.79 mmol) and $Et_2O$ (200 mL) were added to a reaction flask, and $CD_3I$ (26.19 g, 180.7 mmol, 1.8 eq) was added dropwise under nitrogen atmosphere, and the reaction mixture was reacted at room temperature for 2.5 h. *Tert*-butyl 4-oxopiperdinone-1-carboxylate (20.0 g, 100.38 mmol, 1.0eq) was added into 2-methyltetrahydrofuran (160 mL), and then the mixture was cooled to -30 °C to -40 °C and added dropwise with the prepared Grignard reagent, and the temperature was maintained below -30 °C. After the dropwise addition, the resulting reaction mixture was reacted at room temperature for 18 h. After the reaction completion as detected by TLC, saturated ammonium chloride solution (400 mL) and ethyl acetate (400 mL) were added to the reaction mixture, and liquid separation was performed. The aqueous phase was extracted with ethyl acetate (300 mL × 2), and the organic phases were combined, dried over anhydrous sodium sulfate and filtered under vacuum The filtrate was concentrated to give a crude product, which was purified by silica gel column chromatography (PE:EA = 3:1) to give the product (16 g, yield: 73%).

**Preparation Example 2: synthesis of intermediates 6-ethyl-4-chloro-2-oxo-1, 2-dihydro-1,7-naphthyridine-3-carbonitrile and 2,4-dichloro-6-ethyl-1,7-naphthyridine -3-carbonitrile**

Step 1: synthesis of methyl 6-ethyl-3-(cyanoacetamido)-1-pyridine-4-formate

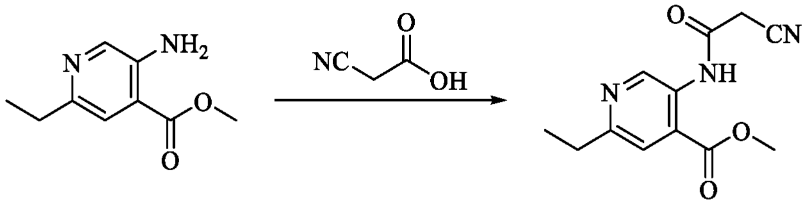

Intermediate methyl 6-ethyl-3-anino-1-pyridine-4-formate (131 g, 727.13 mmol, 1.0 eq) was dissolved in dichloromethane (1.31 L), and then cyanoacetic acid (74.22 g, 872.56 mmol, 1.2 eq) was added under an ice bath, and EDCI (209.07 g, 1090.70 mmol, 1.5 eq) was added in portions. Then the reaction mixture was reacted at 25 °C for 2 h. After the reaction completion as detected by LC-MS, $H_2O$ (1.5 L) was added to the reaction mixture, and liquid separation was performed. The organic phase was washed with $H_2O$ (800 mL × 2), dried over anhydrous sodium sulfate and filtered under vacuum. The filtrate was concentrated to give a crude product, which was slurried with methyl *tert-butyl* ether (500 mL) to give the product (165 g, yield: 91.78%).

Step 2: synthesis of 6-ethyl-4-hydroxy-2-oxo-1,2-dihydro-1,7-naphthyridine-3-carbonitrile

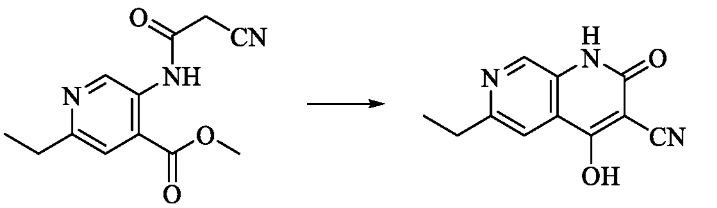

Intermediate methyl 6-ethyl-3-(cyanoacetamido)-1-pyridine-4-formate (165 g, 667.34 mmol, 1.0 eq) was dissolved in ethanol (1.65 L), and sodium ethoxide (136.24 g, 2002.62 mmol, 3.0 eq) was added in portions under an ice bath. After the addition, the reaction mixture was reacted at 25 °C for 2 h. After the reaction completion as detected by LC-MS, the reaction mixture was concentrated, added with $H_2O$ (1.5 L), and adjusted to pH below 4 with concentrated hydrochloric acid under an ice bath, and many light yellow solids were precipitated. The resulting mixture was filtered under vacuum, and the filter cake was dried to give the product (138 g, yield: 96.09%).

Step 3: synthesis of 6-ethyl-4-chloro-2-oxo-1,2-dihydro-1,7-naphthyridine-3-carbonitrile and 2,4-dichloro-6-ethyl-1,7-naphthyridine-3-carbonitrile

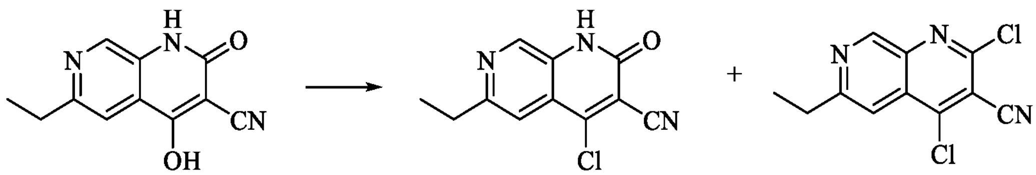

Intermediate 6-ethyl-4-hydroxy-2-oxo-1,2-dihydro-1,7-naphthyridine-3-carbonitrile (605 g, 2.81 mol, 1.0 eq) was dissolved in acetonitrile (3 L), and phosphorus oxychloride (1723 g, 11.24 mol, 4.0 eq) was added under an ice bath, and the reaction mixture was reacted at 100 °C for 2 h. Then the reaction mixture was cooled down, concentrated, added with acetonitrile (1 L) for dispersion, poured into ice water, and adjusted to pH of about 5-6 with saturated sodium

hydroxide solution, and many yellow solids were precipitated. The resulting mixture was filtered under vacuum and dried to give a crude product, which was slurried with *n*-heptane/ethyl acetate (3 L/0.6 L), and filtered under vacuum to give 6-ethyl-4-chloro-2-oxo-1,2-dihydro-1,7-naphthyridine-3-carbonitrile (510 g, yield: 78%).

The filtrate was concentrated to give a crude product, which was purified by silica gel column chromatography (PE:EA = 10:1) to give 2,4-dichloro-6-ethyl-1,7-naphthyridine- 3-carbonitrile (50 g, yield: 7%).

**Example 1: synthesis of 6-isopropyl-4-(4-methoxy-4-methylpiperidin-1-yl)-2- oxo-1,2-dihydro-1,7-naphthyridine-3-carbonitrile (Compound 163)**

Step 1: synthesis of 6-chloro-2*H*-pyrido[3,4-*d*][1,3]oxazine-2,4(1*H*)-dione

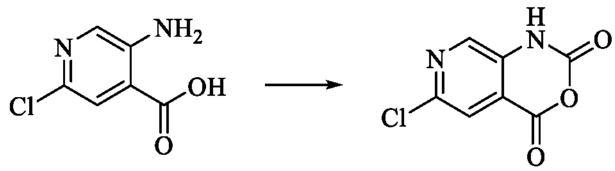

5-amino-2-chloroisonicotinic acid (30 g, 0.1738 mol, 1.0 eq) was dissolved in *N,N*-dimethylformamide (300 mL), and *N,N'*-carbonyldiimidazole (48 g, 0.2955 mol, 1.7 eq) was added in portions at 0 °C, and the reaction mixture was slowly warmed to room temperature overnight. After the reaction completion as detected by LC-MS, the reaction mixture was cooled to room temperature and directly used in the next step without further treatment.

Step 2: synthesis of 6-chloro-4-hydroxy-2-oxo-1,2-dihydro-1,7-naphthyridine-3 - carbonitrile

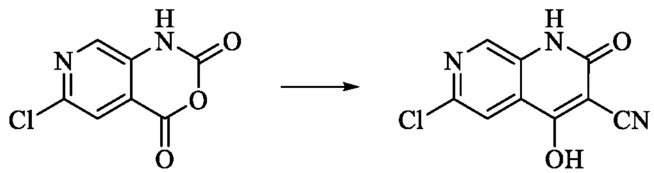

The reaction mixture above was added with triethylamine (35.182 g, 0.3478 mol, 2 eq) and ethyl cyanoacetate (19.665 g, 0.1738 mol) and then reacted for 3 h at 150 °C. After the reaction completion as detected by LC-MS, the reaction mixture was cooled to room temperature, concentrated under reduced pressure, added with water (200 mL), adjusted to pH= 1 with hydrochloric acid (1 mol/L), stirred for 15 min, and filtered under vacuum The filter cake was washed twice with EA and dried at 40 °C to give the product (25.655 g, yield: 66%) in the form of a light brick red solid.

Step 3: synthesis of 4,6-dichloro-2-oxo-1,2-dihydro-1,7-naphthyridine-3-carbonitrile

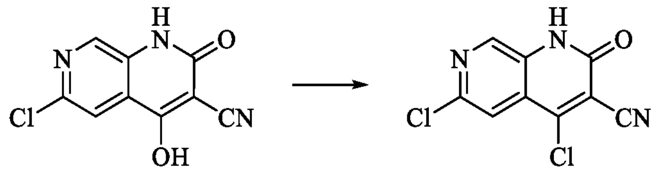

6-chloro-4-hydroxy-2-oxo-1,2-dihydro-1,7-naphthyridine-3-carbonitrile (5.0 g, 0.0226 mol, 1 eq) and phosphorus oxychloride (15 mL) were added to a reaction flask, which was then placed in an oil bath that had been heated to 100 °C. After reaction for about 6 min, the solid began to slowly dissolve, and the color slowly deepened from light yellow. After reaction completion as detected by TLC, the resulting mixture was cooled to room temperature, and an appropriate amount of DCM was added into the flask. The mixture was poured into ice water (100 mL), stirred for 10 min and filtered

under vacuum The filter cake was washed with methyl *tert-butyl* ether, filtered under vacuum, and dried under vacuum at 40 °C to give the product in the form of a light yellow solid. A total of 25.655 g (0.1157 mol) of 6-chloro-4-hydroxy-2-oxo-1,2-dihydro-1,7-naphthyridine-3 -carbonitrile was added in five portions to give the product (19.486 g, yield: 70.1%).

Step 4: synthesis of 6-chloro-4-(4-methoxy-4-methylpiperidin-1-yl)-2-oxo-1, 2-dihydro-1,7-naphthyridine-3-carbonitrile

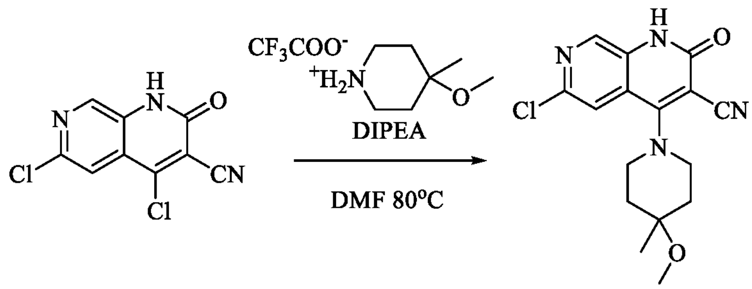


Intermediate 4,6-dichloro-2-oxo-1,2-dihydro-1,7-naphthyridine-3-carbonitrile (2.0 g, 8.33 mmol, 1.0 eq) was dissolved in DMF (10 mL), and DIPEA (6.45 g, 50 mmol, 6.0 eq) and 4-methoxy-4-methylpiperidine trifluoroacetate (2.2 g, 9.16 mmol, 1.1 eq) were added, and the reaction mixture was reacted at 80 °C for 2 h. After the reaction completion as detected by LC-MS, the reaction mixture was added with water (10 mL) and extracted with dichloromethane (10 mL × 3). The organic phase was washed with water (10 mL × 3), dried over anhydrous sodium sulfate, filtered, and concentrated under reduced pressure to give the product (2.7 g of crude product) in the form of a yellow solid.

Step 5: synthesis of 4-(4-methoxy-4-methylpiperidin-1-yl)-2-oxo-6-(prop-1-en-2-yl)-1, 2-dihydro-1,7-naphthyridine-3-carbonitrile

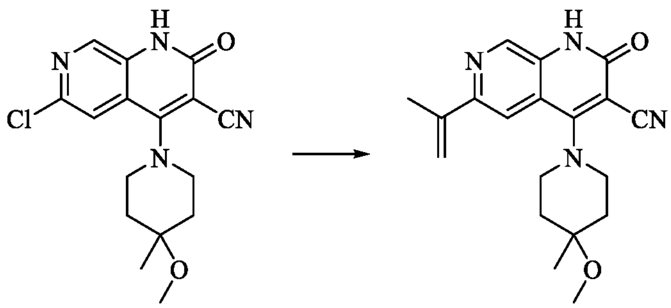

Intermediate 6-chloro-4-(4-methoxy-4-methylpiperidin-1-yl)-2-oxo-1,2-dihydro-1, 7-naphthyridine-3-carbonitrile (500 mg, 1.5 mmol, 1.0 eq) was dissolved in 1,4-dioxane (5 mL) and $H_2O$ (1 mL), and potassium trifluoro(prop-1-en-2-yl)borate (668 mg,4.5 mmol, 3.0 eq) and cesium carbonate (1.466 g, 4.5 mmol, 3.0 eq) were added, and then [1,1'-bis(diphenylphosphino)ferrocene]palladium dichloride (110 mg, 0.15 mmol, 0.1 eq) was added under nitrogen atmosphere. Then the reaction mixture was reacted at 100 °C for 12 h. After the reaction completion as detected by LC-MS, the reaction mixture was added with water (20 mL) and extracted with ethyl acetate (20 mL × 3). The organic phase was dried over anhydrous sodium sulfate and filtered, and the filtrate was concentrated under reduced pressure to give a crude product, which was purified by silica gel column chromatography (DCM:MeOH = 50:1) to give the product (390 mg, yield: 76.9%).

Step 6: synthesis of 6-isopropyl-4-(4-methoxy-4-methylpiperidin-1-yl)-2-oxo-1,2-dihydro-1,7-naphthyridine-3-carbonitrile

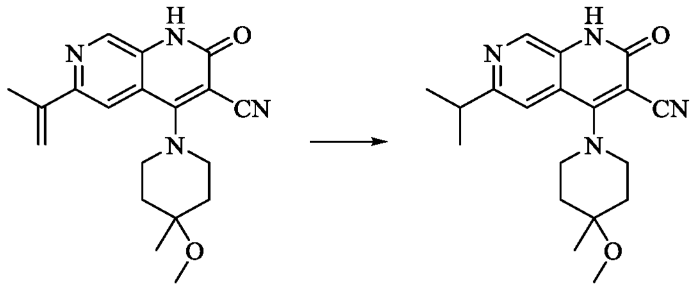

Compound 163

Intermediate 4-(4-methoxy-4-methylpiperidin-1-yl)-2-oxo-6-(prop-1-en-2-yl)-1,2-dihydro-1,7-naphthyridine-3-carbonitrile (390 mg, 1.15 mmol, 1.0 eq) was dissolved in methanol (10 mL), and Pd/C (100 mg) was added, and the reaction mixture was reacted for 12 h under hydrogen atmosphere. After the reaction completion as detected by LC-MS, the reaction mixture was filtered under vacuum, and the filtrate was concentrated under reduced pressure to give a crude product, which was slurried with methyl *tert-butyl* ether and filtered under vacuum to give a crude product, which was separated by preparative thin-layer chromatography (DCM:MeOH = 15:1) to give the product (100 mg, yield: 25.6%).

$^1$HNMR (400 MHz, DMSO-$d_6$) δ (ppm): 11.82 (s, 1H), 8.61(s, 1H), 7.38 (s, 1H), 3.60-3.61 (m, 4H), 3.19 (s, 3H), 3.06-3.13 (m, 1H), 1.90-1.93 (m, 2H), 1.75-1.82 (d, 2H),1.26 (s, 9H).
Molecular formula: $C_{19}H_{24}N_4O_2$; Molecular weight: 340.43; LC-MS (Pos, *m/z*) = 341.19[M+H]$^+$.

**Example 2: synthesis of 6-cyclopropyl-4-(4-methoxy-4-methylpiperidin-1-yl)-2- oxo-1,2-dihydro-1,7-naphthyridine-3-carbonitrile (Compound 164)**

Step 1: synthesis of ethyl 2-chloro-5-nitroisonicotinate

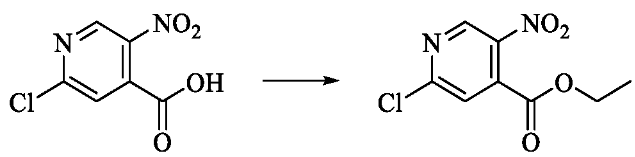

2-chloro-5-nitroisonicotinic acid (20.0 g, 98.74 mmol, 1.0 eq) was dissolved in triethyl orthoformate (43.9 g, 296.20 mmol, 3.0 eq), and the reaction mixture was reacted at 120 °C for 3 h. After starting material disappearance as detected by TLC, the reaction mixture was concentrated under reduced pressure to give a yellow oily liquid, which was added with petroleum ether (150 mL), stirred for 12 h, and filtered. The filter cake was dried at room temperature to give the product (11.0 g, yield: 51.6%).

Step 2: synthesis of ethyl 2-cyclopropyl-5-nitroisonicotinate

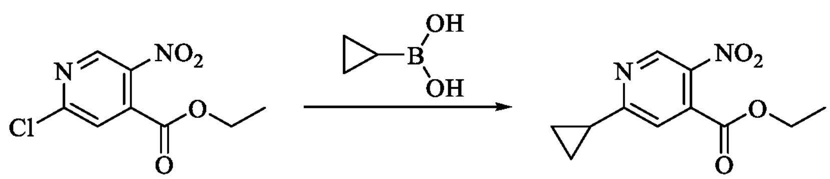

Ethyl 2-chloro-5-nitroisonicotinate (11.0 g, 47.70 mmol, 1.0 eq), cyclopropylboronic acid (10.2 g, 119.25 mmol, 2.5 eq) and potassium phosphate (35.4 g, 166.95 mmol, 3.5 eq) were dissolved in a mixed solvent of water (27.5 mL) and toluene (275 mL), and triphenylphosphine (2.5 g, 9.54 mmol, 0.2 eq) and palladium acetate (1.1 g, 4.77 mmol, 0.1 eq) were added under nitrogen atmosphere. After purge with nitrogen for three times, the reaction mixture was reacted at reflux for 24 h. After reaction completion as detected by TLC, the reaction mixture was concentrated under reduced pressure to give a crude product, which was purified by silica gel column chromatography (EA:PE = 1:30) to give the

product (6.18 g, yield: 55%).

Step 3: synthesis of ethyl 5-amino-2-cyclopropylisonicotinate

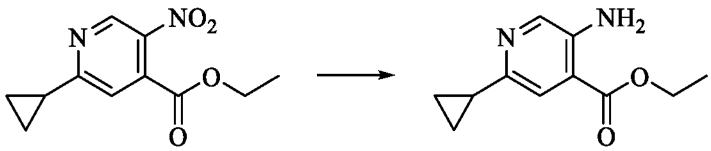

Intermediate ethyl 2-cyclopropyl-5-nitroisonicotinate (6.18 g, 26.16 mmol, 1.0 eq) was dissolved in absolute ethanol (60 mL), and iron powder (5.86 g, 104.64 mmol, 4.0 eq) was added. The reaction mixture was heated to reflux, added dropwise with acetic acid (9.4 g, 156.96 mmol, 6.0 eq), and reacted at reflux for 3 h. After the reaction completion as detected by TLC, the reaction mixture was added with ethyl acetate (100 mL) and filtered while hot. The filter cake was rinsed with ethyl acetate, and the filtrate was concentrated under reduced pressure, added with water (50 mL) and ethyl acetate (100 mL), cooled down in an ice water bath, and adjusted to pH of about 8 with solid sodium bicarbonate. Liquid separation was performed. The aqueous phase was extracted with ethyl acetate (50 mL × 3), and the organic phases were combined, dried over anhydrous magnesium sulfate and filtered, and the filtrate was concentrated under reduced pressure to give the product (4.77 g, yield: 90%).

Step 4: synthesis of ethyl 5-(2-cyanoacetamido)-2-cyclopropylisonicotinate

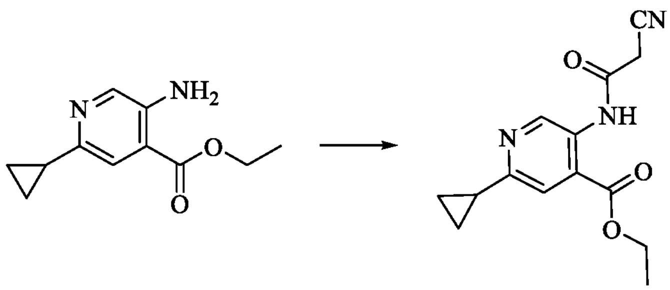

Intermediate ethyl 5-amino-2-cyclopropylisonicotinate (4.77 g, 23.13 mmol, 1.0 eq) was dissolved in dichloromethane (60 mL), and 1-(3-dimethylaminopropyl)-3-ethylcarbodiimide hydrochloride (8.7 g, 46.25 mmol, 2.0 eq) and cyanoacetic acid (3 g, 34.69 mmol, 1.5 eq) were added, and the reaction mixture was stirred at room temperature for 16 h. After starting material disappearance as detected by TLC, the reaction mixture was added with dichloromethane (40 mL) and washed with water (50 mL × 2). The organic phase was washed with saturated aqueous sodium carbonate solution (50 mL), dried over anhydrous magnesium sulfate and filtered, and the filtrate was concentrated under reduced pressure to give the product (5.6 g, yield: 100%), which was used in the next step according to a theoretical amount.

Step 4: synthesis of 6-cyclopropyl-4-hydroxy-2-oxo-1,2-dihydro-1,7-naphthyridine-3-carbonitrile

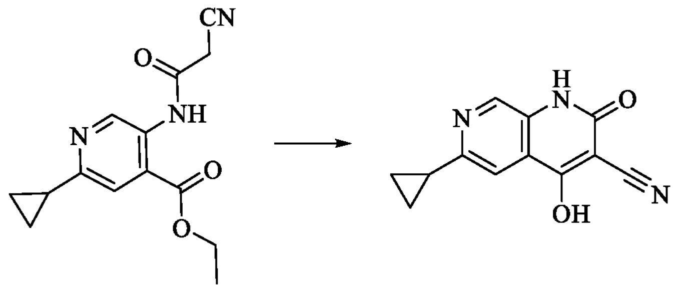

Intermediate ethyl 5-(2-cyanoacetamido)-2-cyclopropylisonicotinate (5.6 g, 20.51 mmol, 1.0 eq) was dissolved in absolute ethanol (100 mL), and then the reaction mixture was stirred for 10 min, added with sodium ethoxide (4.7 g, 69.39 mmol, 3.0 eq) and stirred at room temperature for 1 h. After the presence of remaining starting material as detected by TLC, the reaction mixture was supplemented with sodium ethoxide (4.7 g, 69.39 mmol, 3.0 eq) and stirred at room temperature for 2 h. After starting material disappearance as detected by TLC, the reaction mixture was concentrated under reduced pressure, added with water (200 mL) and extracted with methyl *tert-butyl* ether (100 mL × 2). The aqueous phase was cooled down with ice water and adjusted to pH = 1-2 with concentrated hydrochloric acid, and solids were precipitated. The resulting mixture was filtered, and the filter cake was rinsed with water and dried to give the product (3.95 g, yield: 84.84%).

Step 5: synthesis of 2,4-dichloro-6-cyclopropyl-1,7-naphthyridine-3-carbonitrile

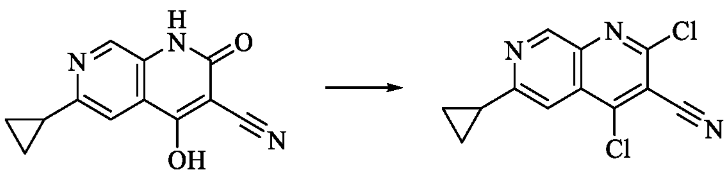

Intermediate 6-cyclopropyl-4-hydroxy-2-oxo-1,2-dihydro-1,7-naphthyridine- 3 - carbonitrile (1 g, 4.4 mmol, 1.0 eq) was dissolved in anhydrous acetonitrile (15 mL), and phosphorus oxychloride (1.35 g, 8.8 mmol, 2.0 eq) was added, and the reaction mixture was warmed to 80 °C and reacted for 1 h. After the presence of most of the starting material as detected by TLC, the reaction mixture was supplemented with phosphorus oxychloride (1.35 g, 8.8 mmol, 2.0 eq) and heated to 90 °C. After the reaction completion as detected by LC-MS, the reaction mixture was cooled to room temperature, concentrated under reduced pressure, added with acetonitrile (10 mL), cooled down with ice water and adjusted to pH= 8-9 with sodium hydroxide solution, and yellow solids were precipitated. The resulting mixture was filtered, and the filter cake was rinsed with water to give the product (1.5 g of crude product), which was used in the next step according to a theoretical amount.

Step 6: synthesis of 4-chloro-6-cyclopropyl-2-oxo-1,2-dihydro-1,7-naphthyridine-3-carbonitrile

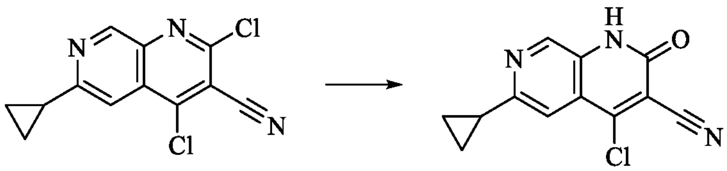

Intermediate 2,4-dichloro-6-cyclopropyl-1,7-naphthyridine-3-carbonitrile (1.16 g, crude product, 4.40 mmol, 1.0 eq) was dissolved in a mixed solvent of trifluoroacetic acid (10 mL) and water (2.5 mL), and the reaction mixture was heated to 60 °C and reacted for 18 h. Then the reaction mixture was cooled to 0 °C, added with water (20 mL) and adjusted to pH = 8-9 with solid sodium hydroxide, and yellow solids were precipitated. The resulting mixture was filtered, and the filter cake was rinsed with water, dried and added with ethyl acetate (10 mL). The mixture was heated to 60 °C,

stirred for 1 h and filtered while hot, and the filter cake was dried to give the product (660 mg, two-step yield: 61%).

Step 7: synthesis of 2-chloro-6-cyclopropyl-4-(4-methoxy-4-methylpiperidin-1-yl) - 1,7-naphthyridine-3-carbonitrile

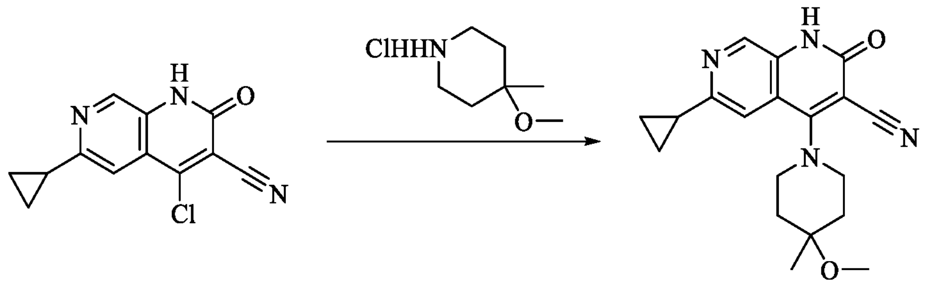

Compound 164

Intermediate 4-chloro-6-cyclopropyl-2-oxo-1,2-dihydro-1,7-naphthyridine-3-carbonitrile (200 mg, 0.81 mmol, 1.0 eq) was dissolved in *N,N*-dimethylacetamide (2 mL), and DIPEA (420.5 mg, 3.26 mmol, 4.0 eq) and 4-methoxy-4-methylpiperidine hydrochloride (188.8 mg, 1.14 mmol, 1.4 eq) were added, and the reaction mixture was heated to 80 °C and reacted for 1 h. After starting material disappearance as detected by TLC, the reaction mixture was cooled to room temperature, poured into ice water (20 mL) and extracted with ethyl acetate (50 mL × 3). The organic phases were combined, washed with water (50 mL × 2), dried over anhydrous magnesium sulfate and filtered, and the filtrate was concentrated under reduced pressure to give a crude product, which was slurried with methyl *tert-butyl* ether (5 mL) for 1 h and filtered under vacuum. The filter cake was dried to give the product (182 mg, yield: 66%).

$^1$HNMR (400 MHz, DMSO-$d_6$) δ (ppm): 11.85 (s, 1H), 8.51 (d, 1H), 7.45 (s, 1H), 3.61-3.60 (d, 4H), 3.19 (s, 3H), 2.27-2.21 (m, 1H), 1.93-1.90(m, 2H), 1.84-1.80(m, 2H), 1.22 (s, 3H), 0.95 (m, 2H), 0.85 (m, 2H).
Molecular formula: $C_{19}H_{22}N_4O_2$; Molecular weight: 338.17; LC-MS (Pos, *m/z*) = 339.13[M+H]$^+$.

**Example 3: synthesis of 4-(4-methoxy-4-methylpiperidin-1-yl)-2-oxo-1,2-dihydro-1,5-naphthyridine-3-carbonitrile (Compound 165)**

Step 1: synthesis of methyl 3-aminopicolinate

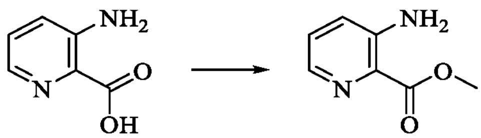

3-aminopicolinic acid (10.0 g, 72.39 mmol, 1.0 eq) was dissolved in anhydrous methanol (100 mL), and sulfuric acid (10 mL) was added dropwise. After the addition, the reaction mixture was heated to 80 °C and reacted for 144 h. After the presence of 20% of the starting material as detected by LC-MS, the reaction mixture was concentrated under reduced pressure, added with ethyl acetate (100 mL) and adjusted to pH of about 9 with saturated aqueous sodium carbonate solution, and liquid separation was performed. The aqueous phase was extracted with ethyl acetate (100 mL × 3), and the organic phases were combined, dried over anhydrous sodium sulfate, filtered and concentrated under reduced pressure to give the product (5.3 g, yield: 48%).

Step 2: synthesis of methyl 3-(2-cyanoacetamido)picolinate

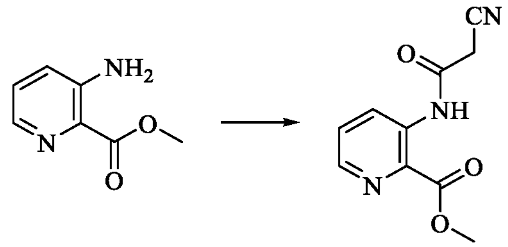

Intermediate methyl 3-aminopicolinate (5.3 g, 34.83 mmol, 1.0 eq) was dissolved in dichloromethane (60 mL), and 1-(3-dimethylaminopropyl)-3-ethylcarbodiimide hydrochloride (10 g, 52.25 mmol, 1.5 eq) and cyanoacetic acid (3.6 g, 41.79 mmol, 1.2 eq) were added. After the addition, the reaction mixture was stirred at room temperature for 1 h. After starting material disappearance as detected by TLC, the reaction mixture was added with dichloromethane (100 mL) and water (50 mL), and stirred for 10 min, and solids were precipitated. The resulting mixture was filtered, and the filter cake was collected and dried to give a part of the product (3.3 g), and the filtrate was subjected to liquid separation. The aqueous phase was extracted with dichloromethane (50 mL), and the organic phases were combined, dried over anhydrous magnesium sulfate and filtered, and the filtrate was concentrated under reduced pressure to give a crude product, which was slurried with methyl *tert-butyl* ether, filtered and dried to give the other part of the product (2.68 g). The two parts were combined to give the product (6.0 g, yield: 78.9%).

Step 3: synthesis of 4-hydroxy-2-oxo-1,2-dihydro-1,5-naphthyridine-3-carbonitrile

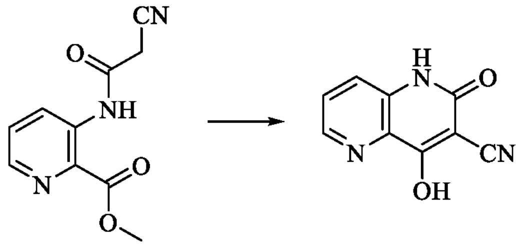

Intermediate methyl 3-(2-cyanoacetamido)picolinate (2.68 g, 12.23 mmol, 1.0 eq) was dissolved in absolute ethanol (100 mL), and the reaction mixture was heated to 50 °C, stirred for 0.5 h, and then added with sodium ethoxide (2.5 g, 36.67 mmol, 3.0 eq). After the addition, the reaction mixture was stirred at 50 °C for 1 h. After starting material disappearance as detected by TLC, the reaction mixture was concentrated under reduced pressure, added with water (50 mL), and adjusted to pH of about 2 with concentrated hydrochloric acid, and solids were precipitated. The resulting mixture was then filtered, and the filter cake was washed successively with water and acetone, and then dried to give the product (2.28 g, yield: 100%).

Step 4: synthesis of 2,4-dichloro-1,2-dihydro-1,5-naphthyridine-3-carbonitrile

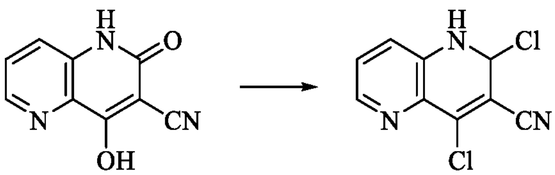

Intermediate 4-hydroxy-2-oxo-1,2-dihydro-1,5-naphthyridine-3-carbonitrile (1.5 g, 8.02 mmol, 1.0 eq) was added to phosphorus oxychloride (6.5 g, 10 mL), and after the addition, the reaction mixture was heated to 110 °C and reacted for 2 h. After starting material disappearance as detected by LC-MS, the reaction mixture was cooled to room temperature, poured into ice water (100 mL) and stirred for 10 min, and yellow solids were precipitated. The resulting mixture was filtered, and the filter cake was rinsed with water and dried to give the product (1.5 g, yield: 83.7%).

Step 5: synthesis of 4-chloro-2-oxo-1,2-dihydro-1,5-naphthyridine-3-carbonitrile

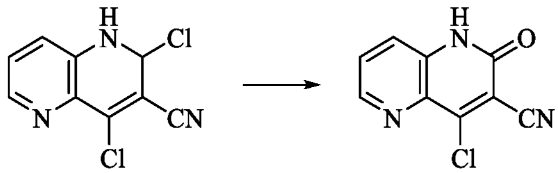

Intermediate 2,4-dichloro-1,2-dihydro-1,5-naphthyridine-3-carbonitrile (1.5 g, 6.69 mmol, 1.0 eq) was dissolved in a mixed solvent of trifluoroacetic acid (15 mL) and water (4 mL), and the reaction mixture was heated to 60 °C and reacted for 16 h. After starting material disappearance as detected by LC-MS, the reaction mixture was cooled to room temperature and poured into ice water (50 mL), and yellow solids were precipitated. The resulting mixture was filtered, and the filter cake was rinsed successively with water and acetone, and dried to give the product (822 mg, yield: 60%).

Step 6: synthesis of 4-(4-methoxy-4-methylpiperidin-1-yl)-2-oxo-1,2-dihydro-1, 5-naphthyridine- 3 - carbonitrile

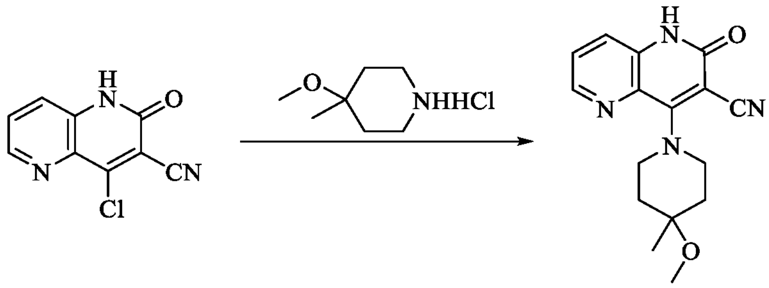

Compound 165

Intermediate 4-chloro-2-oxo-1,2-dihydro-1,5-naphthyridine-3-carbonitrile (500 mg, 2.43 mmol, 1.0 eq) was dissolved in *N*,*N*-dimethylacetamide (3 mL), and DIPEA (1.26 g, 9.72 mmol, 4.0 eq) and 4-methoxy-4-methylpiperidine hydrochloride (564 mg, 3.40 mmol, 1.4 eq) were added. After the addition, the reaction mixture was heated to 80 °C and reacted for 1 h. After starting material disappearance as detected by LC-MS, the reaction mixture was cooled to room temperature and poured into ice water (50 mL), and solids were precipitated. The solids were dissolved in dichloromethane, dried over anhydrous magnesium sulfate and filtered, and the filtrate was concentrated under reduced pressure, to give a crude product, which was added into a mixed solvent of ethyl acetate (10 mL) and methyl *tert-butyl* ether (10 mL), stirred for 48 h and filtered under vacuum. The filter cake was dried to give the product (190 mg, yield: 26.2%).

$^1$H NMR (400 MHz, DMSO-$d_6$) δ (ppm): 11.64 (s, 1H), 8.50-8.48 (d, 1H), 7.66-7.63 (d, 1H), 7.59-7.56 (d, 1H), 4.06-4.02 (m, 2H), 3.68-3.61 (m, 2H), 3.19 (s, 3H), 1.91-1.88 (m, 2H), 1.79-1.71 (m, 2H), 1.19 (s, 3H).
Molecular formula: $C_{16}H_{18}N_4O_2$; Molecular weight: 298.35; LC-MS (Pos, *m/z*) = 298.98[M+H]$^+$.

**Example 4: synthesis of 4-(4-methoxy-4-methylpiperidin-1-yl)-6-methyl-2-oxo-1,2-dihydro-1,5-naphthyridine-3-carbonitrile (Compound 166)**

Step 1: synthesis of ethyl 3-aminopicolinate

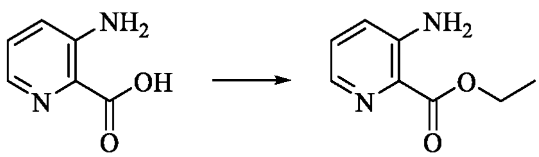

The starting material 3-aminopicolinic acid (18.0 g, 0.13 mol, 1.0 eq) was dissolved in absolute ethanol (300 mL), and sulfuric acid (20 mL) was added dropwise, and the reaction mixture was heated to 80 °C and reacted for 92 h. After the presence of 23% of the starting material as detected by LC-MS, the reaction mixture was concentrated under reduced pressure, added with ethyl acetate (100 mL) and water (200 mL), adjusted to pH of about 9 with aqueous potassium carbonate solution, and filtered. The filter cake was rinsed with ethyl acetate, and the filtrate was subjected to liquid separation. The aqueous phase was extracted with ethyl acetate (100 mL × 4), and the organic phases were combined, dried over anhydrous sodium sulfate and filtered. The filtrate was concentrated under reduced pressure to give a crude product, which was slurried with a mixed solvent of methyl *tert-butyl* ether (100 mL) and petroleum ether (100 mL) for 1 h and filtered. The filter cake was dried to give the product (9.2 g, yield: 42.5%).

Step 2: synthesis of ethyl 3-amino-6-bromopicolinate

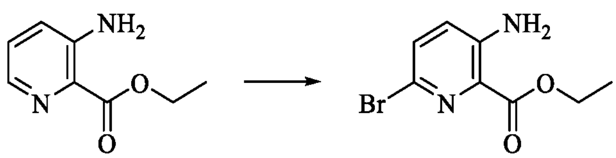

Intermediate ethyl 3-aminopicolinate (9.2 g, 55.36 mmol, 1.0 eq) was dissolved in acetonitrile (225 mL), and NBS (9.8 g, 53.36 mmol, 1.0 eq) was added, and the reaction mixture was reacted at room temperature for 2-3 h. After reaction completion as detected by TLC, the reaction mixture was concentrated under reduced pressure to give a crude product, which was purified by silica gel column chromatography (EA:PE = 1:3) to give the product (12 g, yield: 88.8%).

Step 3: synthesis of ethyl 6-bromo-3-(2-cyanoacetamido)picolinate

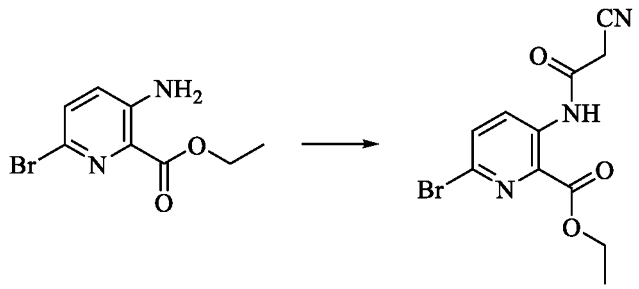

Intermediate ethyl 3-amino-6-bromopicolinate (3.0 g, 12.24 mmol, 1.0 eq) was dissolved in dichloromethane (50 mL), and 1-(3-dimethylaminopropyl)-3-ethylcarbodiimide hydrochloride (4.7 g, 24.48 mmol, 2.0 eq) and cyanoacetic acid (1.56 g, 18.36 mmol, 1.5 eq) were added, and the reaction mixture was stirred at room temperature for 1 h. After starting material disappearance as detected by TLC, the reaction mixture was added with dichloromethane (50 mL), washed successively with water (50 mL × 2) and saturated aqueous sodium carbonate solution (50 mL), dried over anhydrous magnesium sulfate and filtered, and the filtrate was concentrated under reduced pressure to give the product (3.2 g, yield: 84.2%).

Step 4: synthesis of 6-bromo-4-hydroxy-2-oxo-1,2-dihydro-1,5-naphthyridine-3 - carbonitrile

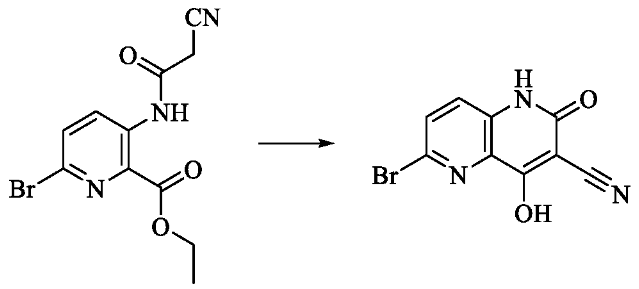

Intermediate ethyl 6-bromo-3-(2-cyanoacetamido)picolinate (3.2 g, 10.26 mmol, 1.0 eq) was dissolved in absolute ethanol (50 mL), and the reaction mixture was heated to 40 °C, added with sodium ethoxide (2.1 g, 30.79 mmol, 3.0 eq) and stirred at 40 °C for 1 h. After starting material disappearance as detected by TLC, the reaction mixture was concentrated under reduced pressure, added with water (50 mL) and adjusted to pH = 2-3 with concentrated hydrochloric acid, and solids were precipitated. The resulting mixture was filtered, and the filter cake was rinsed with water and dried to give the product (2.3 g, yield: 85.1%).

Step 5: synthesis of 2,4,6-trichloro-1,5-naphthyridine-3-carbonitrile

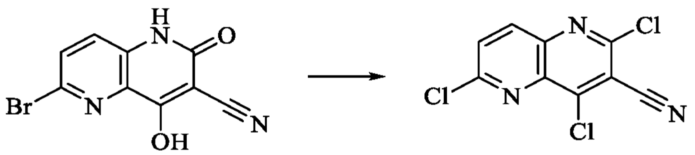

A reaction mixture of intermediate 6-bromo-4-hydroxy-2-oxo-1,2-dihydro-1, 5-naphthyridine-3-carbonitrile (2.3 g, 8.64 mmol, 1.0 eq), phosphorus oxychloride (6.6 g, 43.2 mmol, 5.0 eq) and phosphorus pentachloride (3.6 g, 17.28 mmol, 2.0 eq) was heated to 110 °C and reacted for 3 h. After starting material disappearance as detected by LC-MS, the reaction mixture was concentrated under reduced pressure, added with acetonitrile (10 mL) and adjusted to pH of about 8 with aqueous sodium hydroxide solution, and light yellow solids were obtained. The resulting mixture was filtered, and the filter cake was rinsed with water to give the product, which was used in the next step according to a theoretical amount.

Step 6: synthesis of 4,6-dichloro-2-oxo-1,2-dihydro-1,5-naphthyridine-3-carbonitrile

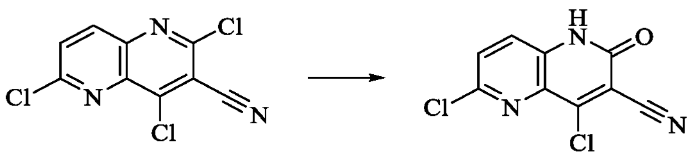

Intermediate 2,4,6-trichloro-1,5-naphthyridine-3-carbonitrile obtained in the previous step was dissolved in a mixed solvent of trifluoroacetic acid (22 mL) and water (5.5 mL), and the reaction mixture was heated to 90 °C and reacted for 2 h. After starting material disappearance as detected by LC-MS, the reaction mixture was cooled to room temperature and poured into ice water (50 mL), and yellow solids were precipitated. The resulting mixture was filtered. The filter cake was added with methanol (20 mL), heated to reflux for 1 h and filtered while hot, and the filtrate was concentrated under reduced pressure, slurried with ethyl acetate (20 mL) for 1 h and filtered. The filter cake was dried to give the product (311 mg, yield: 15%).

Step 7: synthesis of 6-chloro-4-(4-methoxy-4-methylpiperidin-1-yl)-2-oxo-1,2-dihydro-1,5-naphthyridine-3-carbonitrile

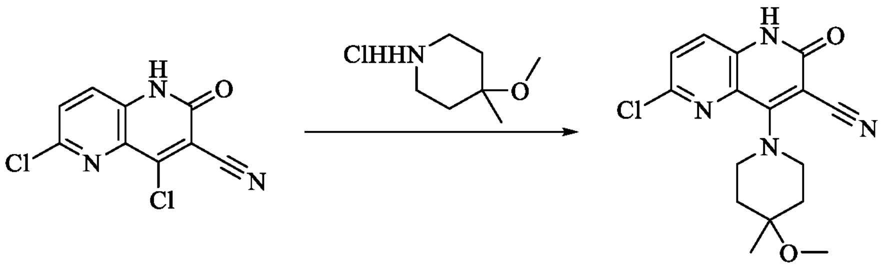


Intermediate 4-chloro-2-oxo-1,2-dihydro-1,5-naphthyridine-3-carbonitrile (310 mg, 1.29 mmol, 1.0 eq) was dissolved in *N*,*N*-dimethylacetamide (2 mL), and DIPEA (666.4 mg, 5.16 mmol, 4.0 eq) and 4-methoxy-4-methylpiperidine hydrochloride (299.5 mg, 1.81 mmol, 1.4 eq) were added. Then the reaction mixture was heated to 80 °C and reacted for 1 h. After starting material disappearance as detected by LC-MS, the reaction mixture was cooled to room temperature and poured into ice water (30 mL), and solids were precipitated. The resulting mixture was filtered, and the filter cake was rinsed with water and dried to give the product (260 mg, yield: 60.5%).

Step 8: synthesis of 4-(4-methoxy-4-methylpiperidin-1-yl)-6-methyl-2-oxo-1, 2-dihydro-1,5-naphthyridine-3-carbonitrile

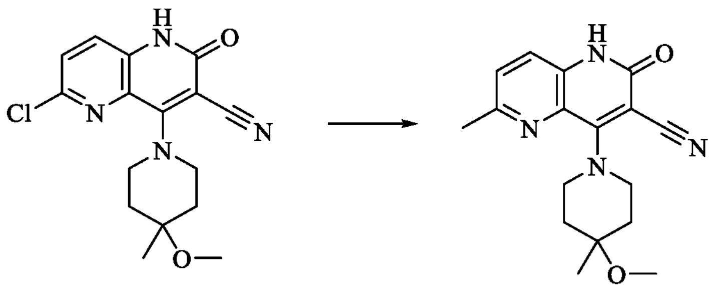

(Compound 166)

Intermediate 6-chloro-4-(4-methoxy-4-methylpiperidin-1-yl)-2-oxo-1,2-dihydro-1, 5-naphthyridine-3-carbonitrile (260 mg, 0.78 mmol, 1.0 eq) (mass fraction 50%) trimethylboroxine (784.5 mg, 3.12 mmol, 4.0 eq) and cesium carbonate (764.2 mg, 2.34 mmol, 3.0 eq) were dissolved in a mixed solvent of 1,4-dioxane (8 mL) and water (1.5 mL). After purge with nitrogen for three times, the reaction mixture was added with [1,1'-bis(diphenylphosphino)ferrocene]palladium dichloride (57.0 mg, 0.078 mmol and 0.1 eq) and reacted at 90 °C for 6 h under nitrogen atmosphere. After the reaction completion as detected by LC-MS, the reaction mixture was concentrated under reduced pressure to give a crude product, which was purified by silica gel column chromatography (DCM:MeOH = 80:1) to give a yellow solid, which was slurried with methyl *tert-butyl* ether and filtered. The filter cake was dried to give the product (160 mg, yield: 65.6%).

$^1$H NMR (400 MHz, DMSO-$d_6$) δ (ppm): 11.57 (s, 1H), 7.56-7.53 (d, 1H), 7.47-7.45 (d, 1H), 4.06-4.02 (d, 2H), 3.65-3.60 (m, 2H), 3.19 (s, 3H), 2.52 (s, 3H), 1.91-1.88 (m, 2H), 1.80-1.73 (m, 2H), 1.19 (s, 3H).
Molecular formula: $C_{17}H_{20}N_4O_2$; Molecular weight: 312.37; LC-MS (Pos, *m*/*z*) = 313.40[M+H]$^+$.

**Example 5: synthesis of 6-ethyl-4-(4-methoxy-4-methylpiperidin-1-yl)-2-oxo-1, 2-dihydro-1,5-naphthyridine-3-carbonitrile (Compound 167)**

Step 1: synthesis of ethyl 3-aminopicolinate

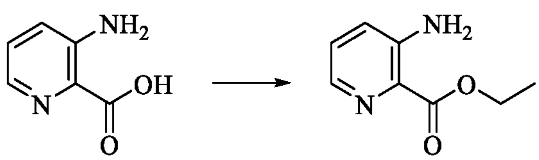

The starting material 3-aminopicolinic acid (20.0 g, 0.14 mol, 1.0 eq) was dissolved in absolute ethanol (300 mL), and sulfuric acid (10 mL) was added dropwise, and the reaction mixture was heated to 80 °C and reacted for 16 h. After the presence of 90% of the starting material as detected by LC-MS, the reaction mixture was supplemented with absolute ethanol (300 mL), added dropwise with sulfuric acid (10 mL), and reacted at reflux for 24 h. After the presence of 30% of the starting material as detected by LC-MS, the reaction mixture was concentrated under reduced pressure, added with ethyl acetate (300 mL) and water (200 mL), adjusted to pH of about 9 with aqueous potassium carbonate solution, and filtered. The filter cake was rinsed with ethyl acetate, and liquid separation was performed. The aqueous phase was extracted with ethyl acetate (100 mL × 3), and the organic phases were combined, dried over anhydrous sodium sulfate, filtered and concentrated under reduced pressure to give the product (10 g, yield: 41.8%).

Step 2: synthesis of ethyl 3-amino-6-bromopicolinate

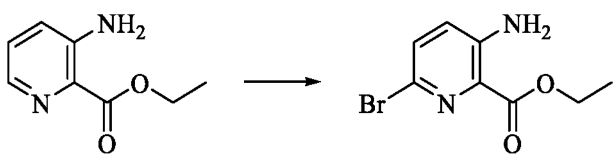

Intermediate ethyl 3-aminopicolinate (6 g, 36.11 mmol, 1.0 eq) was dissolved in acetonitrile (150 mL), and NBS (7.1 g, 39.72 mmol, 1.1 eq) was added, and the reaction mixture was reacted at room temperature for 48 h. After reaction completion as detected by TLC, the reaction mixture was concentrated under reduced pressure to give a crude product, which was purified by silica gel column chromatography (EA:PE = 1:10-1:6) to give the product (6.28 g, yield: 71.3%).

Step 3: synthesis of ethyl 6-bromo-3-(2-cyanoacetamido)picolinate

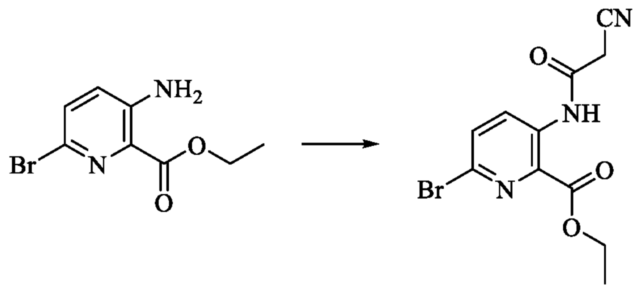

Intermediate ethyl 3-amino-6-bromopicolinate (6.28 g, 25.6 mmol) was dissolved in dichloromethane (100 mL), and 1-(3-dimethylaminopropyl)-3-ethylcarbodiimide hydrochloride (9.8 g, 51.25 mmol, 2.0 eq) and cyanoacetic acid (3.3 g, 38.45 mmol, 1.5 eq) were added, and the reaction mixture was stirred at room temperature for 1 h. After starting material disappearance as detected by TLC, the reaction mixture was added with dichloromethane (50 mL) and washed successively with water (80 mL × 2) and saturated aqueous sodium carbonate solution (80 mL), and solids were precipitated. The resulting mixture was filtered, and the filtrate was subjected to liquid separation. The organic phase was dried over anhydrous magnesium sulfate and filtered, and the filtrate was concentrated under reduced pressure to give the product (7.4 g, yield: 93.6%).

Step 4: synthesis of 6-bromo-4-hydroxy-2-oxo-1,2-dihydro-1,5-naphthyridine-3-carbonitrile

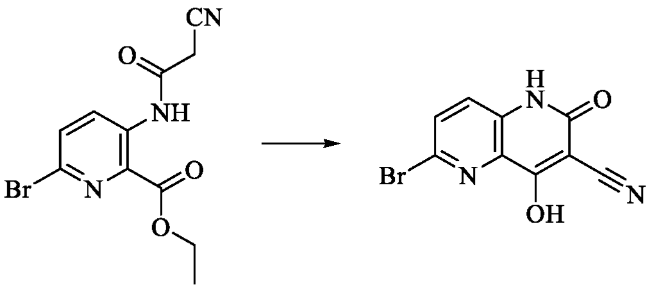

Intermediate ethyl 6-bromo-3-(2-cyanoacetamido)picolinate (7.4 g, 23.71 mmol, 1.0 eq) was dissolved in absolute ethanol (150 mL), and the reaction mixture was heated to 50 °C, stirred for 0.5 h, added with sodium ethoxide (4.8 g, 71.13 mmol, 3.0 eq), and then stirred at 50 °C for 0.5 h. After starting material disappearance as detected by TLC, the reaction mixture was concentrated under reduced pressure, added with water (100 mL), heated to 50 °C for dissolving and adjusted to pH = 2-3 with concentrated hydrochloric acid, and solids were precipitated. The resulting mixture was then filtered, and the filter cake was washed successively with water and acetone, and the filter cake was dried to give the product (6.4 g of crude product), which was used in the next step according to a theoretical amount.

Step 5: synthesis of 2,4,6-trichloro-1,5-naphthyridine-3-carbonitrile

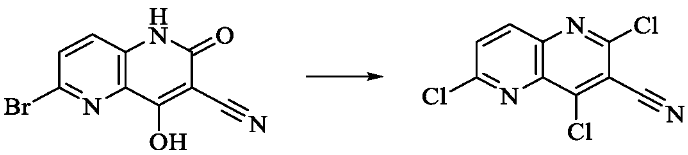

Intermediate 6-bromo-4-hydroxy-2-oxo-1,2-dihydro-1,5-naphthyridine-3-carbonitrile (6.3 g of crude product, 23.71 mmol, 1.0 eq) was added to phosphorus oxychloride (29 g, 189.68 mmol, 8.0 eq), and the reaction mixture was heated to 110 °C and reacted for 4 h. After the presence of most of the starting material as detected by LC-MS, the reaction mixture was added with phosphorus pentachloride (9.8 g, 47.42 mmol, 2.0 eq) and reacted at 110 °C for 3 h. After reaction completion as detected by LC-MS, the reaction mixture was cooled to room temperature, poured into ice water (100 mL) and filtered to give the product (3.0 g of crude product).

Step 6: synthesis of 4,6-dichloro-2-oxo-1,2-dihydro-1,5-naphthyridine-3-carbonitrile

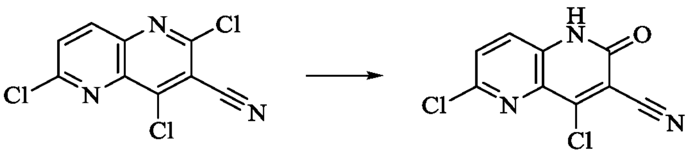

Intermediate 2,4,6-trichloro-1,5-naphthyridine-3-carbonitrile (3.0 g of crude product) was dissolved in a mixed solvent of trifluoroacetic acid (30 mL) and water (7.5 mL), and the reaction mixture was heated to 60 °C and reacted for 3 h. After starting material disappearance as detected by LC-MS, the reaction mixture was cooled to room temperature and poured into ice water (100 mL), and yellow solids were precipitated. The resulting mixture was filtered, and the filter cake was rinsed successively with water and acetone, and dried to give the product (1.9 g, three-step yield: 76%).

Step 7: synthesis of 6-chloro-4-(4-methoxy-4-methylpiperidin-1-yl)-2-oxo-1, 2-dihydro-1,5-naphthyridine-3-carbonitrile

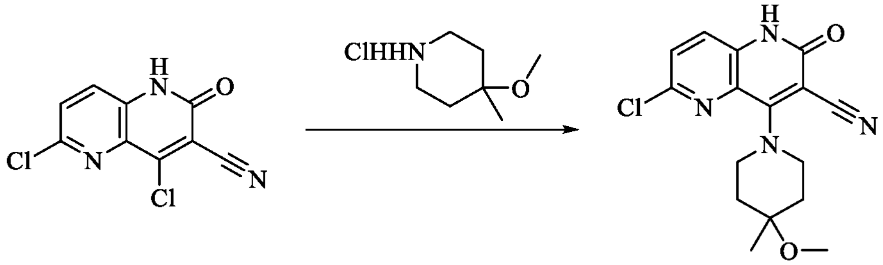

Intermediate 4-chloro-2-oxo-1,2-dihydro-1,5-naphthyridine-3-carbonitrile (1.9 g, 7.92 mmol, 1.0 eq) was dissolved in *N*,*N*-dimethylacetamide (10 mL), and DIPEA (3.5 g, 26.72 mmol, 4.0 eq) and 4-methoxy-4-methylpiperidine hydrochloride (1.5 g, 9.35 mmol, 1.4 eq) were added. Then the reaction mixture was heated to 80 °C and reacted for 1 h. After starting material disappearance as detected by LC-MS, the reaction mixture was cooled to room temperature and poured into ice water (50 mL), and solids were precipitated. The resulting mixture was filtered, and the filter cake was rinsed with water and dried to give the product (561 mg, yield: 21.3%).

Step 8: synthesis of 4-(4-methoxy-4-methylpiperidin-1-yl)-2-oxo-6-vinyl-1,2- dihydro-1,5-naphthyridine-3-carbonitrile

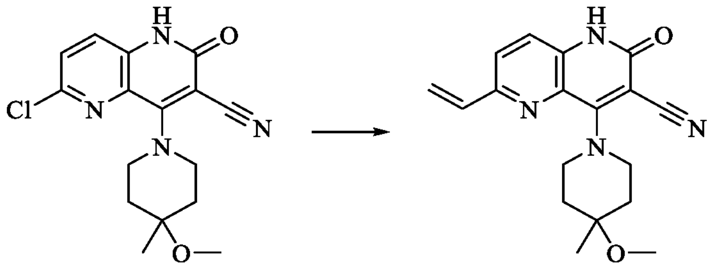

Intermediate 6-chloro-4-(4-methoxy-4-methylpiperidin-1-yl)-2-oxo-1,2-dihydro-1,5-naphthyridine-3-carbonitrile (500 mg, 1.5 mmol, 1.0 eq) was dissolved in a mixed solvent of 1,4-dioxane (15 mL) and $H_2O$ (3 mL), and potassium trifluoro(prop-1-en-2-yl)borate (301.9 mg, 2.25 mmol, 1.5 eq) and cesium carbonate (1.5 g, 4.5 mmol, 3.0 eq) were added, and then [1,1'-bis(diphenylphosphino)ferrocene]palladium dichloride (109.8 mg, 0.15 mmol, 0.1 eq) was added under nitrogen atmosphere. Then the reaction mixture was reacted at 100 °C for 15 h under nitrogen atmosphere. After the reaction completion as detected by LC-MS, the reaction mixture was concentrated under reduced pressure to give a crude product, which was purified by silica gel column chromatography (DCM:MeOH = 80:1) to give the product (206 mg, yield: 42.3%).

Step 9: synthesis of 6-ethyl-4-(4-methoxy-4-methylpiperidin-1-yl)-2-oxo-1,2- dihydro-1,5-naphthyridine-3-carbonitrile

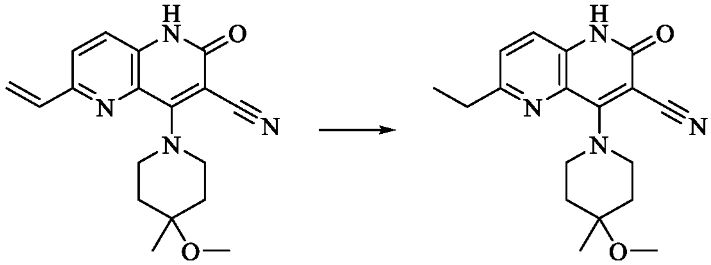

(Compound 167)

Intermediate 4-(4-methoxy-4-methylpiperidin-1-yl)-2-oxo-6-vinyl-1,2-dihydro-1,5-naphthyridine-3-carbonitrile (206 mg, 0.64 mmol, 1.0 eq) was dissolved in absolute methanol (20 mL), and Pd/C (200 mg) was added. After purge with hydrogen for three times, the reaction mixture was reacted for 12 h under hydrogen atmosphere. After the reaction completion as detected by LC-MS, the reaction mixture was filtered under vacuum. The filter cake was rinsed with

methanol, and the filtrate was concentrated under reduced pressure to give an off-white solid, which was slurried with methyl *tert-butyl* ether (3 mL) and filtered. The filter cake was dried to give the product (86.9 mg, yield: 41.9%).

$^1$H NMR (400 MHz, DMSO-$d_6$) δ (ppm): 11.56 (s, 1H), 7.58-7.56 (d, 1H), 7.49-7.47 (d, 1H), 4.10-4.06 (d, 2H), 3.67-3.62 (m, 2H), 3.19 (s, 3H), 2.85-2.79 (m, 2H), 1.92-1.88 (m, 2H), 1.80-1.75 (m, 2H), 1.28-1.24 (m, 3H), 1.19 (s, 3H).
Molecular formula: $C_{18}H_{22}N_4O_2$; Molecular weight: 326.40; LC-MS (Pos, *m/z*) = 327.41[M+H]$^+$.

**Example 6: synthesis of 3-cyano-4-(4-methoxy-4-methylpiperidin-1-yl)-6-methyl-2-oxo-1,2-dihydro-1,7-naphthyridine 7-oxide (Compound 178)**

Step 1: synthesis of 6-chloro-4-(4-methoxy-4-methylpiperidin-1-yl)-2-oxo-1,2-dihydro-1,7-naphthyridine-3-carbonitrile

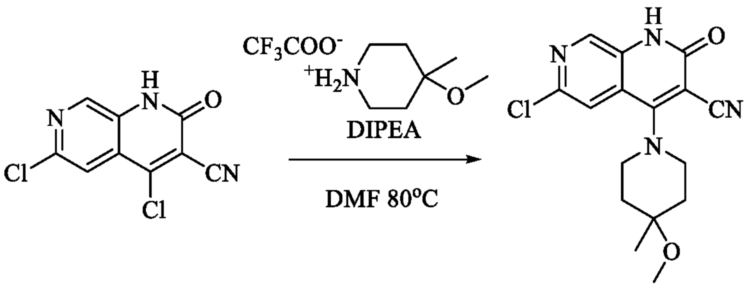


The product 4,6-dichloro-2-oxo-1,2-dihydro-1,7-naphthyridine-3-carbonitrile (2.0 g, 8.33 mmol, 1.0 eq) obtained in step 3 of Example 1 was dissolved in DMF (10 mL), and DIPEA (6.45 g, 50 mmol, 6.0 eq) and 4-methoxy-4-methylpiperidine trifluoroacetate (2.2 g, 9.16 mmol, 1.1 eq) were added, and the reaction mixture was reacted at 80 °C for 2 h. After the reaction completion as detected by LC-MS, the reaction mixture was added with water (10 mL) and extracted with dichloromethane (10 mL × 3). The organic phase was washed with water (10 mL × 3), dried over anhydrous sodium sulfate, filtered, and concentrated under reduced pressure to give the product (2.7 g of crude product) in the form of a yellow solid.
$^1$H NMR (400 MHz, DMSO-$d_6$) δ (ppm): 12.11 (s, 1H), 8.45 (s, 1H), 7.61 (s, 1H), 3.61-3.59 (m, 4H), 3.18 (s, 3H), 1.91-1.88 (m, 2H), 1.81-1.76 (m, 2H), 1.21 (s, 3H).

Step 2: synthesis of 4-(4-methoxy-4-methylpiperidin-1-yl)-6-methyl-2-oxo-1, 2-dihydro-1,7-naphthyridine-3-carbonitrile

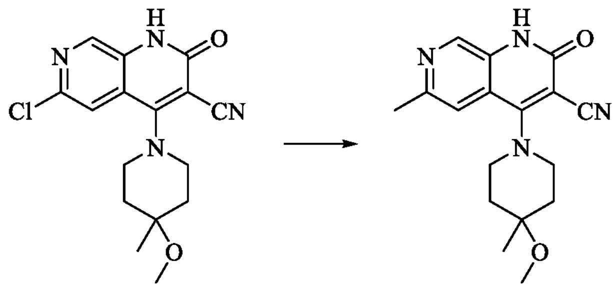

Intermediate 6-chloro-4-(4-methoxy-4-methylpiperidin-1-yl)-2-oxo-1,2-dihydro-1, 7-naphthyridine-3-carbonitrile (1.3 g, 3.91 mmol, 1.0 eq), cesium carbonate (3.8 g, 11.73 mmol, 3.0 eq) and trimethylboroxine (50% THF solution, 3.9 g, 15.62 mmol, 4.0 eq) were dissolved in 1,4-dioxane (20 mL). After the addition, the reaction mixture was purged with nitrogen for three times and then added with [1,1'-bis(diphenylphosphino)ferrocene] palladium dichloride (286 mg, 0.39 mmol, 0.1 eq). After the addition, the reaction mixture was purged with nitrogen for three times again and heated to reflux for 12 h. After the presence of remaining starting material as detected by TLC, the reaction mixture was supplemented with trimethylboroxine (50% THF solution, 3.9 g, 15.62 mmol, 4.0 eq) and [1,1'-bis(diphenylphosphino)ferrocene]palladium dichloride (286 mg, 0.39 mmol, 0.1 eq) and reacted at reflux for 4 h. As starting material disappearance as detected by TLC, the reaction mixture was cooled to room temperature, added with water (50 mL) and dichloromethane (100 mL) and stirred for 5 min, and solids were precipitated. The resulting mixture was filtered, and the filter cake was rinsed with dichloromethane. Liquid separation was performed. The aqueous phase was extracted with dichloromethane (100 mL × 3), and the organic phases were combined, dried over anhydrous sodium sulfate and filtered. The mother liquor was concentrated under reduced pressure to give a crude product, which was purified by silica gel column chromatography (MeOH:DCM = 1:100-1:50) to give the product (309.9 mg, yield: 25.4%).

Step 3: synthesis of 3-cyano-4-(4-methoxy-4-methylpiperidin-1-yl)-6-methyl-2- oxo-1,2-dihydro-1,7-naphthyridine 7-oxide

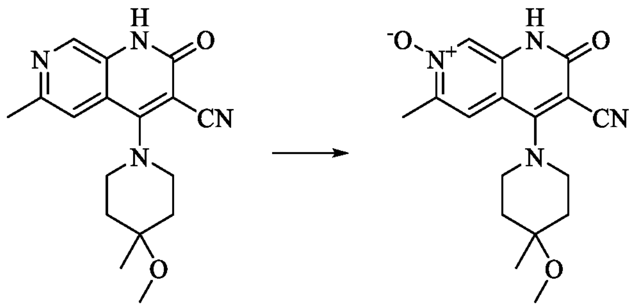

Compound 178

4-(4-methoxy-4-methylpiperidin-1-yl)-6-methyl-2-oxo-1,2-dihydro- 1,7-naphthyridine-3-carbonitrile (3.0 g, 9.6 mmol, 1.0 eq) was dissolved in dichloromethane (30 mL), and the reaction mixture was cooled to 0 °C under an ice bath, added with m-chloroperoxybenzoic acid (70%, 2.367 g, 9.6 mmol, 1.0 eq), and then reacted for 12 h. After the reaction completion as detected by LC-MS, the reaction mixture was added with dichloromethane (30 mL) and potassium carbonate (2.65 g, 19.2 mmol, 2.0 eq), stirred for 30 min and filtered under vacuum. The filter cake was slurried with water (50 mL) and filtered, and the filtrate was concentrated under reduced pressure. The residue was combined with the filter cake and dried to give the product (2.9 g, yield: 92%).

$^1$HNMR(400 MHz, DMSO-$d_6$) δ (ppm): 11.69 (s, 1H), 8.19(s, 1H), 7.66 (s, 1H), 3.54-3.65 (m, 4H), 3.19 (s, 3H), 2.37 (s, 3H), 1.87-1.91 (d, 2H), 1.74-1.82 (m, 2H), 1.21 (s, 3H).
Molecular formula: $C_{17}H_{20}N_4O_3$; Molecular weight: 328.36; LC-MS (Pos, *m/z*) = 329.15[M+H]$^+$.

**Example 7: synthesis of 6-(hydroxymethyl)-4-(4-methoxy-4-methylpiperidin-1-yl) -2-oxo-1,2-dihydro-1,7-naphthyridine-3-carbonitrile (Compound 177)**

Step 1: synthesis of (3-cyano-4-(4-methoxy-4-methylpiperidin-1-yl)-2-oxo-1, 2-dihydro-1,7-naphthyridin-6-yl) methyl acetate

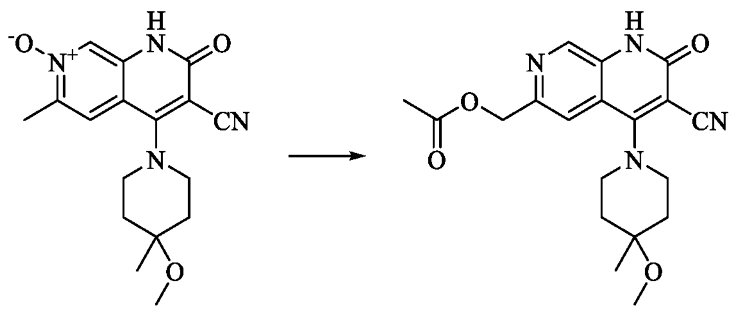

The product 3-cyano-4-(4-methoxy-4-methylpiperidin-1-yl)-6-methyl-2-oxo-1,2-dihydro-1,7-naphthyridine 7-oxide (1.5 g, 4.56 mmol, 1.0 eq) of Example 6 was suspended in acetic anhydride (20 mL) and the reaction mixture was

reacted at 140 °C for 5 h. After reaction completion as detected by LC-MS, the reaction mixture was concentrated under reduced pressure, added with water (50 mL) and extracted with ethyl acetate (50 mL × 3). The organic phase was dried over anhydrous sodium sulfate and filtered under vacuum. The filtrate was concentrated under reduced pressure to give a crude product, which was purified by silica gel column chromatography (DCM:MeOH = 50:1) to give the product (220 mg, yield: 13%).

Step 2: synthesis of 6-(hydroxymethyl)-4-(4-methoxy-4-methylpiperidin-1-yl)-2- oxo-1,2-dihydro-1,7-naphthyridine-3-carbonitrile

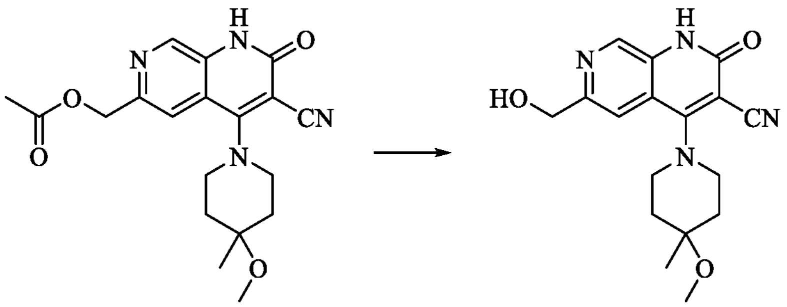

Compound 177

Intermediate (3-cyano-4-(4-methoxy-4-methylpiperidin-1-yl)-2-oxo-1,2-dihydro-1, 7-naphthyridin-6-yl)methyl acetate (220 mg, 0.59 mmol, 1.0 eq) and lithium hydroxide monohydrate (170 mg, 4.05 mmol, 6.9 eq) were dissolved in methanol (2 mL) and water (2 mL), and the reaction mixture was reacted for 12h. After the reaction completion as detected by LC-MS, the reaction mixture was concentrated under reduced pressure to give a crude product, which was purified by silica gel column chromatography (DCM:MeOH = 10:1) to give the product (110 mg, yield: 57 %).

$^1$H NMR (400 MHz, DMSO-$d_6$) δ (ppm): 11.97 (s, 1H), 8.57 (s, 1H), 7.66 (s, 1H), 5.55-5.58 (m, 1H), 4.59-4.61 (d, 2H), 3.60-3.61 (d, 4H), 3.19 (s, 3H), 1.91-1.94 (d, 2H), 1.73-1.80 (m, 2H), 1.23 (s, 3H).
Molecular formula: $C_{17}H_{20}N_4O_3$; Molecular weight: 328.37; LC-MS (Pos, *m/z*) = 329.15[M+H]$^+$.

**Example 8: synthesis of 6-ethyl-4-(4-methoxy-4-methylpiperidin-1-yl)-2-oxo-1, 2-dihydro-1,7-naphthyridine-3-carbonitrile-7-oxynitride (Compound 180)**

Step 1: synthesis of 6-chloro-4-(4-methoxy-4-methylpiperidin-1-yl)-2-oxo-1, 2-dihydro-1,7-naphthyridine-3-carbonitrile

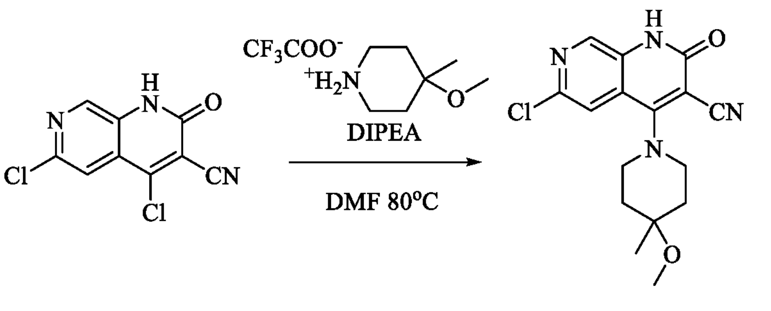


The product 4,6-dichloro-2-oxo-1,2-dihydro-1,7-naphthyridine-3-carbonitrile (2.0 g, 8.33 mmol, 1.0 eq) obtained in step 3 of Example 1 was dissolved in DMF (10 mL), and DIPEA (6.45 g, 50 mmol, 6.0 eq) and 4-methoxy-4-methylpiperidine trifluoroacetate (2.2 g, 9.16 mmol, 1.1 eq) were added, and the reaction mixture was reacted at 80 °C for 2 h. After the reaction completion as detected by LC-MS, the reaction mixture was added with water (10 mL) and extracted with dichloromethane (10 mL × 3). The organic phase was washed with water (10 mL × 3), dried over anhydrous sodium sulfate, filtered, and concentrated under reduced pressure to give the product (2.7 g of crude product) in the form of a yellow solid.

$^1H$ NMR (400 MHz, DMSO-$d_6$) δ (ppm): 12.11 (s, 1H), 8.45 (s, 1H), 7.61 (s, 1H), 3.61-3.59 (m, 4H), 3.18 (s, 3H), 1.91-1.88 (m, 2H), 1.81-1.76 (m, 2H), 1.21 (s, 3H).
Molecular formula: $C_{16}H_{17}N_4O_2Cl$; Molecular weight: 332.79; LC-MS (Pos, *m/z*) = 333.7[M+H]$^+$.

Step 2: synthesis of 4-(4-methoxy-4-methylpiperidin-1-yl)-2-oxo-6-vinyl1,2- dihydro-1,7-naphthyridine-3-carbonitrile

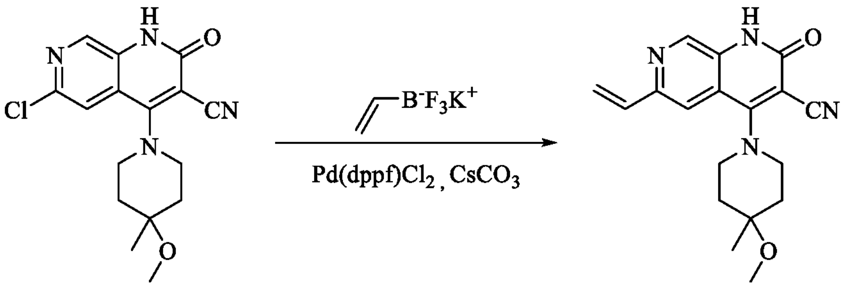


Intermediate 6-chloro-4-(4-methoxy-4-methylpiperidin-1-yl)-2-oxo-1,2-dihydro-1, 7-naphthyridine-3-carbonitrile (2.7 g of crude product, 8.11 mmol, 1.0 eq) was dissolved in 1,4-dioxane (20 mL) and $H_2O$ (5 mL), and potassium vinyltrifluoroborate (1.63 g, 12.17 mmol, 1.5 eq), cesium carbonate (3.965 g, 12.17 mmol, 1.5 eq) and [1,1'-bis(diphenylphosphino)ferrocene]palladium dichloride (297 mg, 0.41 mmol, 0.05 eq) were added, and the reaction mixture was reacted at 100 °C for 8 h under nitrogen atmosphere. After the reaction completion as detected by LC-MS, the reaction mixture was added with water (20 mL) and extracted with dichloromethane (30 mL × 3). The organic phase was dried over anhydrous sodium sulfate, filtered and concentrated under reduced pressure to give a crude product, which was purified by silica gel column chromatography (DCM:MeOH = 70:1) to give the product (1.15 g, yield: 43%) in the form of a yellow solid.

Step 3: synthesis of 6-ethyl-4-(4-methoxy-4-methylpiperidin-1-yl)-2-oxo-1,2- dihydro-1,7- naphthyridine-3-carbonitrile

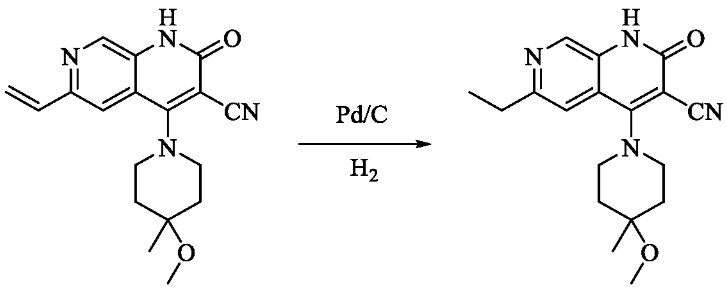


Intermediate 4-(4-methoxy-4-methylpiperidin-1-yl)-2-oxo-6-vinyl-1,2-dihydro-1, 7-naphthyridine-3-carbonitrile (150 mg, 0.46 mmol, 1.0 eq) was dissolved in methanol (5 mL), and Pd/C (100 mg) was added. After purge with hydrogen for three times, the reaction mixture was reacted for 1 h under hydrogen atmosphere. After the reaction completion as detected by LC-MS, the reaction mixture was filtered under vacuum, and the filtrate was concentrated under reduced pressure to give the product (120 mg, yield: 80%).

Step 5: synthesis of 6-ethyl-4-(4-methoxy-4-methylpiperidin-1-yl)-2-oxo-1, 2-dihydro-1,7-naphthyridine-3-carbonitrile-7-oxynitride

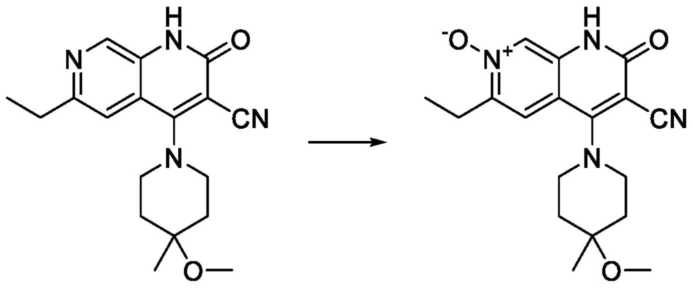

Compound 180

6-ethyl-4-(4-methoxy-4-methylpiperidin-1-yl)-2-oxo-1,2-dihydro-1,7-naphthyridine-3-carbonitrile (2.00 g, 6.13 mmol, 1.0 eq) was dissolved in dichloromethane (20.00 mL), and 3-chloroperoxybenzoic acid (70%, 1.66 g, 6.74 mmol, 1.1 eq) was added under an ice bath, and the reaction mixture was reacted at 25 °C for 4 h under nitrogen atmosphere. After the reaction completion as detected by LC-MS, the reaction mixture was added with water (20 mL) and adjusted to pH = 8-9 with potassium carbonate, and solids were precipitated. The resulting mixture was then filtered under vacuum to give the product (1.20 g, yield: 57.2%).

$^1$HNMR (400 MHz, DMSO-$d_6$) δ (ppm): 11.77 (s, 1H), 8.18 (s, 1H), 7.51 (s, 1H), 3.52-3.62 (m, 4H), 3.18 (s, 3H), 2.76-2.81 (m, 2H), 1.88-1.91 (d, 2H), 1.73-1.80 (m, 2H), 1.05-1.20 (m, 6H).
Molecular formula: $C_{18}H_{22}N_4O_3$; Molecular weight: 342.40; LC-MS (Pos, *m/z*) = 343.40[M+H]$^+$.

**Example 9: synthesis of 6-ethyl-4-(4-hydroxy-4-methylpiperidin-1-yl)-2-oxo-1, 2-dihydro-1,7-naphthyridine-3-carbonitrile (Compound 181)**

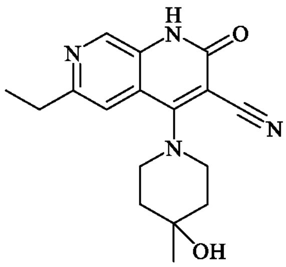

Compound 181

This compound was synthesized by referring to the synthesis method of Compound 180.

$^1$H NMR (400 MHz, DMSO-$d_6$) δ (ppm): 11.88 (s, 1H), 8.58 (s, 1H), 7.39 (s, 1H), 4.57 (s, 1H), 3.58-3.73 (m, 4H), 2.78-2.84 (m, 2H), 1.66-1.83 (m, 4H), 1.10-1.30 (m, 6H).
Molecular formula: $C_{17}H_{20}N_4O_2$; Molecular weight: 312.37; LC-MS (Pos, *m/z*) = 313.37[M+H]$^+$.

**Example 10: synthesis of 6-acetyl-2-hydroxy-4-(4-methoxy-4-methylpiperidin-1-yl)-1, 7-naphthyridine-3-carbonitrile (Compound 183)**

Step 1: synthesis of 6-(1-bromoethyl)-2,4-dichloro-1,7-naphthyridine-3-carbonitrile

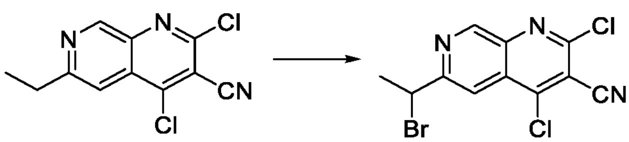

To a 1 L single-neck flask were added carbon tetrachloride (600.00 mL), 2,4-dichloro-6-ethyl-1,7-naphthyridine-3-carbonitrile (30.00 g, 119.00 mmol, 1 eq), NBS (42.36 g, 238.00 mmol, 2 eq) and AIBN (15.00 g), and the reaction

mixture was reacted at 90 °C for 2 h. After the reaction completion as detected by LC-MS, the reaction mixture was cooled to 10-15 °C and filtered under vacuum. The filtrate was concentrated to dryness, and recrystallized with PE and EA to give the product 6-(1-bromoethyl)-2,4-dichloro-1,7-naphthyridine-3-carbonitrile (35 g).

Step 2: synthesis of 6-(1-bromoethyl)-2-chloro-4-(4-methoxy-4-methylpiperidin -1-yl)-1,7-naphthyridine-3-carbonitrile

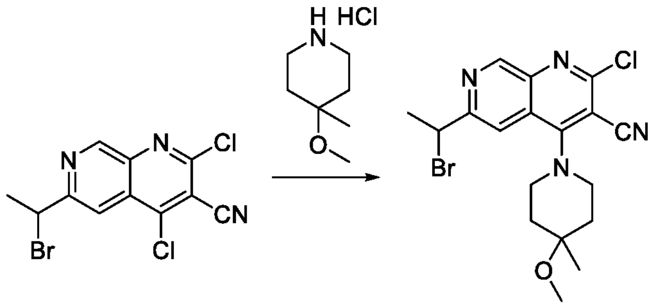

To a 1 L single-neck flask were added ethanol (400.00 mL), 6-(1-bromoethyl)-2, 4-dichloro-1,7-naphthyridine-3-carbonitrile (20.00 g, 60.40 mmol, 1 eq), 4-methoxy- 4-methylpiperidine hydrochloride (11.00 g, 66.44 mmol, 1.1 eq) and triethylamine (13.45 g, 132.88 mmol, 2.2 eq), and the reaction mixture was reacted at 90 °C for 2 h. After the reaction completion as detected by LC-MS, the reaction mixture was cooled to room temperature, concentrated to dryness under reduced pressure, added with water (400 mL), stirred for 1 h, and filtered under vacuum. The filter cake was recrystallized with ethanol to give the product 6-(1-bromoethyl)-2-chloro-4-(4-methoxy-4-methylpiperidin-1-yl)-1,7-naphthyridine-3-carbonitrile (20 g).

Step 3: synthesis of 2-hydroxy-6-(1-hydroxyethyl)-4-(4-methoxy-4-methylpiperidin-1-yl)-1,7-naphthyridine-3-carbonitrile

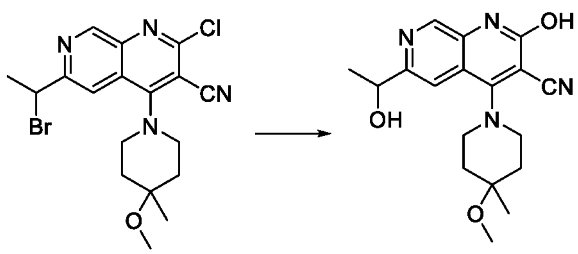

To a 250 mL single-neck flask were added acetic acid (60.00 mL), water (30.00 mL) and 6-(1-bromoethyl)-2-chloro-4-(4-methoxy-4-methylpiperidin-1-yl)-1,7-naphthyridine-3-carbonitrile (15.00 g), and the reaction mixture was reacted at 100 °C for 6 h. After the reaction completion as detected by LC-MS, the reaction mixture was cooled to room temperature, concentrated to dryness under reduced pressure, added with water (100 mL), adjusted to pH = 7-8, and extracted with ethyl acetate. The organic phases were combined, dried over anhydrous sodium sulfate and filtered under vacuum, and the filtrate was concentrated under reduced pressure to dryness to give a crude product. The crude product was first thermally slurried with EA and then recrystallized with ethanol to give the product 2-hydroxy-6-(1-hydroxyethyl)-4-(4-methoxy-4-methylpiperidine-1-yl)-1,7-n aphthyridine-3-carbonitrile (6 g).

Step 4: synthesis of 6-acetyl-2-hydroxy-4-(4-methoxy-4-methylpiperidin-1-yl)-1,7-naphthyridine-3-carbonitrile

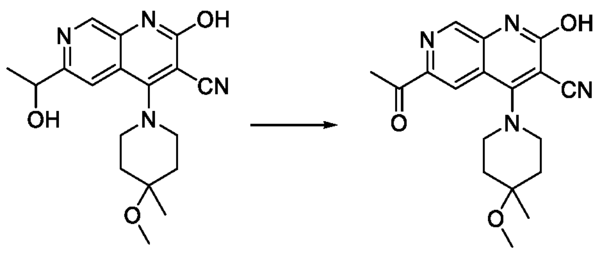

Compound 183

To a 250 mL single-neck flask were added DCM (90.00 mL), 2-hydroxy-6- (1-hydroxyethyl)-4-(4-methoxy-4-methylpiperidin-1-yl)-1,7-naphthyridine-3-carbonitrile (4.50 g, 13.15 mmol, 1 eq) and Dess-Martin oxidant (6.14 g, 1.1 eq, 14.47 mmol), and the reaction mixture was stirred at room temperature for 2 h. After the reaction completion as detected by LC-MS, the reaction mixture was added with water (60 mL) and saturated sodium thiosulfate solution (30 mL), and stirred for 1 h. Liquid separation was performed. The organic phase was dried over anhydrous sodium sulfate and filtered under vacuum, and the filtrate was concentrated to dryness under reduced pressure to give a crude product, which was purified by column chromatography to give the product 6-acetyl-2- hydroxy-4-(4-methoxy-4-methylpiperidin-1-yl)-1,7-naphthyridine-3-carbonitrile (0.7 g).

$^1$H NMR (400 MHz, DMSO) δ (ppm): 12.33 (s, 1H), 8.70 (s, 1H), 8.19 (s, 1H), 3.62-3.64 (d, 4H), 3.20 (s, 3H), 2.63 (s, 3H), 1.92-1.96 (m, 2H), 1.76-1.78 (m, 2H), 1.24 (s, 3H).
Molecular formula: $C_{18}H_{20}N_4O_3$; Molecular weight: 340.38; LC-MS (Pos, *m/z*) = 341.21[M+H]$^+$.

**Example 11: synthesis of 6-ethyl-2-hydroxy-4-(4-(methoxy-*d*$_3$)-4-methylpiperidin -1-yl)-1,7-naphthyridine-3-carbonitrile (tautomer of Compound 184)**

Step 1: synthesis of *tert-butyl* 4-hydroxy-4-methylpiperidine-1-carboxylate

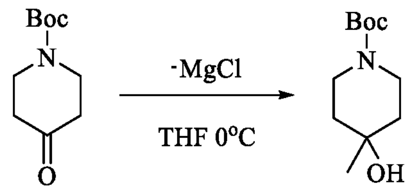


The starting material *tert*-butyl 4-oxopiperidine-1-carboxylate (5.0 g, 25 mmol, 1.0 eq) was dissolved in tetrahydrofuran (25 mL), and then the reagent methylmagnesium chloride (9 mL, 27 mmol, 1.1 eq) was added at 0 °C under nitrogen atmosphere. Then the reaction mixture was reacted for 2 h. After the reaction completion as detected by TLC, the reaction mixture was adjusted to pH = 4 with diluted hydrochloric acid, added with water (30 mL), and then extracted with ethyl acetate (30 mL × 3). The organic phase was dried, filtered and concentrated under reduced pressure to give a crude product, which was purified by silica gel column chromatography (PE:EA = 5:1) to give the product (5.2 g, yield: 96%).

Step 2: synthesis of *tert*-butyl 4-(methoxy-*d*$_3$)-4-methylpiperidine-1-carboxylate

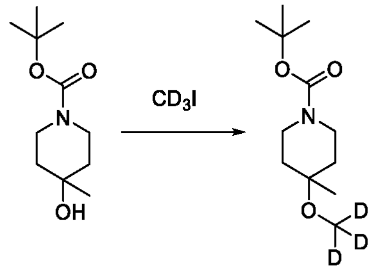


In a 100 mL single-neck flask, purge with nitrogen was performed, *tert*-butyl 4-hydroxy-4-methylpiperidine-1-carboxylate (2.70 g, 12.55 mmol, 1 eq) and THF (27.00 mL) were added, and then sodium hydride (60%, 0.76 g, 1.5 eq) was added in portions. The reaction mixture was reacted at room temperature for 0.5 h, then added dropwise with $CD_3I$ (4.00 g, 27.62 mmol, 2.2 eq) and reacted overnight at 30 °C after the addition. After the reaction completion as detected by TLC, the reaction mixture was concentrated to dryness under reduced pressure and added with EA (100 mL), and liquid separation was performed. The organic phase was washed with water, dried over anhydrous sodium sulfate and filtered under vacuum, and the filtrate was concentrated to dryness under reduced pressure to give the product *tert*-butyl 4-(methoxy-$d_3$)-4-methylpiperidine-1-carboxylate (4.3 g).

Step 3: synthesis of 4-(methoxy-$d_3$)-4-methylpiperidine hydrochloride

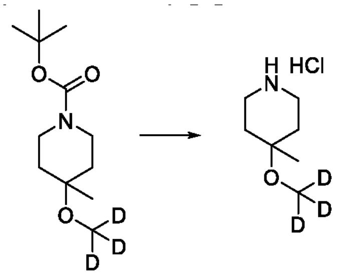

In a 500 mL single-neck flask, purge with nitrogen was performed, *tert*-butyl 4-(methoxy-$d_3$)-4-methylpiperidine-1-carboxylate (4.30 g) was added, and then hydrogen chloride-ethanol (8.60 mL) and ethanol (8.60 mL) were added. The reaction mixture was reacted at 30 °C for 2 h. After the reaction completion as detected by TLC, the reaction mixture was concentrated to dryness under reduced pressure, added with EA (30 mL), stirred for 0.5 h, and filtered under vacuum to give the product 4-(methoxy-$d_3$)-4-methylpiperidine hydrochloride (1.20 g).

Step 4: synthesis of 6-ethyl-2-hydroxy-4-(4-(methoxy-$d_3$)-4-methylpiperidin-1-yl)-1, 7-naphthyridine-3-carbonitrile

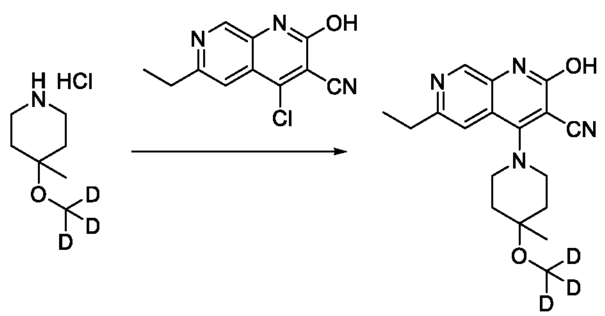

Tautomer of Compound 184

To a 100 mL single-neck flask were added 4-chloro-6-ethyl-2-hydroxy-1, 7-naphthyridine-3-carbonitrile (1.50 g, 6.47 mmol, 1 eq), 4-(methoxy-$d_3$)- 4-methylpiperidine hydrochloride (1.20 g, 7.12 mmol, 1.1 eq), ethanol (15.00 mL) and TEA (1.44 g, 14.23 mmol, 2.2 eq), and the reaction mixture was reacted at 90 °C for 1 h. After the reaction completion

as detected by LC-MS, the reaction mixture was cooled down, concentrated to dryness under reduced pressure, added with water (50 mL), stirred for 0.5 h, and filtered under vacuum to give a crude product, which was recrystallized with ethanol to give the product 6-ethyl-2-hydroxy-4-(4-(methoxy-*d*$_3$)-4-methylpiperidin -1-yl)-1,7-naphthyridine-3-carbonitrile (2.03 g).

$^1$HNMR (400 MHz, DMSO) δ (ppm): 11.91 (s, 1H), 8.58 (s, 1H), 7.40 (s, 1H), 3.60-3.61 (d, 4H), 2.78-2.84 (q, 2H), 1.89-1.92 (m, 2H), 1.77-1.82 (m, 2H), 1.26 (s, 6H).
Molecular formula: $C_{18}H_{19}F_3N_4O_2$; Molecular weight: 329.42; LC-MS (Pos, *m*/*z*) =330.21[M+H$^+$].

**Example 12: synthesis of (*R*)-6-(1-hydroxyethyl)-4-(4-methoxy-4-methylpiperidin -1-yl)-2-oxo-1,2-dihydro-1,7-naphthyridine-3-carbonitrile and (*S*)-6-(1-hydroxyethyl) -4-(4-methoxy-4-methylpiperidin-1-yl)-2-oxo-1,2-dihydro-1,7-naphthyridine-3-carbonitrile**

Step 1: synthesis of 6-chloro-4-(4-methoxy-4-methylpiperidin-1-yl)-2-oxo-1, 2-dihydro-1,7-naphthyridine-3-carbonitrile

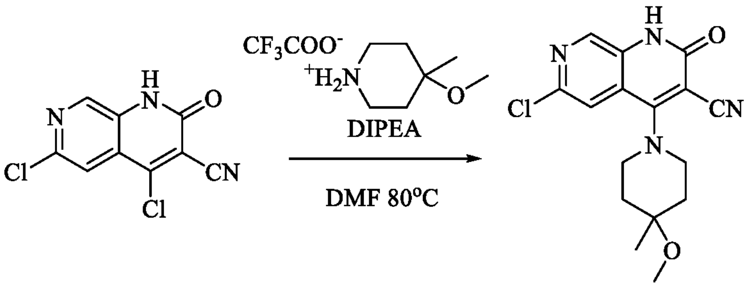


The product 4,6-dichloro-2-oxo-1,2-dihydro-1,7-naphthyridine-3-carbonitrile (2.0 g, 8.33 mmol, 1.0 eq) obtained in step 3 of Example 1 was dissolved in DMF (10 mL), and DIPEA (6.45 g, 50 mmol, 6.0 eq) and 4-methoxy-4-methylpiperidine trifluoroacetate (2.2 g, 9.16 mmol, 1.1 eq) were added, and the reaction mixture was reacted at 80 °C for 2 h. After the reaction completion as detected by LC-MS, the reaction mixture was added with water (10 mL) and extracted with dichloromethane (10 mL × 3). The organic phase was washed with water (10 mL × 3), dried over anhydrous sodium sulfate, filtered, and concentrated under reduced pressure to give the product (2.7 g of crude product) in the form of a yellow solid.

$^1$H NMR (400 MHz, DMSO-*d*$_6$) δ (ppm): 12.11 (s, 1H), 8.45 (s, 1H), 7.61 (s, 1H), 3.61-3.59 (m, 4H), 3.18 (s, 3H), 1.91-1.88 (m, 2H), 1.81-1.76 (m, 2H), 1.21 (s, 3H).
Molecular formula: $C_{16}H_{17}N_4O_2Cl$; Molecular weight: 332.79; LC-MS (Pos, *m*/*z*) = 333.7[M+H]$^+$.

Step 2: synthesis of 4-(4-methoxy-4-methylpiperidin-1-yl)-2-oxo-6-vinyl-1, 2-dihydro-1,7-naphthyridine-3-carbonitrile

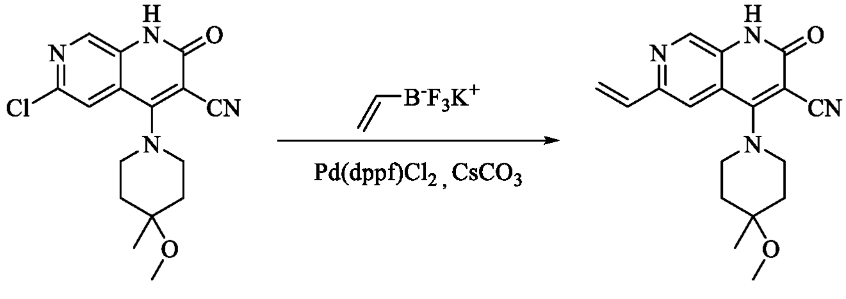


Intermediate 6-chloro-4-(4-methoxy-4-methylpiperidin-1-yl)-2-oxo-1,2-dihydro-1, 7-naphthyridine-3-carbonitrile (2.7 g of crude product, 8.11 mmol, 1.0 eq) was dissolved in 1,4-dioxane (20 mL) and $H_2O$ (5 mL), and potassium vinyltrifluoroborate (1.63 g, 12.17 mmol, 1.5 eq), cesium carbonate (3.965 g, 12.17 mmol, 1.5 eq) and [1,1'-bis(diphenylphosphino)ferrocene]palladium dichloride (297 mg, 0.41 mmol, 0.05 eq) were added, and the reaction mixture was reacted at 100 °C for 8 h under nitrogen atmosphere. After the reaction completion as detected by LC-MS, the reaction mixture was added with water (20 mL) and extracted with dichloromethane (30 mL × 3). The organic phase was dried over anhydrous sodium sulfate, filtered and concentrated under reduced pressure to give a crude product, which was purified by silica gel column chromatography (DCM:MeOH = 70:1) to give the product (1.15 g, yield: 43%) in the form of a yellow solid.

Step 3: synthesis of 6-(1,2-dihydroxyethyl)-4-(4-methoxy-4-methylpiperidin-1-yl)-2-oxo-1,2-dihydro-1,7-naphthyridine-3-carbonitrile

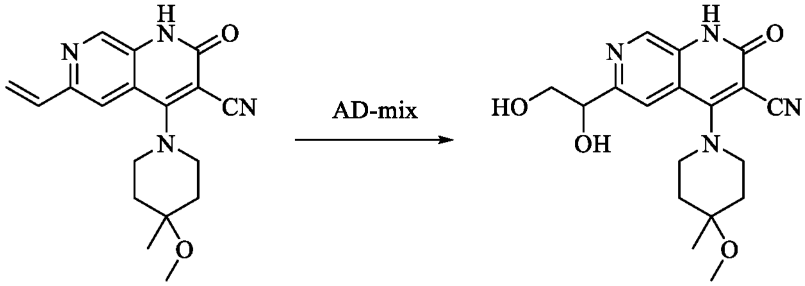


Intermediate 4-(4-methoxy-4-methylpiperidin-1-yl)-2-oxo-6-vinyl-1,2-dihydro-1,7-naphthyridine-3-carbonitrile (500 mg, 1.542 mmol, 1.0 eq) was dissolved in *tert*-butanol (10 mL) and water (10 mL), and methanesulfonamide (147 mg, 1.542 mmol, 1.0 eq) and ADmix-β (6.0 g) were added, and the reaction mixture was reacted at room temperature for 12 h. After the reaction completion as detected by LC-MS, the reaction mixture was added with water (10 mL) and extracted with dichloromethane (30 mL × 3). The organic phase was dried over anhydrous sodium sulfate, filtered and concentrated under reduced pressure to give the product (552 mg, yield: 100%).

Step 4: synthesis of 6-formyl-4-(4-methoxy-4-methylpiperidin-1-yl)-2-oxo-1, 2-dihydro-1,7-naphthyridine-3-carbonitrile

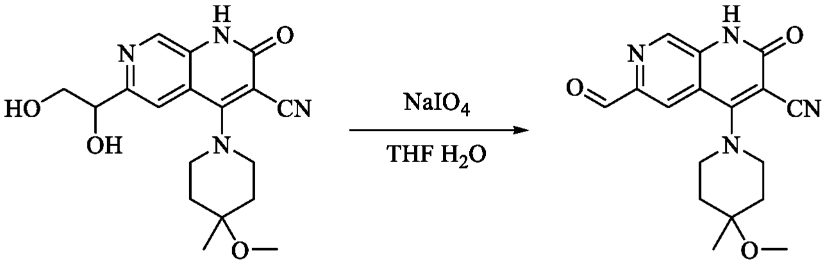


Intermediate 6-(1,2-dihydroxyethyl)-4-(4-methoxy-4-methylpiperidin-1-yl)-2-oxo-1, 2-dihydro-1,7-naphthyridine-3-carbonitrile (552 mg, 1.542 mmol, 1.0 eq) was dissolved in tetrahydrofuran (10 mL) and water (2 mL), and sodium periodate (650 mg, 3.084 mmol, 2.0 eq) was added, and the reaction mixture was reacted for 4 h. After the reaction completion as detected by LC-MS, the reaction mixture was added with water (10 mL) and extracted with ethyl acetate (20 mL × 3). The organic phase was dried over anhydrous sodium sulfate, filtered and concentrated under reduced pressure to give a crude product, which was purified by silica gel column chromatography (DCM:MeOH = 60:1) to give the product (160 g, two-step yield: 32%) in the form of a yellow solid.

Step 5: synthesis of 6-(1-hydroxyethyl)-4-(4-methoxy-4-methylpiperidin-1-yl)- 2-oxo-1,2-dihydro-1,7-naphthyridine-3-carbonitrile

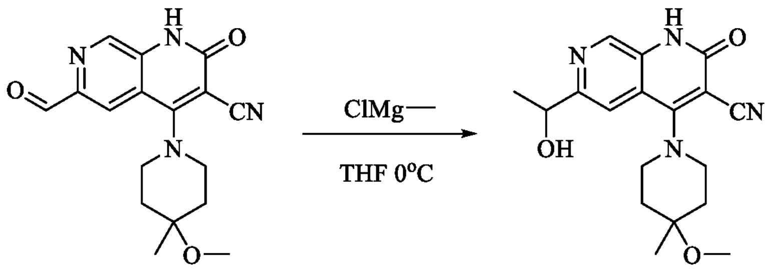


Compound M

Intermediate 6-formyl-4-(4-methoxy-4-methylpiperidin-1-yl)-2-oxo-1,2-dihydro-1, 7-naphthyridine-3-carbonitrile (160 mg, 0.49 mmol, 1.0 eq) was dissolved in tetrahydrofuran (5 mL), and methylmagnesium chloride (1 mL) was added dropwise at 0 °C, and the reaction mixture was reacted for 1 h. After the reaction completion as detected by LC-MS, the reaction mixture was added with water (10 mL) and extracted with ethyl acetate (20 mL × 3). The organic phase was dried over anhydrous sodium sulfate, filtered and concentrated under reduced pressure to give a crude product, which was purified by silica gel column chromatography (DCM:MeOH = 40:1) to give the product (108 mg, yield: 64%).

$^1$H NMR (400 MHz, DMSO-$d_6$) δ (ppm): 11.93 (s, 1H), 8.58 (s, 1H), 7.72 (s, 1H), 5.48-5.49 (d, 1H), 4.75-4.81 (m, 1H), 3.56-3.65 (m, 4H), 3.20 (s, 3H), 1.91-1.95 (m, 2H), 1.73-1.79 (m, 2H), 1.38-1.40 (d, 3H), 1.23 (s, 3H).
Molecular formula: $C_{18}H_{22}N_4O_3$; Molecular weight: 342.40; LC-MS (Pos, *m/z*) = 343.17[M+H]$^+$.

Compound M (0.3925 g) was dissolved in methanol to give a solution at a concentration of 2 mg/mL, and enantiomers were separated out by Shimadzu LC-20AD preparative liquid chromatograph under the following condition: the compounds obtained from the corresponding fractions at 6 min and 12 min, respectively, were collected. The compound obtained from the corresponding fraction at 6 min was Compound A, and the compound obtained from the corresponding fraction at 12 min was Compound B. The solvent was removed by rotary evaporation to give Compound A (0.1814 g) and Compound B (0.1984 g). The Compound A and Compound B are enantiomers, and the structures thereof are as follows; when the Compound A is one of the structures, the Compound B is the other one:

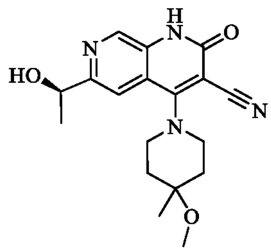

and

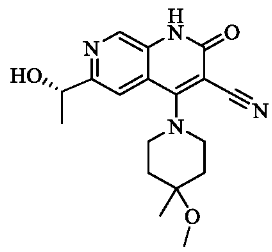

.

**Example 13: synthesis of 1-(3-cyano-4-(4-methoxy-4-methylpiperidin-1-yl)-2- oxo-1,2-dihydro-1,7-naphthyridin-6-yl)ethyl acetate (Compound 189)**

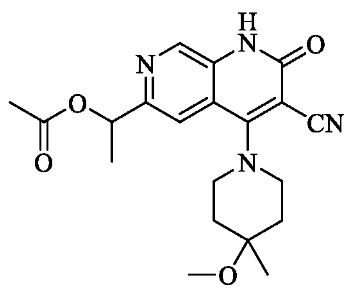

Step: synthesis of 1-(3-cyano-4-(4-methoxy-4-methylpiperidin-1-yl)-2-oxo-1, 2-dihydro-1,7-naphthyridin-6-yl)ethyl acetate

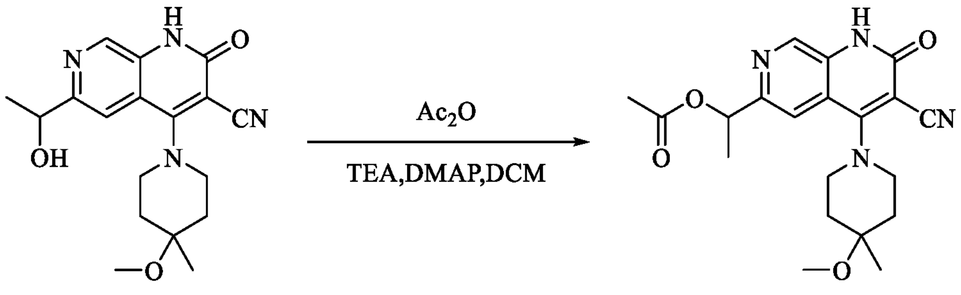


Compound 189

The product 6-(1-hydroxyethyl)-4-(4-methoxy-4-methylpiperidin-1-yl)-2-oxo-1,2-dihydro-1,7-naphthyridine-3-carbonitrile (400 mg, 1.17 mmol, 1.0 eq) obtained in step 5 of Example 12 was dissolved in DCM (4 mL), and DMAP (3 mg, 0.023 mmol, 0.02 eq), triethylamine (590 mg, 5.84 mmol, 5.0 eq) and acetic anhydride (240 mg, 2.34 mmol, 2.0 eq) were added, and the reaction mixture was heated to 35 °C and reacted for 2 h. After the presence of a small amount of remaining starting material as detected by TLC, the reaction mixture was concentrated under reduced pressure, added with water (20 mL) and extracted with DCM (20 mL $\times$ 3). The organic phases were combined, dried over anhydrous magnesium sulfate and filtered, and the filtrate was concentrated under reduced pressure to give a crude product, which was purified by preparative thin-layer chromatography (MeOH:DCM = 1:15) to give the product (238.8 mg, yield: 51.3%).

$^1$H-NMR (400MHz, DMSO-$d_6$) $\delta$ (ppm): 12.02 (s, 1H), 8.63 (s, 1H), 7.57 (s, 1H), 5.88-5.86 (m, 1H), 3.59-3.58 (m, 4H), 3.19 (s, 3H), 2.51-2.50 (s, 3H), 1.94-1.91 (m, 2H), 1.81-1.76 (m, 2H), 1.54-1.53 (d, 3H), 1.23 (s, 3H).
Molecular formula: $C_{20}H_{24}N_4O_4$; Molecular weight: 384.44; LC-MS (Pos, *m/z*) = 385.01[M+H]$^+$.

**Example 14: synthesis of 6-ethyl-4-(4-hydroxy-4-(methyl-*d*$_3$)piperidin-1-yl)-2-oxo-1, 2-dihydro-1,7-naphthyridine-3-carbonitrile (Compound 190)**

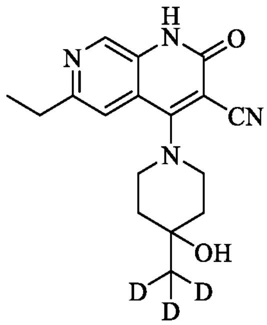

Step 1: synthesis of 4-hydroxy-4-(methyl-*d*$_3$)piperidine hydrochloride

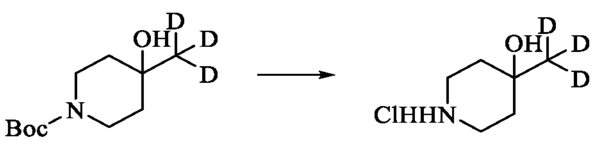

*Tert*-butyl 4-hydroxy-4-(methyl-*d*$_3$)piperidine-1-carboxylate (3.80 g, 17.4 mmol) was added to ethanol (7.60

mL), and ethanol-hydrogen chloride solution (7.60 mL) was added, and the reaction mixture was reacted at room temperature for 17 h. After the reaction completion as detected by TLC, the reaction mixture was concentrated, added with ethanol (10 mL) and ethyl acetate (30 mL), stirred at room temperature for 1.5 h, and filtered under vacuum to give the product (1.70 g, yield: 63.3%).

Step 2: synthesis of 6-ethyl-4-(4-hydroxy-4-(methyl-*d*$_3$)piperidin-1-yl)-2-oxo-1, 2-dihydro-1,7-naphthyridine-3-carbonitrile

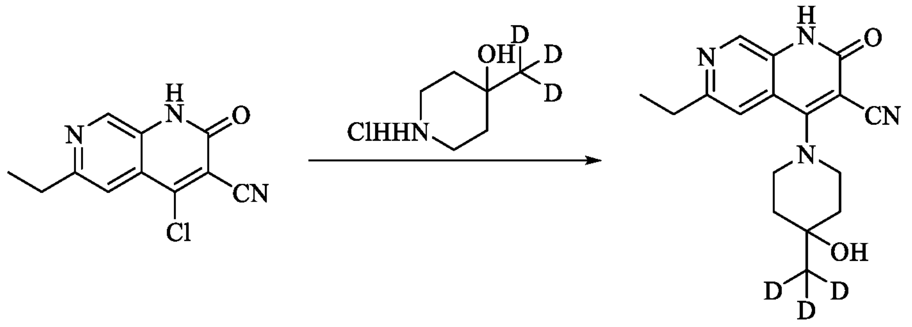

Compound 190

4-chloro-6-ethyl-2-oxo-1,2-dihydro-1,7-naphthyridine-3-carbonitrile (2.33 g, 10 mmol), 4-hydroxy-4-(methyl-*d*$_3$)piperidine hydrochloride (1.70 g, 11 mmol) and triethylamine (2.23 g, 22.00 mmol) were added to ethanol (23.3 mL), and the reaction mixture reacted at 80 °C for 2 h. After the reaction completion as detected by LC-MS, the reaction mixture was cooled to room temperature and concentrated under reduced pressure to give a crude product, which was purified by silica gel column chromatography (DCM:MeOH = 10:1) to give the product (1.65 g, yield: 52.4%).

$^1$H-NMR (400 MHz, DMSO-*d*$_6$) δ (ppm): 11.88 (s, 1H), 8.57 (s, 1H), 7.38 (s, 1H), 4.56(s, 1H), 3.56-3.83 (m, 4H), 2.73-2.84 (m, 2H), 1.69-1.83 (m, 4H), 1.10-1.30 (m, 3H).
Molecular formula: $C_{17}H_{17}D_3N_4O_2$; Molecular weight: 315.18; LC-MS (Pos, *m*/*z*) = 315.92[M+H]$^+$.

**Example 15: synthesis of 6-(1-hydroxyethyl)-4-(4-methoxy-4-(methyl-*d*$_3$)piperidin -1-yl)-2-oxo-1,2-dihydro-1,7-naphthyridine-3-carbonitrile (Compound 191)**

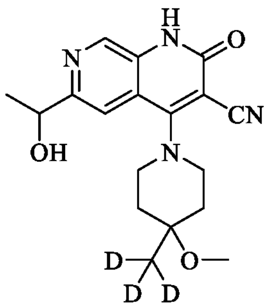

Step 1: synthesis of 6-(1-bromoethyl)-2,4-dichloro-1,7-naphthyridine-3-carbonitrile

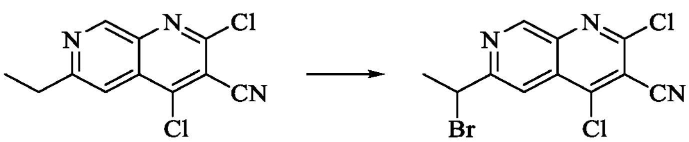

2,4-dichloro-6-ethyl-1,7-naphthyridine-3-carbonitrile (85 g, 337 mmol, 1.0 eq), NBS (120 g, 674 mmol, 2.0 eq) and AIBN (42.5 g, 5.95 mmol, 0.5 eq) were dissolved in carbon tetrachloride (850 mL), and the reaction mixture was reacted at 90 °C for 4 h. After the reaction completion as detected by LC-MS, the reaction mixture was cooled to 10-15 °C and filtered under vacuum. The filtrate was concentrated and recrystallized with PE and EA (5:1, 120 mL) to give the product (50 g, yield: 44.8%).

Step 2: synthesis of *tert*-butyl 4-methoxy-4-(methyl-$d_3$)piperidine-1-carboxylate

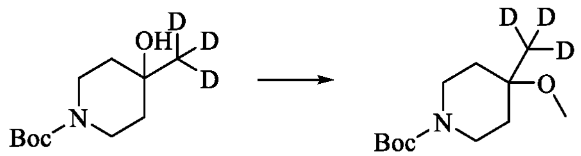

*tert*-butyl 4-hydroxy-4-(methyl-$d_3$)piperidine-1-carboxylate (16.0 g, 73.4 mmol, 1.0 eq) was added to THF (160 mL), and NaH (mass fraction 60%, 4.40 g, 110.04 mmol, 1.5 eq) was added in portions under nitrogen atmosphere. After the addition, the reaction mixture was reacted at room temperature for 0.5 h, added dropwise with iodomethane (22.91 g, 161.40 mmol, 2.2 eq), and then reacted at 30 °C for 16 h after the addition. After the reaction completion as detecetd by TLC, the reaction mixture was concentrated, added with ethyl acetate (300 mL), washed with water (100 mL × 2), dried over anhydrous sodium sulfate and filtered under vacuum. The filtrate was concentrated to give a crude product, which was purified by silica gel column chromatography (PE:EA = 10:1) to give the product (16.5 g, yield: 96.9%).

Step 3: synthesis of 4-methoxy-4-(methyl-$d_3$)piperidine hydrochloride

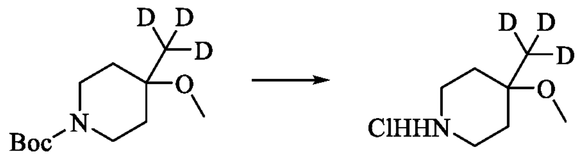

*Tert*-butyl 4-methoxy-4-(methyl-$d_3$)piperidine-1-carboxylate (16.5 g, 71.0 mmol, 1.0 eq) was added to ethanol (33 mL), and ethanol-hydrogen chloride solution (33 mL) was added, and the reaction mixture was reacted at 30 °C for 3 h. After the reaction completion as detected by TLC, the reaction mixture was concentrated, added with ethanol (30 mL) and ethyl acetate (90 mL), stirred at room temperature for 1 h, and filtered under vacuum to give the product (3.36 g, yield: 28.0%).

Step 4: synthesis of 6-(1-bromoethyl)-2-chloro-4-(4-methoxy-4-(methyl-$d_3$) piperidin-1-yl)-1,7-naphthyridine-3-carbonitrile

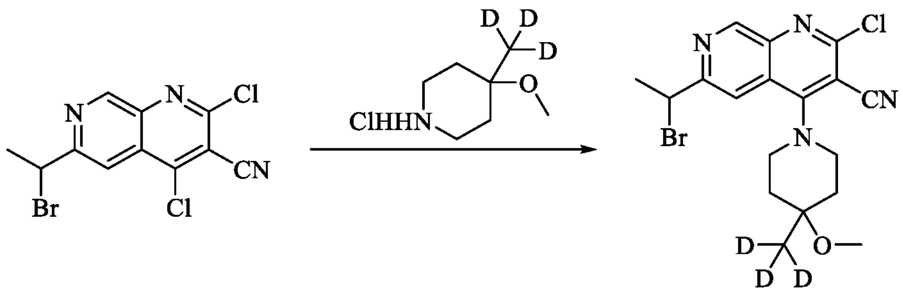

6-(1-bromoethyl)-2,4-dichloro-1,7-naphthyridine-3-carbonitrile (6.00 g, 18.12 mmol, 1.0 eq), 4-methoxy-4-(methyl-$d_3$)piperidine hydrochloride (3.36 g, 19.9 mmol, 1.1 eq) and triethylamine (4.03 g, 39.9 mmol, 2.2 eq) were added

to ethanol (60 mL), and the reaction mixture was reacted at 90 °C for 2 h. After the reaction completion as detected by LC-MS , the reaction mixture was cooled to room temperature, concentrated, added with water (150 mL), stirred for 0.5 h, and filtered under vacuum, and the filter cake was recrystallized with ethanol (50 mL) to give the product (6.20 g, yield: 80.1%).

Step 5: synthesis of 6-(1-hydroxyethyl)-4-(4-methoxy-4-(methyl-*d*$_3$)piperidin-1-yl)- 2-oxo-1,2-dihydro-1,7-naphthyridine-3-carbonitrile

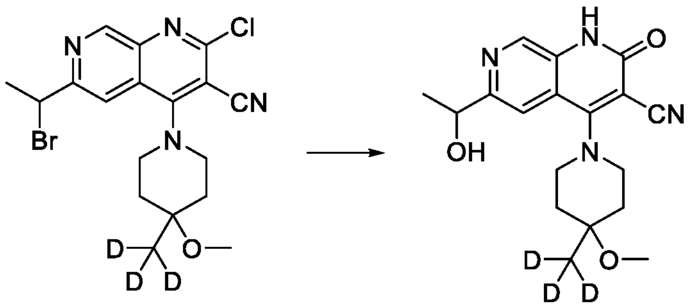

Compound 191

6-(1-bromoethyl)-2-chloro-4-(4-methoxy-4-(methyl-*d*$_3$)piperidin-1-yl)-1,7-naphthyridine-3-carbonitrile (6.20 g, 14.54 mmol, 1.0 eq) and sodium acetate (2.50 g, 30.53 mmol, 2.1 eq) were added to acetic acid (24.80 mL) and water (12.40 mL), and the reaction mixture was reacted at 100 °C for 3 h. After the presence of product at a proportion of 60% as detected by LC-MS, the reaction mixture was cooled to room temperature and concentrated under reduced pressure to give a crude product, which was purified by silica gel column chromatography (DCM:MeOH = 10:1) to give the product (3.31 g, yield: 66.0%).

$^1$H-NMR (400 MHz, DMSO-*d*$_6$) δ (ppm): 11.94 (s, 1H), 8.58 (s, 1H), 7.72 (s, 1H), 5.48-5.50 (d, 1H), 4.74-4.82 (m, 1H), 3.55-3.62 (m, 4H), 3.19 (s, 3H), 1.90-1.99 (m, 2H), 1.71-1.80 (m, 2H), 1.38-1.40 (d, 3H).
Molecular formula: $C_{18}H_{19}D_3N_4O_3$; Molecular weight: 345.42; LC-MS (Pos, *m/z*) = 346.40[M+H]$^+$.

**Example 16: synthesis of 6-acetyl-4-(4-methoxy-4-(methyl-*d*$_3$)piperidin-1-yl)-2-oxo-1, 2-dihydro-1,7-naphthyridine-3-carbonitrile (Compound 192)**

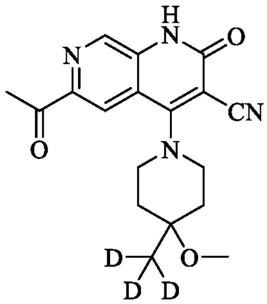

Step 1: synthesis of 6-acetyl-4-(4-methoxy-4-(methyl-*d*$_3$)piperidin-1-yl)-2-oxo-1, 2-dihydro-1,7-naphthyridine-3-carbonitrile

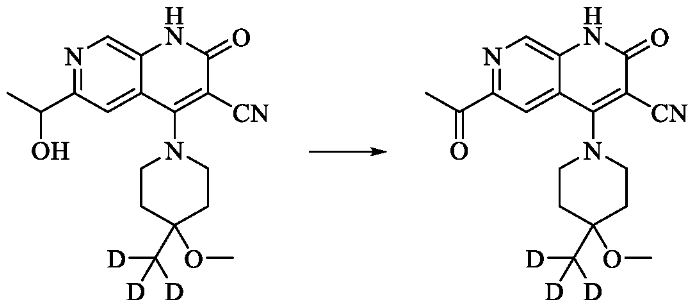

Compound 192

To a reaction flask were added dichloromethane (40.00 mL), 6-(1-hydroxyethyl)-4- (4-methoxy-4-(methyl-$d_3$)piperidin-1-yl)-2-oxo-1,2-dihydro-1,7-naphthyridine-3-carbonitrile (2.00 g, 5.8 mmol, 1.0 eq) (the product of Example 15) and Dess-Martin oxidant (2.70 g, 6.37 mmol, 1.1 eq), and the reaction mixture was reacted at 22 °C for 3 h. After the reaction completion as detected by LC-MS, the reaction mixture was added with water (25 mL) and saturated aqueous sodium thiosulfate solution (15 mL) and stirred for 0.5 h, and liquid separation was performed. The organic phase was dried over anhydrous sodium sulfate and filtered under vacuum, and the filtrate was concentrated to give a crude product, which was recrystallized with ethanol (20 mL) to give the product (1.65 g, yield: 83%).

$^1$H-NMR (400MHz, DMSO-$d_6$) δ (ppm): 12.31 (s, 1H), 8.70 (s, 1H), 8.19 (s, 1H), 3.58-3.65 (m, 4H), 3.20 (s, 3H), 2.63(s, 3H), 1.92-2.01 (m, 2H), 1.71-1.81 (m, 2H).
Molecular formula: $C_{18}H_{17}D_3N_4O_3$; Molecular weight: 343.40; LC-MS (Pos, *m/z*) = 344.35[M+H]$^+$.

**Example 17: synthesis of 1-(3-cyano-4-(4-methoxy-4-methylpiperidin-1-yl)-2-oxo-1, 2-dihydro-1,7-naphthyridin-6-yl)ethyl-2,2-dimethylbutyrate (Compound 193)**

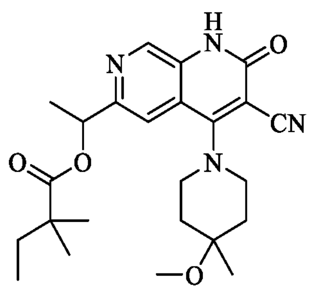

Step: synthesis of 1-(3-cyano-4-(4-methoxy-4-methylpiperidin-1-yl)-2-oxo-1, 2-dihydro-1,7-naphthyridin-6-yl)ethyl-2,2-dimethylbutyrate

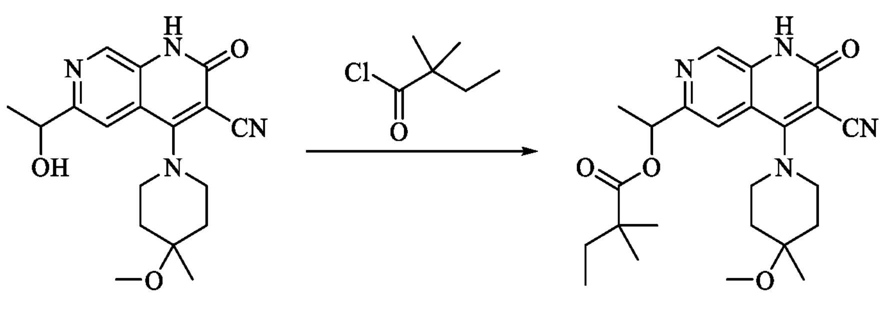

Compound 193

The product 6-(1-hydroxyethyl)-4-(4-methoxy-4-methylpiperidin-1-yl)-2-oxo-1, 2-dihydro-1,7-naphthyridine-3-carbonitrile (200 mg, 0.58 mmol, 1.0 eq) obtained in step 5 of Example 12 was dissolved in DCM (5 mL), and DMAP

(7.08 g, 0.058 mmol, 0.1 eq) and triethylamine (175.9 mg, 1.74 mmol, 3.0 eq) were added. The reaction mixture was cooled down under an ice water bath and added dropwise with 2,2-dimethylbutyrylchloride (86.5 mg, 0.64 mmol, 1.1 eq), and reacted for 2 h after the addition. After the presence of 40% of starting material as detected by TLC, the reaction mixture was supplemented with 2,2-dimethylbutyrylchloride (39 mg, 0.29 mmol, 0.5 eq), reacted for 1 h, added with water (20 mL) and extracted with DCM (20 mL × 3). The organic phases were combined, dried over anhydrous magnesium sulfate and filtered, and the filtrate was concentrated under reduced pressure to give a crude product, which was purified by preparative thin-layer chromatography (MeOH:DCM = 1:20) to give the product (120 mg, yield: 46.9%).

$^1$H-NMR (400MHz, DMSO-$d_6$) δ (ppm): 12.03 (s, 1H), 8.63 (s, 1H), 7.58 (s, 1H), 5.89-5.84 (m, 1H), 3.60-3.59 (m, 4H), 3.19 (s, 3H), 1.94-1.91 (m, 2H), 1.81-1.79 (m, 2H), 1.77-1.75 (m, 5H), 1.72-1.71 (d, 3H), 1.55-1.53 (s, 6H), 1.51 (d, 3H).

Molecular formula: $C_{24}H_{32}N_4O_4$; Molecular weight: 440.54; LC-MS (Pos, *m/z*) = 441.53[M+H]$^+$.

**Example 18: synthesis of 4-(4-hydroxy-4-methylpiperidin-1-yl)-6-(1-hydroxyethyl) -2-oxo-1,2-dihydro-1,7-naphthyridine-3-carbonitrile (Compound 194)**

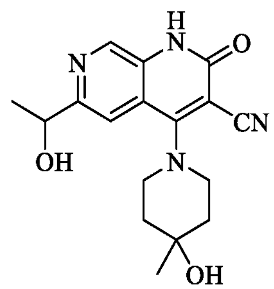

Steps:

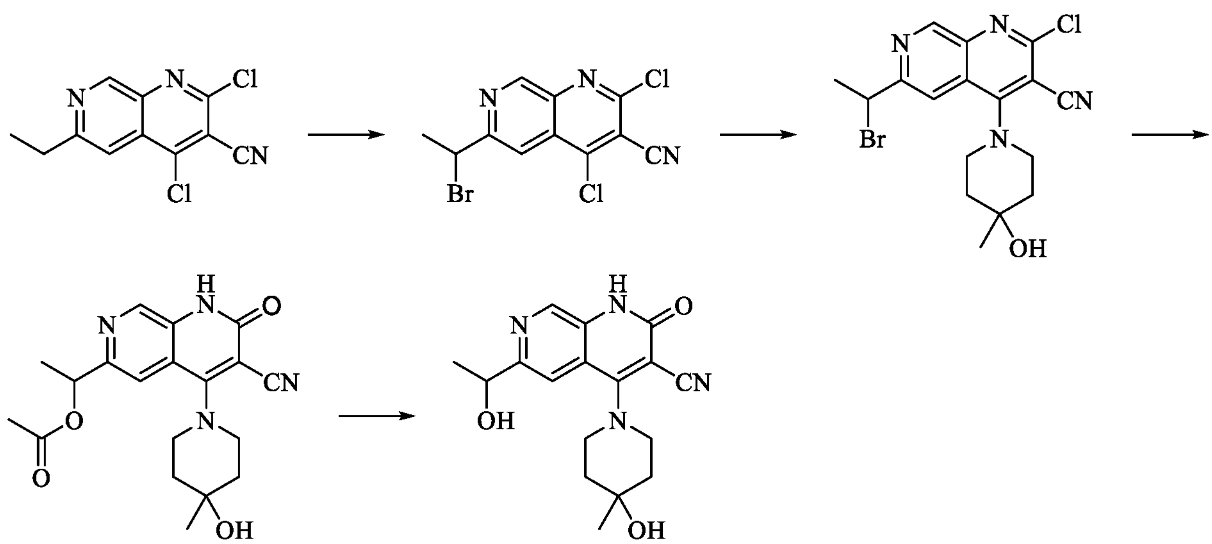

Step 1: synthesis of 6-(1-bromoethyl)-2,4-dichloro-1,7-naphthyridine-3-carbonitrile

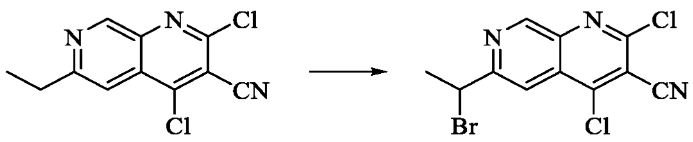

The starting material 2,4-dichloro-6-ethyl-1,7-naphthyridine-3-carbonitrile (3 g, 11.9 mmol, 1.0 eq), NBS (4.23 g, 23.8 mmol, 2.0 eq) and AIBN (977 mg, 5.95 mmol, 0.5 eq) were dissolved in carbon tetrachloride (60 mL), and the reaction mixture was reacted at 90 °C for 2 h. After the reaction completion as detected by LC-MS, the reaction mixture was concentrated under reduced pressure to give a crude product, which was purified by silica gel column chromatography (PE:EA = 20:1) to give the product (3.2 g, yield: 81%).

Step 2: synthesis of 6-(1-bromoethyl)-2-chloro-4-(4-hydroxy-4-methylpiperidin-1 -yl)-1,7-naphthyridine-3-carbonitrile

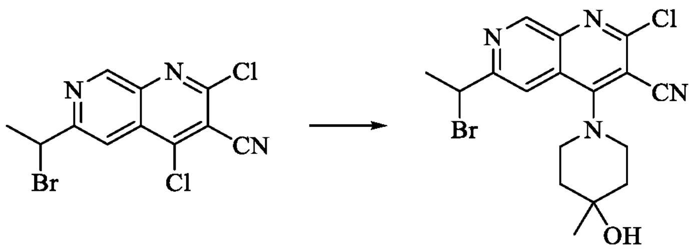

6-(1-bromoethyl)-2,4-dichloro-1,7-naphthyridine-3-carbonitrile (3.2 g, 9.67 mmol, 1.0 eq), 4-methylpiperidin-4-ol hydrochloride (1.75 g, 11.6 mmol, 1.2 eq) and DIPEA (3.75 g, 29.01 mmol, 3.0 eq) were dissolved in ethanol (30 mL) and the reaction mixture was reacted at 90 °C for 90 min. After the reaction completion as detected by TLC, the reaction mixture was concentrated under reduced pressure to give a crude product, which was purified by silica gel column chromatography (PE:EA = 2:1) to give the product (1.1 g, yield: 27%).

Step 3: synthesis of 1-(3-cyano-4-(4-hydroxy-4-methylpiperidin-1-yl)-2-oxo-1, 2-dihydro-1,7-naphthyridin-6-yl)ethyl acetate

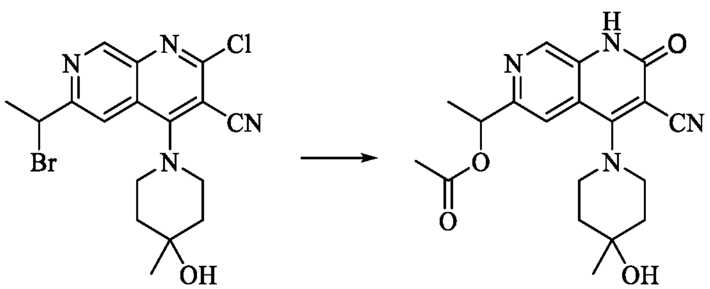

The starting materials 6-(1-bromoethyl)-2-chloro-4-(4-hydroxy-4-methylpiperidin- 1-yl)-1,7-naphthyridine-3-carbonitrile (1.1 g, 2.68 mmol, 1.0 eq) and sodium acetate (429 mg, 5.36 mmol, 2.0 eq) were dissolved in water (2 mL) and acetic acid (8 mL), and the reaction mixture was reacted at 80 °C for 22 h. After the reaction completion as detected by LC-MS, the reaction mixture was concentrated under reduced pressure to give the product.

Step 4: synthesis of 4-(4-hydroxy-4-methylpiperidin-1-yl)-6-(1-hydroxyethyl)- 2-oxo-1,2-dihydro-1,7-naphthyridine-3-carbonitrile

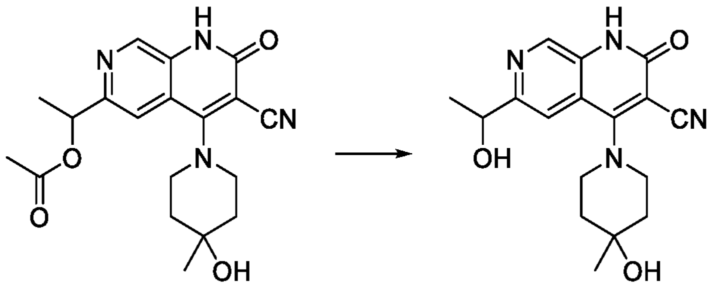

Compound 194

1-(3-cyano-4-(4-hydroxy-4-methylpiperidin-1-yl)-2-oxo-1,2-dihydro- 1,7-naphthyridin-6-yl)ethyl acetate (994 mg, 2.68 mmol, 1.0 eq) and potassium carbonate (222 mg, 1.61 mmol, 0.6 eq) were dissolved in water (5 mL) and ethanol (5 mL), and the reaction mixture was reacted at 60 °C for 2.5 h. After the reaction completion as detected by TLC, the reaction mixture was concentrated under reduced pressure to give a crude product, which was purified by silica gel column chromatography (MeOH: DCM = 1:20) to give the product (470 mg, yield: 53%).

$^1$H-NMR (300 MHz, DMSO-$d_6$) δ (ppm): 11.92 (s, 1H), 8.58 (s, 1H), 7.73 (s, 1H), 5.5 (s, 1H), 4.77-4.79 (d, 1H), 4.61 (s, 1H), 3.58-3.72 (m, 4H), 1.68-1.77 (m, 4H), 1.37-1.40 (d, 3H), 1.25 (s, 3H) .

Molecular formula: $C_{17}H_{20}N_4O_3$; Molecular weight: 328.37; LC-MS (Pos, *m/z*) = 329.35[M+H]$^+$.

**Example 19: synthesis of 1-(3-cyano-4-(4-methoxy-4-methylpiperidin-1-yl)-2-oxo-1, 2-dihydro-1,7-naphthyridin-6-yl)ethyl cyclopropanecarboxylate (Compound 195)**

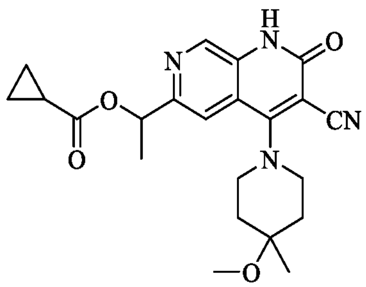

Step: synthesis of 1-(3-cyano-4-(4-methoxy-4-methylpiperidin-1-yl)-2-oxo-1, 2-dihydro-1,7-naphthyridin-6-yl)ethyl cyclopropanecarboxylate

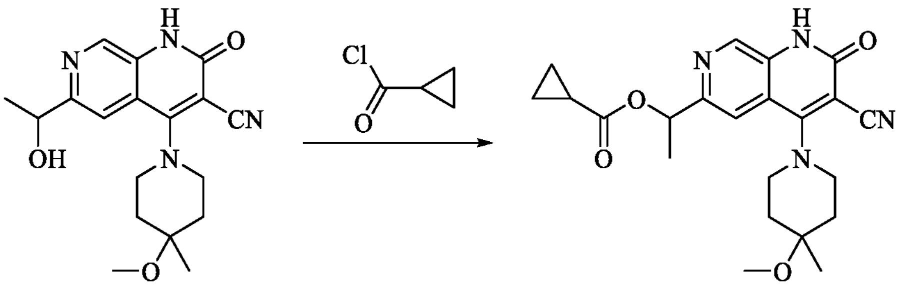

Compound 195

The product 6-(1-hydroxyethyl)-4-(4-methoxy-4-methylpiperidin-1-yl)-2-oxo-1,2-dihydro-1,7-naphthyridine-3-carbonitrile (330 mg, 0.96 mmol, 1.0 eq) obtained in step 5 of Example 12 was dissolved in DCM (5 mL), and DMAP (11.7 mg, 0.096 mmol, 0.1 eq) and triethylamine (291.2 mg, 2.88 mmol, 3.0 eq) were added. The reaction mixture was cooled down under an ice water bath, added dropwise with cyclopropanecarbonyl chloride (130.6 mg, 1.25 mmol, 1.3 eq) and reacted for 2 h. After the presence of a small amount of remaining starting material as detected by TLC, the

reaction mixture was added with water (20 mL) and extracted with DCM (20 mL × 3). The organic phases were combined, dried over anhydrous magnesium sulfate and filtered, and the filtrate was concentrated under reduced pressure to give a crude product, which was purified by preparative thin-layer chromatography (MeOH:DCM = 1:20) to give the product (260 mg, yield: 65.9%).

$^1$H-NMR (400MHz, DMSO-$d_6$) δ (ppm): 12.03 (s, 1H), 8.63 (s, 1H), 7.54 (s, 1H), 5.90-5.84 (m, 1H), 3.59-3.58 (m, 4H), 3.19 (s, 3H), 1.95-1.91 (m, 2H), 1.80-1.60 (m, 3H), 1.55-1.53 (d, 3H), 1.23 (s, 3H), 0.97-0.92 (m, 4H). Molecular formula: $C_{22}H_{26}N_4O_4$; Molecular weight: 410.47; LC-MS (Pos, $m/z$) = 411.13[M+H]$^+$.

**Example 20: synthesis of 1-(3-cyano-4-(4-methoxy-4-methylpiperidin-1-yl)-2-oxo-1, 2-dihydro-1,7-naphthyridin-6-yl)ethyl valerate (Compound 196)**

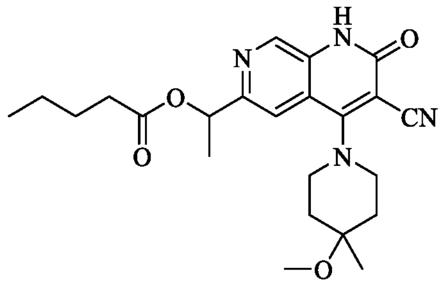

Step: synthesis of 1-(3-cyano-4-(4-methoxy-4-methylpiperidin-1-yl)-2-oxo-1,2 - dihydro-1,7-naphthyridin-6-yl)ethyl valerate

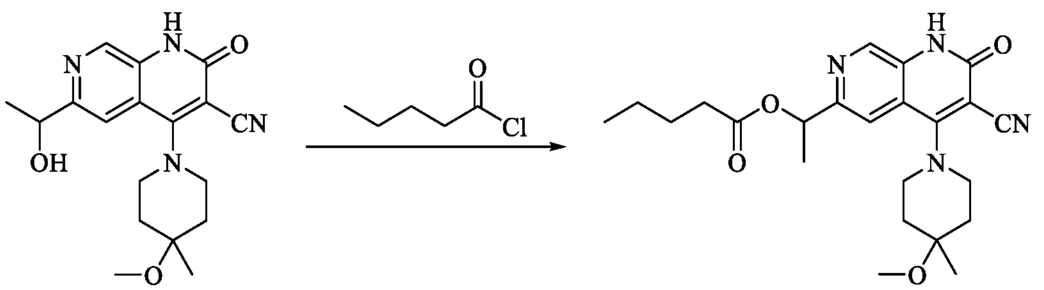

Compound 196

The product 6-(1-hydroxyethyl)-4-(4-methoxy-4-methylpiperidin-1-yl)-2-oxo-1,2-dihydro-1,7-naphthyridine-3-carbonitrile (330 mg, 0.96 mmol, 1.0 eq) obtained in step 5 of Example 12 was dissolved in DCM (5 mL), and DMAP (11.7 mg, 0.096 mmol, 0.1 eq) and triethylamine (291.2 mg, 2.88 mmol, 3.0 eq) were added. The reaction mixture was cooled down under an ice water bath, added dropwise with valeryl chloride (151 mg, 1.25 mmol, 1.3 eq) and reacted for 2 h. After the presence of a small amount of remaining starting material as detected by TLC, the reaction mixture was added with water (20 mL) and extracted with DCM (20 mL × 3). The organic phases were combined, dried over anhydrous magnesium sulfate and filtered, and the filtrate was concentrated under reduced pressure to give a crude product, which was purified by preparative thin-layer chromatography (MeOH:DCM = 1:20) to give the product (246 mg, yield: 60%).

$^1$H-NMR (400MHz, DMSO-$d_6$) δ (ppm): 12.02 (s, 1H), 8.63 (s, 1H), 7.56 (s, 1H), 5.92-5.85 (m, 1H), 3.61-3.59 (m, 4H), 3.19 (s, 3H), 2.39-2.33 (m, 2H), 1.95-1.90 (m, 2H), 1.80-1.77 (m, 2H), 1.58 (m, 5H), 1.54-1.52 (m, 2H), 1.50-1.48 (s, 3H), 1.30 (m, 3H).
Molecular formula: $C_{23}H_{30}N_4O_4$; Molecular weight: 426.52; LC-MS (Pos, $m/z$) = 427.18[M+H]$^+$.

**Example 21: Synthesis of disodium 1-(3-cyano-4-(4-methoxy-4-methylpiperidin-1 -yl)-2-oxo-1,2-dihydro-1,7-naphthyridin-6-yl)ethyl phosphate**

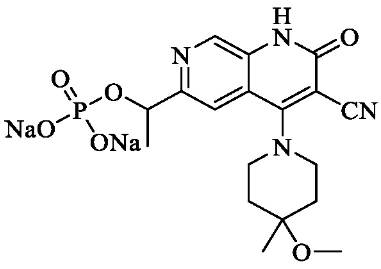

Compound 204

Steps:

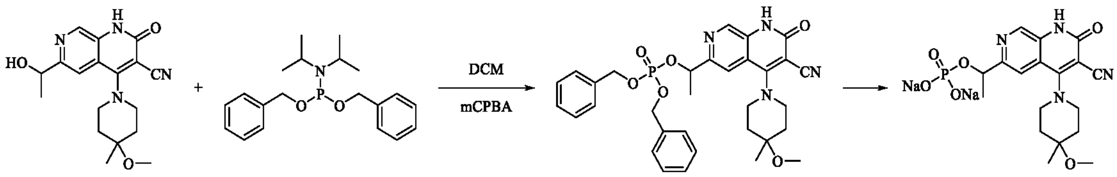


Step 1: synthesis of dibenzyl(1-(3-cyano-4-(4-methoxy-4-methylpiperidin-1-yl) -2-oxo-1,2-dihydro-1,7-naphthyridin-6-yl)ethyl)phosphate

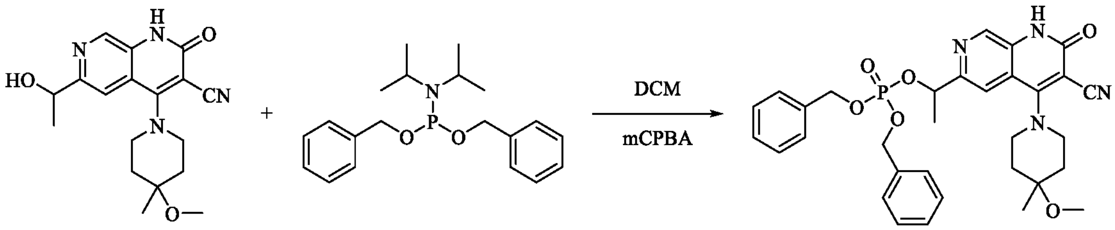


The product 6-(1-hydroxyethyl)-4-(4-methoxy-4-methylpiperidin-1-yl)-2-oxo-1, 2-dihydro-1,7-naphthyridine-3-carbonitrile (675 mg, 1.97 mmol, 1.0 eq) obtained in step 5 of Example 12 and 1*H*-tetrazole (276.2 mg, 3.94 mmol, 2.0 eq) were dissolved in DCM (10 mL), and the reaction mixture was cooled to 0 °C under nitrogen atmosphere and added dropwise with dibenzyl diisopropylphosphoramidite (1 g, 2.96 mmol, 1.5 eq). After the dropwise addition, the reaction mixture was reacted at room temperature for 48 h, cooled to 0 °C, and then added dropwise with a solution of mCPBA (mass fraction 85%, 510.8 mg, 2.96 mmol, 1.5 eq) in dichloromethane (10 mL). Then the reaction mixture was reacted at 0 °C for 0.5-1 h, added with saturated sodium carbonate solution (30 mL) and stirred for 5 min, and liquid separation was performed. The aqueous phase was extracted with dichloromethane (30 mL × 2), and the organic phases were combined, washed successively with 5% sodium sulfite solution (30 mL), saturated sodium carbonate solution (30 mL) and brine (30 mL), dried over anhydrous magnesium sulfate and filtered. The filtration was concentrated under reduced pressure to give a crude product, which was purified by preparative thin-layer chromatography (MeOH:DCM = 1:20) to give the product (148 mg, yield: 12.5%).

Step 2: synthesis of potassium 1-(3-cyano-4-(4-methoxy-4-methylpiperidin-1-yl)-2-oxo-1,2-dihydro-1,7-naphthyridin-6-yl)phosphate

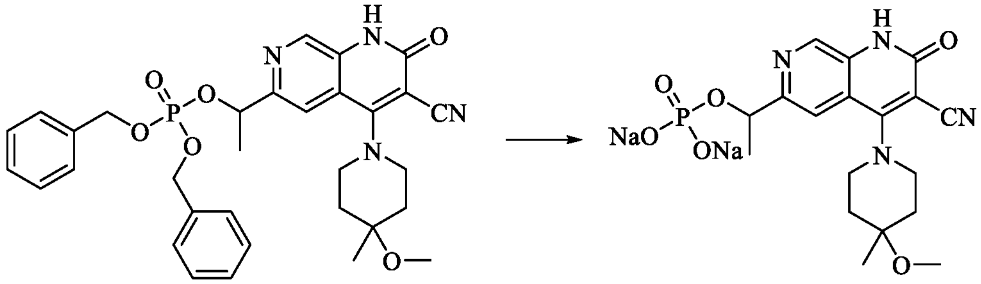

Dibenzyl (1-(3-cyano-4-(4-methoxy-4-methylpiperidin-1-yl)-2-oxo-1,2-dihydro-1, 7-naphthyridin-6-yl)ethyl)phosphate (130 mg, 0.22 mmol, 1.0 eq) was dissolved in DCM (3 mL), and TFA (3 mL) was added. The reaction mixture was reacted at room temperature for 48 h, concentrated under reduced pressure at room temperature, added with methanol (5 mL) and water (5 mL). Then the reaction mixture was cooled down under an ice bath and adjusted to pH of about 8 with solid sodium carbonate, and solids were precipitated. The resulting mixture was filtered, and the filter cake was dissolved in water (10 mL), purified by preparative HPLC (water + 0.1% aqueous ammonia), and lyophilized to give the product (54.7 mg, yield: 53.3%) in the form of a light yellow solid.

$^1$H-NMR (300 MHz, $D_2O$) $\delta$ (ppm): 8.38 (s, 1H), 7.74 (s, 1H), 5.20-5.15 (m, 1H), 3.75 (m, 4H), 3.20 (s, 3H), 1.95-1.79 (m, 4H), 1.42-1.40 (d, 3H), 1.21 (s, 3H).
Molecular formula: $C_{18}H_{21}K_2N_4O_6P$; Molecular weight: 498.56; LC-MS (Pos, *m/z*) = 423.23[M+H]$^+$.

The present invention can be better understood according to the following experimental examples. However, it is easily understood by those skilled in the art that the contents described in experimental examples are only used to illustrate the present invention, and should not and will not limit the present invention described in detail in the claims.

**Experimental Example 1: PDE9 Enzymatic Evaluation**

Test samples: the compounds disclosed herein (prepared according to corresponding examples of the present invention) and the compound I-11 of patent WO2017019723A1 (prepared by referring to the examples for synthesis in WO2017019723A1) having the following structural formula:

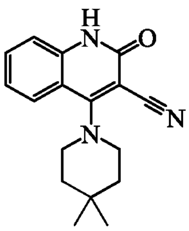

(I-11)

1. Experimental materials and instruments

PDE9A2 enzyme (BPS, Cat. No. 60090)
384 well plate (Perkin Elmer, Cat. No. 6007279)

2. Experimental procedures

Preparation of the compounds: Compounds were formulated into 10 mM compound stock solution in DMSO for long term storage. The obtained compound stock solution was 100-fold diluted with DMSO to give 100 $\mu$M compound mother liquor, which was 3-fold diluted with DMSO to give 8-10 concentration gradients of diluted compound mother liquor (100×).
Incubation with compound: Diluted compound mother liquor was pipetted into a 384-well plate using an ultramicro-liquid pipetting system (Echo); 200 nL of the diluted compound mother liquor and 10 $\mu$L of PDE9A2 enzyme solution were added to each compound well; after centrifugation at 1000 rpm for 1 min, the mixtures were incubated at room temperature for 15 min. Subsequently, 10 $\mu$L of the substrate mixture was added, and after centrifugation at 1000

rpm for 1 min, the resulting mixture was incubated at room temperature with oscillating for 30 min. Finally, stop solution was added to end the reaction, and the resulting reaction system was incubated at room temperature with oscillating for 60 min. In the maximum reading well (Max), the compound was replaced by solvent; in the minimum reading well (Min), the compound and the enzyme solution were replaced by solvent.
Detection: the fluorescence readings (F) at 480 nm/535 nm were detected using a microplate reader.
Calculation: The inhibition rate was calculated according to the following formula and $IC_{50}$ was fitted using GraphPad Prism 5.0:

$$\text{Inhibition rate (\%)} = \frac{FMax - Fcompound}{FMax - FMin} \times 100\%$$

3. The experiment results are shown in Table 2 below:

Table 2

| Test samples | PDE9A2 $IC_{50}$ (nM) |
|---|---|
| Compound I-11 | 70 |
| Compound 163 | 50 |
| Compound 164 | 25 |
| Compound 165 | 24 |
| Compound 166 | 19 |
| Compound 167 | 11 |
| Compound 177 | 15 |
| Compound 183 | 48 |
| Compound 184 | 19 |
| Compound A | 61 |
| Compound B | 3 |
| Compound 191 | 5 |
| Compound 192 | 64 |
| Compound 194 | 37 |

As can be seen from Table 2, the compounds disclosed herein have very good PDE9 enzymatic inhibition activity and thus have potential value for clinical application.

**Experimental Example 2:** Evaluation of Dog Liver Microsomal Stability of Compounds Disclosed Herein

**Test samples:** the compounds disclosed herein and the compound 1-8 of patent WO2017019723A1 (prepared by referring to the examples for synthesis in WO2017019723A1) having the following structural formula:

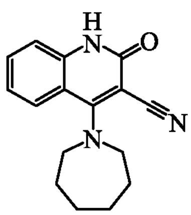

(I-8)

**Composition of the incubation system:**

| Substances to be added | Initial concentration | Proportion (%) | Final concentration |
|---|---|---|---|
| Phosphate buffer | 100 mM | 50 | 50 mM |
| $MgCl_2$ | 20 mM | 5 | 1 mM |
| Liver microsome | 20 mg protein/mL | 2.5 | 0.5 mg protein/mL |
| Water to be supplemented | - | 22.5 | - |
| Compound | 10 μM | 10 | 1 μM |
| β-NADPH | 10 mM | 10 | 1 mM |

**Compound preparation:**

An appropriate amount of the compound was precisely weighed out and dissolved in DMSO to prepare a 5.0 mM stock solution. The 5.0 mM stock solution was diluted to 1.0 mM with DMSO, and then diluted with water to 10 μM compound working solution for later use (DMSO content in the reaction system: 0.1% v/v).

**Experimental procedures:**

(1) The liver microsomes (20 mg protein/mL) were taken out from a -80 °C refrigerator, pre-incubated on a 37 °C water bath thermostatic oscillator for 3 min, and thawed for use.
(2) A mixed solution of the incubation system (without compound and β-NADPH) was prepared according to "composition of the experimental incubation system" described above, and pre-incubated on a 37 °C water bath thermostatic oscillator for 2 min.
(3) Control group (without β-NADPH) 30 μL of water and 30 μL of compound working solution (10 μM) were added to 240 μL of the mixed solution of the incubation system in step (2), and the mixture was vortexed for 30 s and mixed well; the total volume of the mixture was 300 μL; the sample was duplicated. The mixture was placed on a 37 °C water bath thermostatic oscillator for incubation, and timing was started; the sampling was conducted at 0 min and 60 min.
(4) Sample group: 70 μL of β-NAOPP solution (10 mM) and 70 μL of compound working solution (10 μM) were added to 560 μL of the mixed solution in step (2), and the total volume of the mixture was 700 μL; the mixture was vortexed for 30 s and mixed well; the sample was duplicated. The mixture was placed on a 37 °C water bath thermostatic oscillator for incubation, and timing was started; the sampling was conducted at 0 min, 5 min, 10 min, 20 min, 30 min and 60 min.
(5) The mixture was vortexed for 3 min, and centrifuged at 4000 rpm for 10 min.
(6) 50 μL of the supernatant was taken and added to 150 μL of water, and the resulting mixture was vortexed and mixed well before LC/MS/MS analysis.

**Data analysis:**

The half-life ($t_{1/2}$) and clearance rate (Cl) were calculated using the following first-order kinetic formula:

$$C_t = C_0 * e^{-kt}$$

$$t_{1/2} = \ln 2/k = 0.693/k$$

$$Cl_{int} = V_d * k$$

$$V_d = 1/\text{protein content in liver microsomes}$$

Note: k denotes the slope of the logarithm of the remaining amount of a compound vs. time, $V_d$ denotes apparent volume of distribution, and Co denotes compound concentration at 0 h.

**Results:**

Table 3. Experiment on stability of the compounds disclosed herein in dog liver microsomes

| | $CL_{int}$ (mL/min/mg) | $t_{1/2}$ (min) |
|---|---|---|
| Compound 1-8 | 0.1158 | 12 |
| Compound 166 | 0.0034 | 408 |
| Compound 167 | 0.0100 | 139 |
| Compound B | 0.0016 | 866 |
| Compound 204 | 0.0028 | 495 |

As can be seen from Table 3, the compounds disclosed herein have a lower clearance rate in dog liver microsomes than the compound in prior art.

## Claims

1. A compound of general formula (I) or a pharmaceutically acceptable salt, an isomer, a deuterated compound, a metabolite or a prodrug thereof:

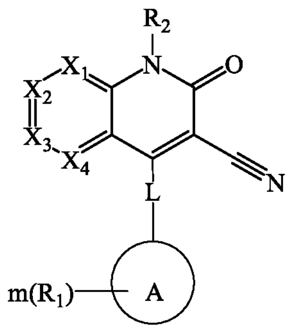

(I)

wherein $X_1$, $X_2$ and $X_4$ are each independently selected from CR' and N, $X_3$ is selected from $CR_3$ and N, and at least one of $X_1$, $X_2$, $X_3$ and $X_4$ is N, wherein the N heteroatom may be optionally oxidized to

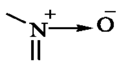

;

R' and $R_3$, at each occurrence, are each independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, $(C_{1-6}$ alkyl$)_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, $(C_{1-6}$ alkyl$)_2$aminocarbonyl, 4-6 membered heterocyclylcarbonyl and 5-6 membered heteroaryl-oxy, wherein the $C_{1-6}$ alkyl, $C_{1-6}$ alkoxy, $C_{1-6}$ alkylamino, $(C_{1-6}$ alkyl$)_2$amino, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, $(C_{1-6}$ alkyl$)_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, $(C_{1-6}$ alkyl$)_2$amino, $C_{1-6}$ alkylcarbonylamino, $C_{1-6}$ alkyl-

sulfonylamino, $C_{1-6}$ alkylcarbonyloxy, $C_{3-6}$ cycloalkyl, $C_{3-6}$ cycloalkylcarbonyloxy, $C_{2-8}$ alkynyl, halogenated $C_{1-6}$ alkyl, $C_{2-8}$ alkenyl, halogenated $C_{1-6}$ alkoxy,

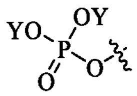,

4-6 membered heterocyclyl unsubstituted or optionally substituted with one or more independent substituents, and heteroaryl unsubstituted or optionally substituted with one or more independent substituents;
the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-6}$ alkyl and $C_{1-6}$ alkoxy;
Y is selected from metal ions and organic ammonium ions, and is preferably $Na^+$, $K^+$ or $NH_4^+$;
L is a bond or $-NH-(CH_2)t-$, wherein t is 0, 1, 2 or 3;
ring A is 3-12 membered heterocyclyl, 5-10 membered heteroaryl, 3-12 membered cycloalkyl or 3-12 membered cycloalkenyl, wherein the 3-12 membered heterocyclyl has heteroatoms selected from one of or any combinations of O, S and N, the S atom may be optionally oxidized to S(O) or $S(O)_2$, the C atom may be optionally oxidized to C(O), the N heteroatom may be optionally oxidized to

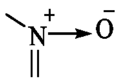,

and the 5-10 membered heteroaryl has heteroatoms selected from one of or any combinations of O, S and N;
each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, $(C_{1-6}$ alkyl$)_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, $(C_{1-6}$ alkyl)2amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, $(C_{1-6}$ alkyl$)_2$amino, $C_{1-6}$ alkylcarbonylamino and $C_{1-6}$ alkylsulfonylamino;
m is 0, 1, 2 or 3; and
$R_2$ is selected from hydrogen, $C_{1-6}$ alkyl, $C_{2-8}$ alkenyl, $C_{2-8}$ alkynyl and halogenated $C_{1-6}$ alkyl;

with the proviso that

(1) when $X_2$ is N, $X_1$ is CH, $X_3$ is $CR_3$, and $X_4$ is CH, ring A is

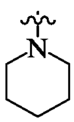 or 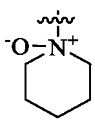:

(i) when

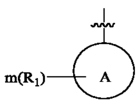 is 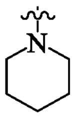, 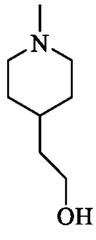, 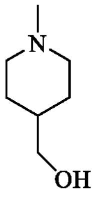, 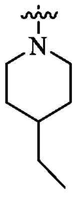, 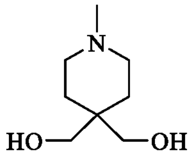 or 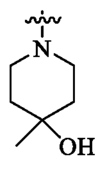,

$R_3$ is not H;

(ii) when

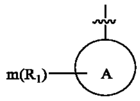 is 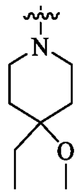,

$R_3$ is not Cl; and
(iii) when

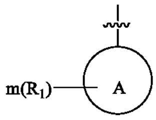 is 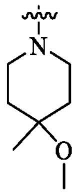,

$R_3$ is not H,

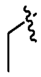, 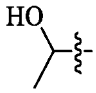, 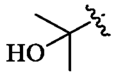, 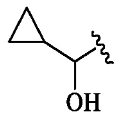,

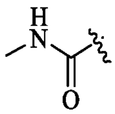, 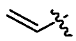, 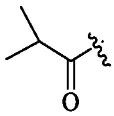, 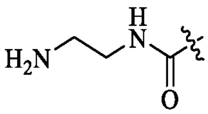, 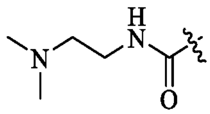, 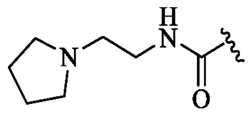,

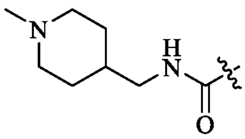, 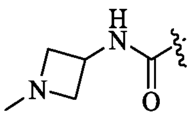, 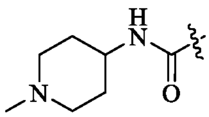, 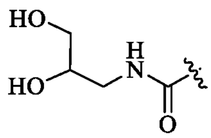, 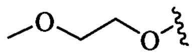,

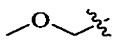, 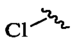, 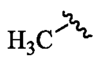, 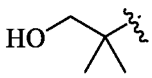 or 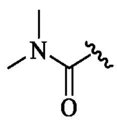;

(2) when $X_2$ and $X_4$ are each N, $X_1$ is CH, $X_3$ is $CR_3$, ring A is

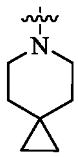,

and m is 0, $R_3$ is not methylthio;
(3) when $X_4$ is N, $X_1$ and $X_2$ are each CH, $X_3$ is $CR_3$, ring A is

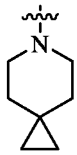 or 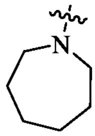,

and m is 0, $R_3$ is not hydrogen; and
(4) when $X_1$ is N, $X_2$ and $X_4$ are CR', $X_3$ is $CR_3$, ring a is pyrrolidinyl or

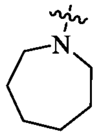,

and m is 0, $R_2$ is not H or $C_{1-6}$ alkyl;

preferably, when $X_2$ is N, $X_1$ is CH, $X_3$ is $CR_3$, $X_4$ is CH, and

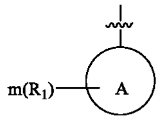

is

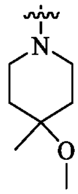,

$R_3$ is selected from isopropyl, cyclopropyl, hydroxymethyl,

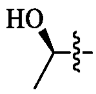, 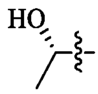, $C_{1-6}$ alkylcarbonyl, $C_{1-6}$ alkylcarbonyloxy $C_{1-6}$ alkyl, $C_{1-6}$ alkyl substituted with

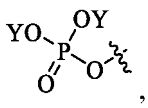,

$C_{3-6}$ cycloalkylcarbonyloxy $C_{1-6}$ alkyl and deuterated $C_{1-6}$ alkyl; and
preferably, when $X_2$ is N, $X_1$ is CH, $X_3$ is $CR_3$, and $X_4$ is CH, ring A is

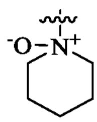.

**2.** The compound or the pharmaceutically acceptable salt, the isomer, the deuterated compound, the metabolite or

the prodrug thereof according to claim 1, having a structure shown as general formula (II):

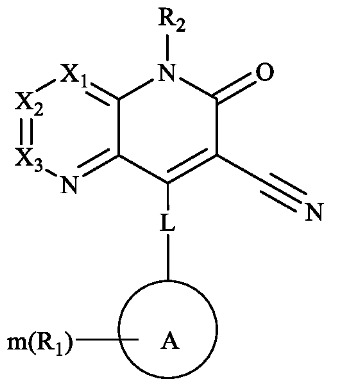

(II)

wherein, $X_1$, $X_2$, $X_3$, $R_1$, $R_2$, ring A, L and m are defined as in claim 1;
with the proviso that

(1) when $X_2$ is N, $X_1$ is CH, $X_3$ is $CR_3$, ring A is

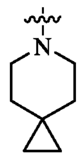

,

and m is 0, $R_3$ is not methylthio; and
(2) When $X_1$ and $X_2$ are each CH, $X_3$ is $CR_3$, ring A is

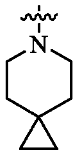

or

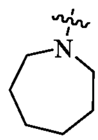

,

and m is 0, $R_3$ is not hydrogen.

3. The compound or the pharmaceutically acceptable salt, the isomer, the deuterated compound, the metabolite or the prodrug thereof according to claim 2, having a structure shown as general formula (III):

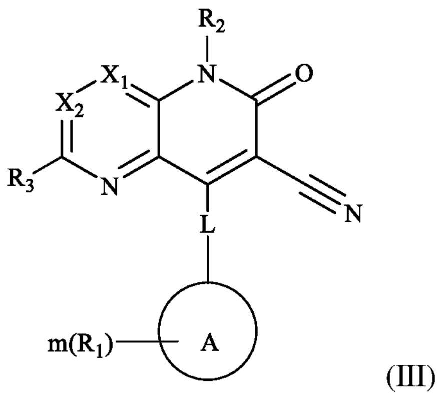

(III)

wherein, $X_1$, $X_2$, $R_1$, $R_2$, $R_3$, ring A, L and m are defined as in claim 2;

with the proviso that

(1) when $X_2$ is N, $X_1$ is CH, ring A is

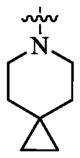

,

and m is 0, $R_3$ is not methylthio; and
(2) when $X_1$ and $X_2$ are each CH, ring A is

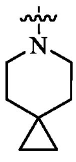

or

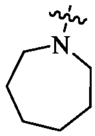

,

and m is 0, $R_3$ is not hydrogen.

**4.** The compound or the pharmaceutically acceptable salt, the isomer, the deuterated compound, the metabolite or the prodrug thereof according to claim 3,
wherein,

$X_1$ and $X_2$ are each independently selected from CR' and N, and the N heteroatom may be optionally oxidized to

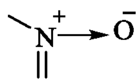

;

R' and $R_3$, at each occurrence, are each independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkoxy, $C_{2-8}$ alkenyl, $C_{2-8}$ alkynyl, $C_{1-6}$ alkylsulfonyl, $C_{1-6}$ alkylthio, $C_{3-6}$ cycloalkyl, 4-6 membered heterocyclyl, 5-6 membered heteroaryl, aryl, $C_{1-6}$ alkylcarbonyl, aminocarbonyl, $C_{1-6}$ alkylaminocarbonyl, ($C_{1-6}$ alkyl)$_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino, $C_{1-6}$ alkylsulfonylamino, $C_{1-6}$ alkylcarbonyloxy, $C_{3-6}$ cycloalkylcarbonyloxy, $C_{3-6}$ cycloalkyl, $C_{2-8}$ alkynyl, halogenated $C_{1-6}$ alkyl, $C_{2-8}$ alkenyl, halogenated $C_{1-6}$ alkoxy, 4-6 membered heterocyclyl unsubstituted or optionally substituted with one or more independent substituents, and heteroaryl unsubstituted or optionally substituted with one or more independent substituents;
the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-6}$ alkyl and $C_{1-6}$ alkoxy;
L is a bond or -NH-($CH_2$)t-, wherein t is 0, 1, 2 or 3;
ring A is 4-7 membered monocyclic heterocyclyl, wherein the 4-7 membered monocyclic heterocyclyl has heteroatoms selected from one of or combinations of two of O, S and N, and contains at least one N, ring A is connected to L via the N atom, the S atom may be optionally oxidized to $S(O)_2$, the C atom may be optionally oxidized to C(O), and the N atom may be optionally oxidized to

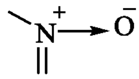;

preferably, ring A is selected from

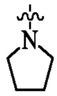, 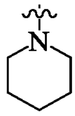, 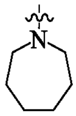 and 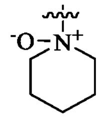;

each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, ($C_{1-6}$ alkyl)2amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with one or more groups selected fro m hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino and $C_{1-6}$ alkylsulfonylamino;
$R_2$ is selected from hydrogen, $C_{1-6}$ alkyl, $C_{2-8}$ alkenyl, $C_{2-8}$ alkynyl and halogenated $C_{1-6}$ alkyl; and
m is 0, 1 or 2;

with the proviso that
when $X_1$ and $X_2$ are each CH, ring A is

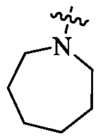,

and m is 0, $R_3$ is not hydrogen.

**5.** The compound or the pharmaceutically acceptable salt, the isomer, the deuterated compound, the metabolite or the prodrug thereof according to claim 4,
wherein,

R' and $R_3$, at each occurrence, are each independently selected from hydrogen, deuterium, halogen, $C_{1-4}$ alkyl, $C_{3-6}$ cycloalkyl, 5-6 membered heteroaryl, aryl, $C_{1-4}$ alkoxy, $C_{2-6}$ alkenyl, $C_{1-4}$ alkylaminocarbonyl, $C_{1-4}$ alkylcarbonyl, ($C_{1-4}$ alkyl)$_2$aminocarbonyl and aminocarbonyl, wherein the $C_{1-4}$ alkyl, $C_{3-6}$ cycloalkyl, 5-6 membered heteroaryl, aryl, $C_{1-4}$ alkoxy, $C_{2-6}$ alkenyl, $C_{1-4}$ alkylaminocarbonyl, $C_{1-4}$ alkylcarbonyl, ($C_{1-4}$ alkyl)2aminocarbonyl and aminocarbonyl are unsubstituted or optionally substituted with one or more groups independently selected from hydroxy, amino, $C_{1-4}$ alkyl, $C_{1-4}$ alkoxy, $C_{3-6}$ cycloalkyl, $C_{1-4}$ alkylamino, $C_{1-6}$ alkylcarbonyloxy, $C_{3-6}$ cycloalkylcarbonyloxy, ($C_{1-4}$ alkyl)$_2$amino, and 4-6 membered heterocyclyl unsubstituted or substituted with $C_{1-4}$ alkyl;
L is a bond;
ring A is

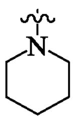 or 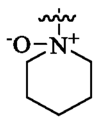;

each $R_1$ is independently selected from hydrogen, deuterium, $C_{1-4}$ alkyl and $C_{1-4}$ alkoxy;
$R_2$ is selected from hydrogen and $C_{1-4}$ alkyl; and

m is 0, 1 or 2.

**6.** The compound or the pharmaceutically acceptable salt, the isomer, the deuterated compound, the metabolite or the prodrug thereof according to claim 3, wherein,

$X_1$ and $X_2$ are each independently selected from CR' and N, and the N heteroatom may be optionally oxidized to

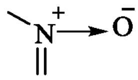;

R' and $R_3$, at each occurrence, are each independently selected from hydrogen, deuterium, hydroxy, amino, carboxyl, cyano, nitro, halogen, $C_{1-4}$ alkyl, $C_{1-4}$ alkoxy, $C_{1-4}$ alkylamino, ($C_{1-4}$ alkyl)$_2$amino, halogenated $C_{1-4}$ alkyl, halogenated $C_{1-4}$ alkoxy, $C_{2-6}$ alkenyl, $C_{2-6}$ alkynyl, $C_{1-4}$ alkylsulfonyl, $C_{1-4}$ alkylthio, $C_{3-6}$ cycloalkyl, 4-6 membered heterocyclyl, 5-6 membered heteroaryl, aryl, $C_{1-4}$ alkylcarbonyl, aminocarbonyl, $C_{1-4}$ alkylaminocarbonyl, ($C_{1-4}$ alkyl)$_2$aminocarbonyl, 4-6 membered heterocyclylcarbonyl and 5-6 membered heteroaryl-oxy, wherein the $C_{1-4}$ alkyl, $C_{1-4}$ alkoxy, $C_{1-4}$ alkylamino, ($C_{1-4}$ alkyl)$_2$amino, halogenated $C_{1-4}$ alkoxy, $C_{2-6}$ alkenyl, $C_{2-6}$ alkynyl, $C_{1-4}$ alkylsulfonyl, $C_{1-4}$ alkylthio, $C_{3-6}$ cycloalkyl, 4-6 membered heterocyclyl, 5-6 membered heteroaryl, aryl, $C_{1-4}$ alkylcarbonyl, aminocarbonyl, $C_{1-4}$ alkylaminocarbonyl, ($C_{1-4}$ alkyl)$_2$aminocarbonyl, 4-6 membered heterocyclylcarbonyl and 5-6 membered heteroaryl-oxy are unsubstituted or optionally substituted with one or more groups independently selected from hydroxy, amino, carboxyl, cyano, nitro, halogen, $C_{1-4}$ alkyl, $C_{1-4}$ alkoxy, $C_{1-4}$ alkoxy $C_{1-4}$ alkoxy, $C_{1-4}$ alkylamino, ($C_{1-4}$ alkyl)$_2$amino, $C_{1-4}$ alkylcarbonylamino, $C_{1-4}$ alkylsulfonylamino, $C_{1-4}$ alkylcarbonyloxy, $C_{3-6}$ cycloalkylcarbonyloxy, $C_{3-6}$ cycloalkyl, $C_{2-6}$ alkynyl, halogenated $C_{1-4}$ alkyl, $C_{2-6}$ alkenyl, halogenated $C_{1-4}$ alkoxy, 4-6 membered heterocyclyl unsubstituted or optionally substituted with one or more independent substituents, and heteroaryl unsubstituted or optionally substituted with one or more independent substituents;

the substituents for the aforementioned 4-6 membered heterocyclyl optionally substituted with one or more independent substituents and heteroaryl optionally substituted with one or more independent substituents are selected from hydroxy, amino, carboxyl, cyano, nitro, halogen, $C_{1-6}$ alkyl and $C_{1-6}$ alkoxy;

L is a bond;

ring A is 7-12 membered spiro-heterocyclyl, wherein the spiro-heterocyclyl has heteroatoms selected from one of or combinations of two of O, S and N, and contains at least one N, ring A is connected to L via the N atom, the S atom may be optionally oxidized to $S(O)_2$, the C atom may be optionally oxidized to C(O), and the N heteroatom may be optionally oxidized to

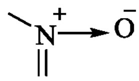;

preferably, ring A is selected from

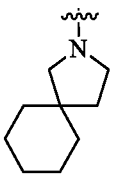, 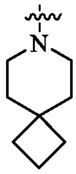, 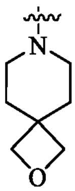, 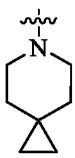, 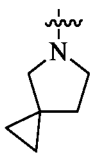, 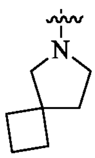

and

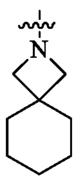;

each $R_1$ is independently selected from hydrogen, deuterium, hydroxy, amino, carboxyl, cyano, nitro, halogen, $C_{1-4}$ alkyl, $C_{1-4}$ alkoxy, $C_{1-4}$ alkylamino, ($C_{1-4}$ alkyl)$_2$amino, halogenated $C_{1-4}$ alkyl, halogenated $C_{1-4}$ alkoxy, $C_{2-6}$ alkenyl, $C_{2-6}$ alkynyl, $C_{1-4}$ alkylsulfonyl, $C_{1-4}$ alkylthio, 3-12 membered cycloalkyl, 3-12 membered cycloalkenyl, 3-12 membered heterocyclyl, aryl and 5-10 membered heteroaryl, wherein the $C_{1-4}$ alkyl, $C_{1-4}$ alkoxy, $C_{1-4}$ alkylamino, ($C_{1-4}$ alkyl)$_2$amino, halogenated $C_{1-4}$ alkyl, halogenated $C_{1-4}$ alkoxy, $C_{2-6}$ alkenyl, $C_{2-6}$ alkynyl, $C_{1-4}$ alkylsulfonyl, $C_{1-4}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-4}$ alkyl, $C_{1-4}$ alkoxy, $C_{1-4}$ alkoxy $C_{1-4}$ alkoxy, $C_{1-4}$ alkylamino, ($C_{1-4}$ alkyl)$_2$amino, $C_{1-4}$ alkylcarbonylamino and $C_{1-4}$ alkylsulfonylamino;
$R_2$ is selected from hydrogen, $C_{1-4}$ alkyl, $C_{2-6}$ alkenyl, $C_{2-6}$ alkynyl and halogenated $C_{1-4}$ alkyl; and
m is 0, 1 or 2;

with the proviso that

(1) when $X_2$ is N, $X_1$ is CH, ring A is

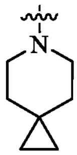

,

and m is 0, $R_3$ is not methylthio; and
(2) when $X_1$ and $X_2$ are each CH, ring A is

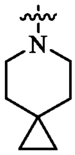

,

and m is 0, $R_3$ is not hydrogen.

**7.** The compound or the pharmaceutically acceptable salt, the isomer, the deuterated compound, the metabolite or the prodrug thereof according to claim 1, having a structure shown as general formula (IV):

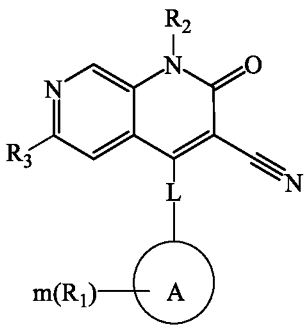

(IV)

wherein,

N at position $X_2$ may be oxidized to

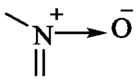

;

$R_3$ is selected from hydrogen, deuterium, amino, carboxyl, cyano, halogen, $C_{1-6}$ alkyl, $C_{1-4}$ alkoxy, $C_{1-4}$ alkylamino, ($C_{1-4}$ alkyl)2amino, $C_{2-6}$ alkenyl, $C_{2-6}$ alkynyl, $C_{1-4}$ alkylsulfonyl, $C_{1-4}$ alkylthio, $C_{3-6}$ cycloalkyl, 4-6 membered nitrogen-containing heterocyclyl, 5-6 membered heteroaryl, aryl, $C_{1-4}$ alkylcarbonyl, $C_{1-4}$ alkylaminocarbonyl, ($C_{1-4}$ alkyl)2aminocarbonyl and aminocarbonyl, wherein the $C_{1-4}$ alkyl, $C_{1-4}$ alkoxy, $C_{1-4}$ alkylamino, ($C_{1-4}$ alkyl)$_2$amino, $C_{2-4}$ alkenyl, $C_{2-4}$ alkynyl, $C_{1-4}$ alkylsulfonyl, $C_{1-4}$ alkylthio, $C_{3-6}$ cycloalkyl, 4-6 membered nitrogen-containing heterocyclyl, 5-6 membered heteroaryl, aryl, $C_{1-4}$ alkylcarbonyl, $C_{1-4}$ alkylaminocarbonyl, ($C_{1-4}$ alkyl)$_2$aminocarbonyl and aminocarbonyl are unsubstituted or optionally substituted with one or more groups independently selected from hydroxy, amino, cyano, halogen, $C_{1-4}$ alkyl, $C_{1-4}$ alkoxy, $C_{1-4}$ alkylamino, ($C_{1-4}$ alkyl)$_2$amino, $C_{1-4}$ alkylcarbonyloxy,

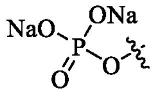,

$C_{3-6}$ cycloalkylcarbonyloxy, $C_{3-6}$ cycloalkyl, and 4-6 membered heterocyclyl unsubstituted or substituted with one or more independent $C_{1-4}$ alkyl groups; L is a bond or -NH-($CH_2$)t-, wherein t is 0, 1, 2 or 3;
ring A is

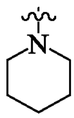 or 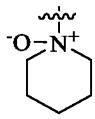;

m is 0, 1 or 2;
each $R_1$ is independently selected from hydrogen, deuterium, hydroxy, amino, carboxyl, cyano, nitro, halogen, $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl, wherein the $C_{1-6}$ alkyl, $C_{1-6}$ alkoxy, $C_{1-6}$ alkylamino, ($C_{1-6}$ alkyl)$_2$amino, halogenated $C_{1-6}$ alkyl, halogenated $C_{1-6}$ alkoxy, $C_{2-8}$ alkenyl, $C_{2-8}$ alkynyl, $C_{1-6}$ alkylsulfonyl, $C_{1-6}$ alkylthio, 3-12 membered cycloalkyl, 3-12 membered cycloalkenyl, 3-12 membered heterocyclyl, aryl and 5-10 membered heteroaryl are unsubstituted or optionally substituted with a group selected from hydroxy, amino, carboxyl, cyano, nitro, halogen, $C_{1-6}$ alkyl, $C_{1-6}$ alkoxy, $C_{1-6}$ alkoxy $C_{1-6}$ alkoxy, $C_{1-6}$ alkylamino, ($C_{1-6}$ alkyl)$_2$amino, $C_{1-6}$ alkylcarbonylamino and $C_{1-6}$ alkylsulfonylamino; and
$R_2$ is selected from hydrogen, $C_{1-6}$ alkyl, $C_{2-8}$ alkenyl, $C_{2-8}$ alkynyl and halogenated $C_{1-6}$ alkyl;

with the proviso that

(i) when

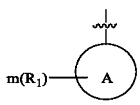 is 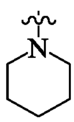, 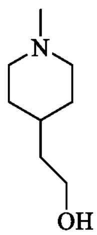, 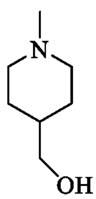, 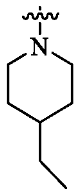, 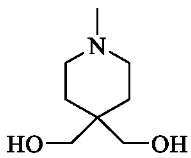 or 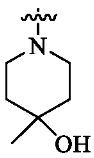,

$R_3$ is not H;
(ii) when

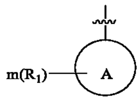 is 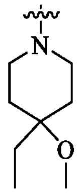,

$R_3$ is not Cl; and
(iii) when

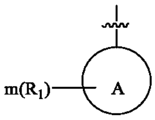 is 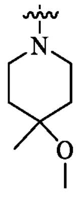,

$R_3$ is not H,

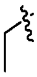, 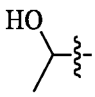, 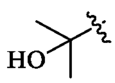, 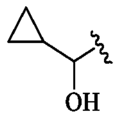,

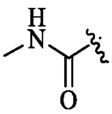, 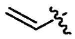, 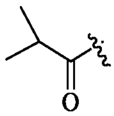, 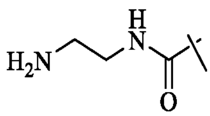, 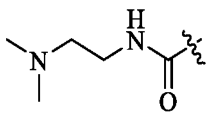, 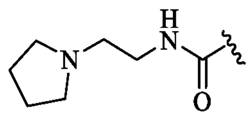,

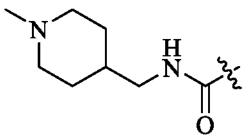, 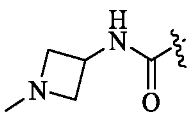, 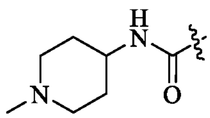, 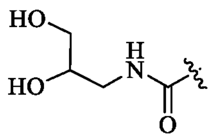, 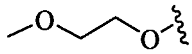,

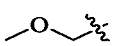, 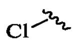, 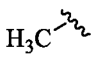, 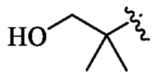 or 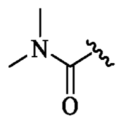;

preferably, when

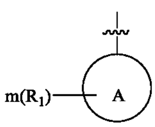 is 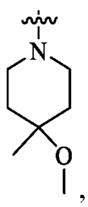,

$R_3$ is selected from isopropyl, cyclopropyl, hydroxymethyl,

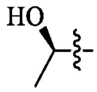, 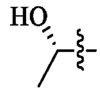,

$C_{1-6}$ alkylcarbonyl, $C_{1-6}$ alkylcarbonyloxy $C_{1-6}$ alkyl, $C_{1-6}$ alkyl substituted with $C_{1-6}$ alkyl; and

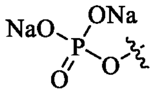,

C3-6 cycloalkylcarbonyloxy $C_{1-6}$ alkyl and deuterated
preferably, ring A is

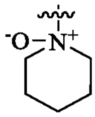.

**8.** The compound or the pharmaceutically acceptable salt, the isomer, the metabolite or the prodrug thereof according to any one of claims 1-7, selected from compounds of the following structures:

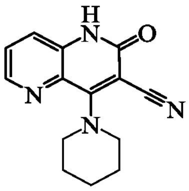, 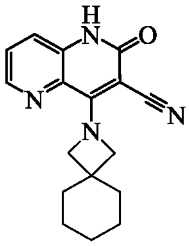, 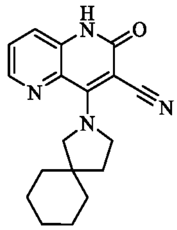, 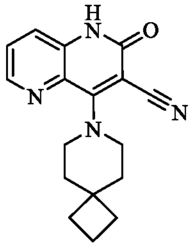, 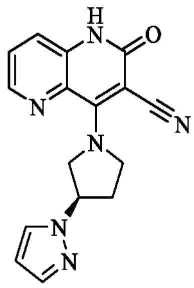,

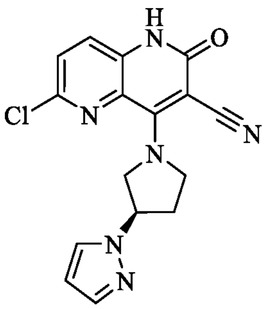, 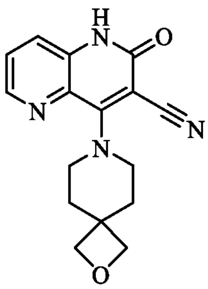, 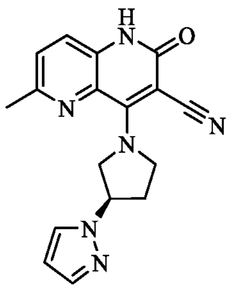, 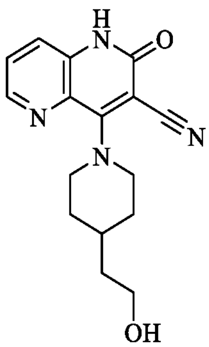,

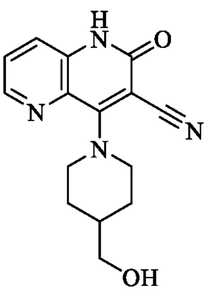, 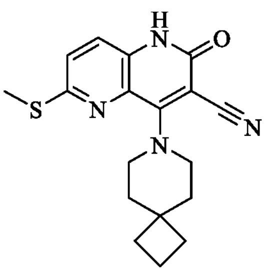, 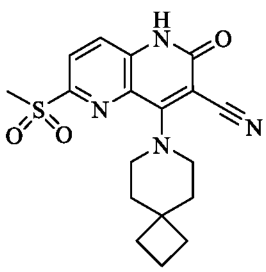, 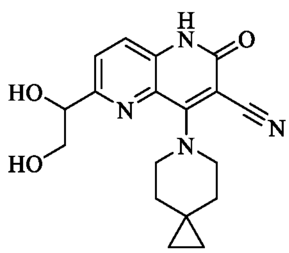,

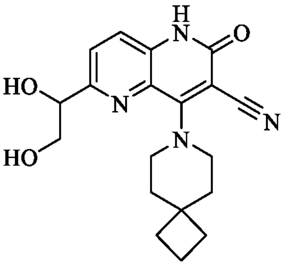 , 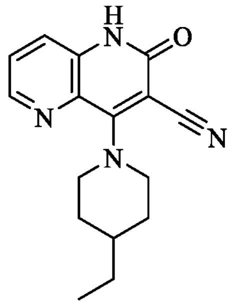 , 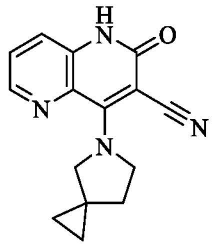 , 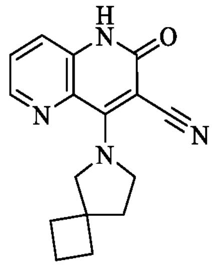 ,

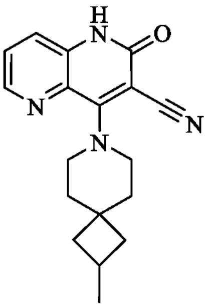 , 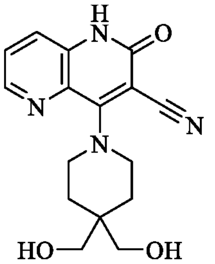 , 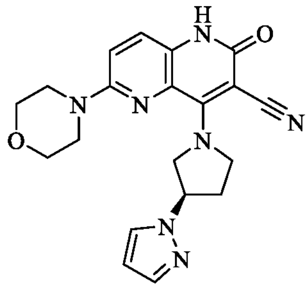 , 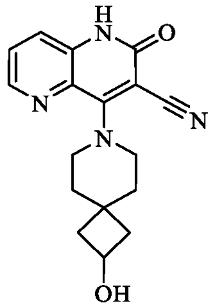 ,

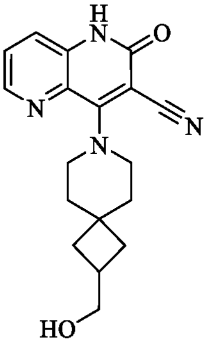 , 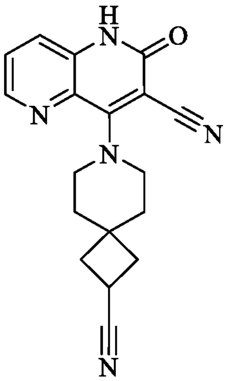 , 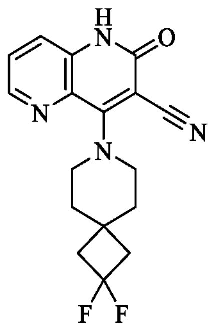 , 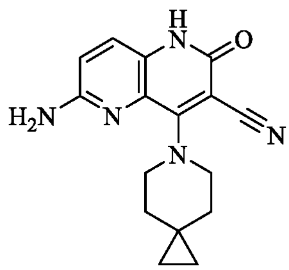 ,

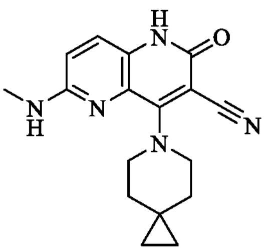 , 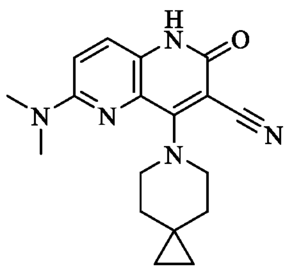 , 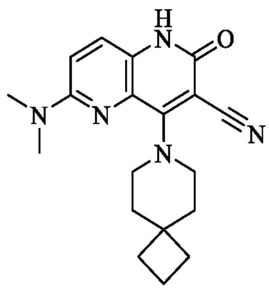 ,

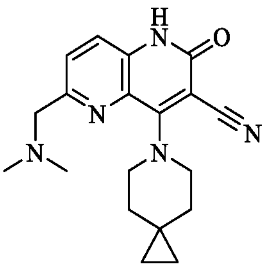 , 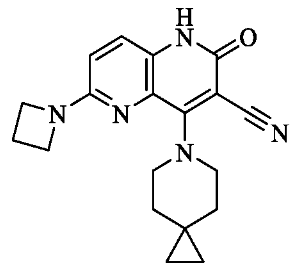 , 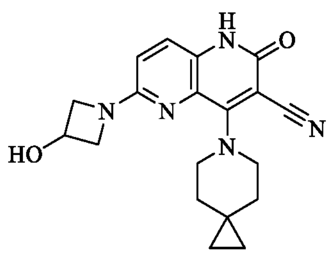 ,

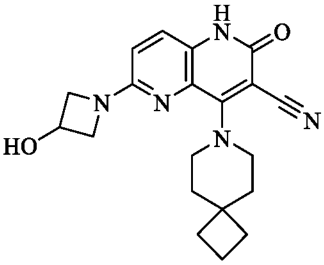 ,

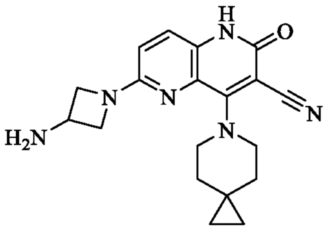 ,

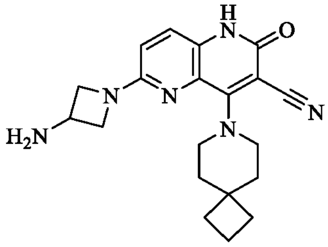 ,

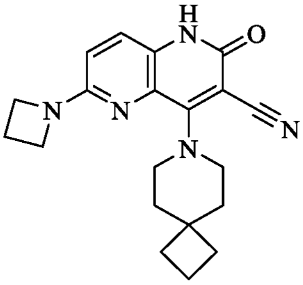 ,

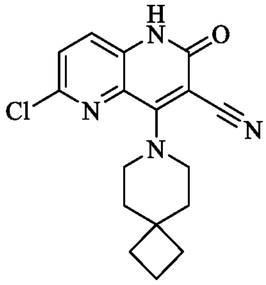 ,

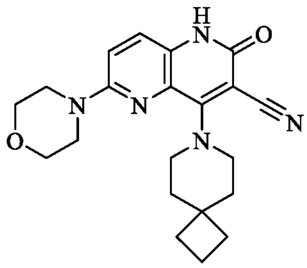 ,

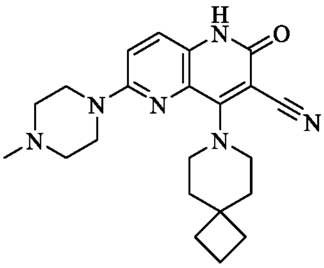 ,

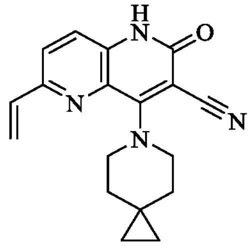 ,

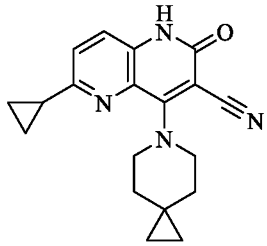 ,

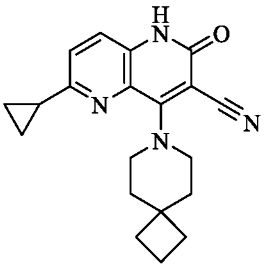 ,

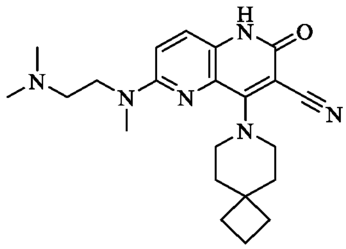 ,

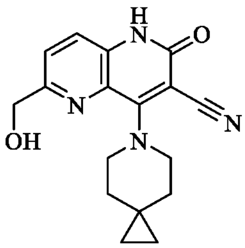 ,

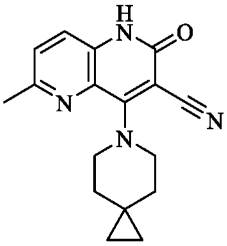 ,

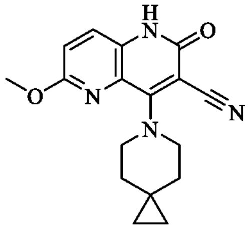 ,

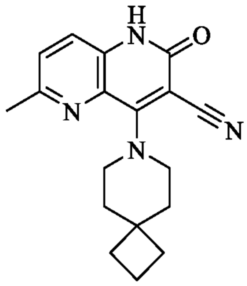 ,

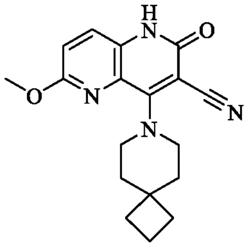 ,

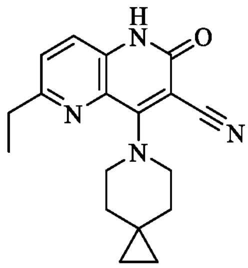,
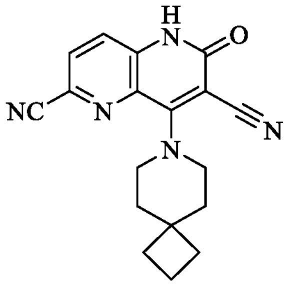,
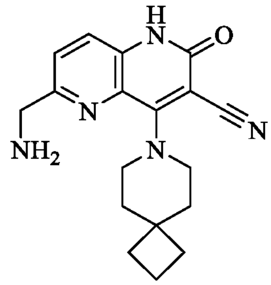,
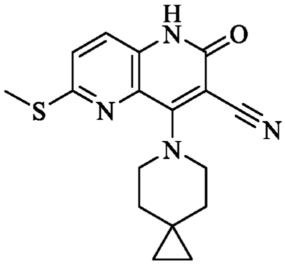,
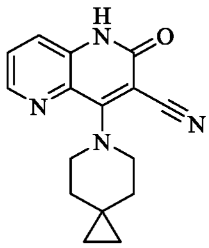,
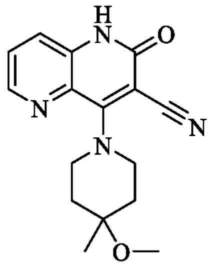,
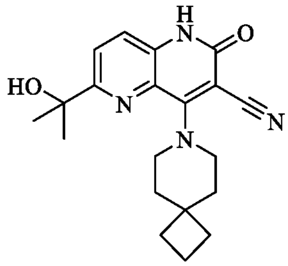,
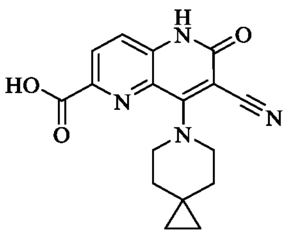,
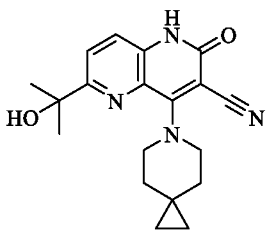,
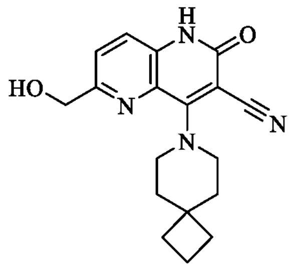,
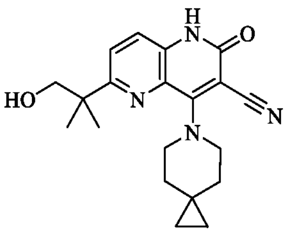,
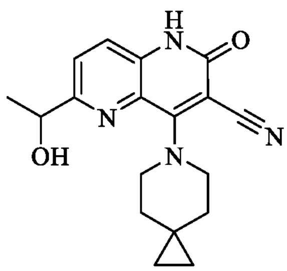,
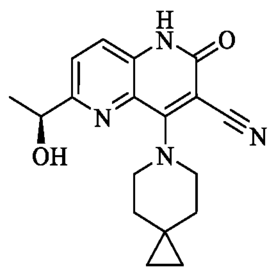,
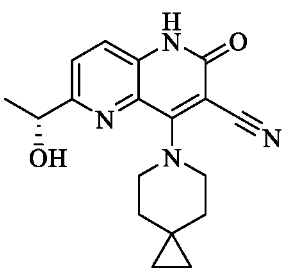,
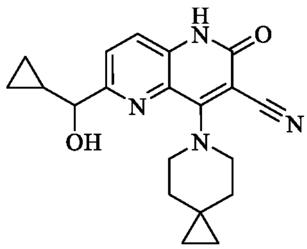,
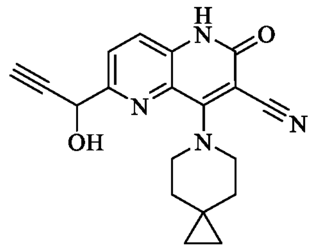,

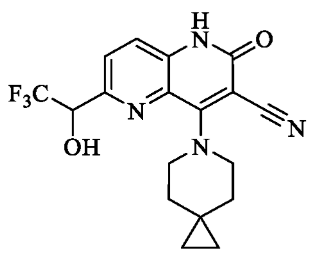,
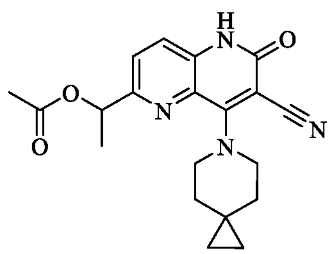,
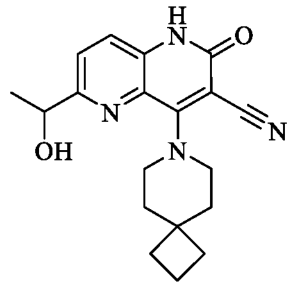,

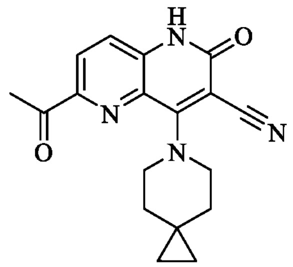,
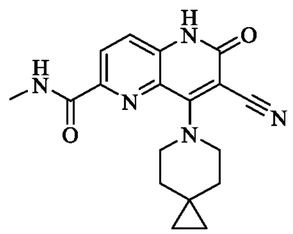,
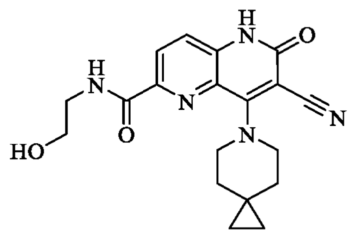,

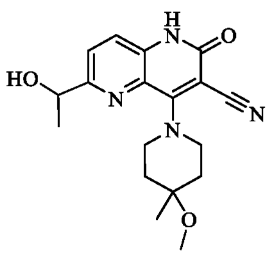,
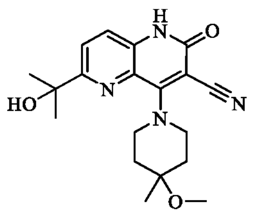,
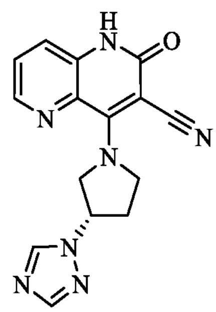,
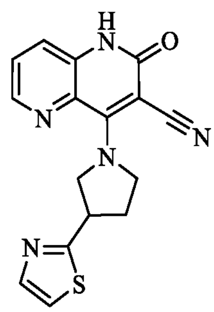,

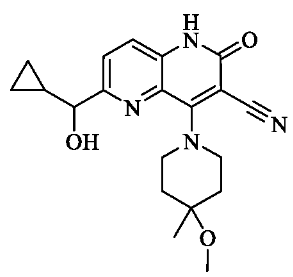,
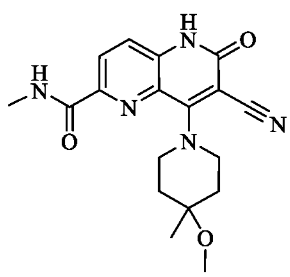,
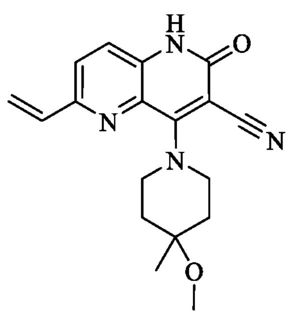,

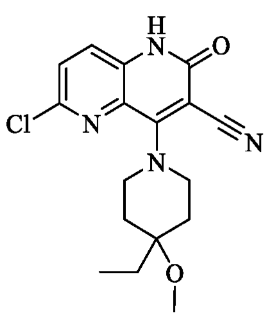,
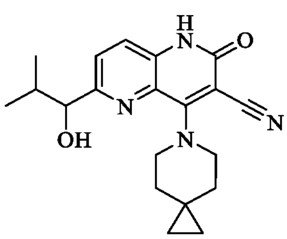,
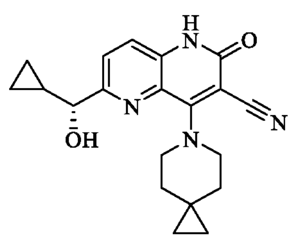,

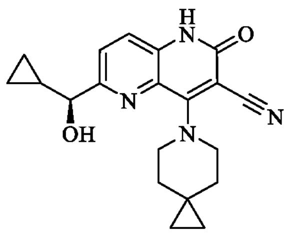 , 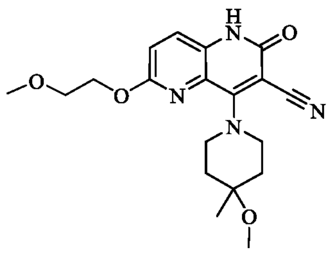 , 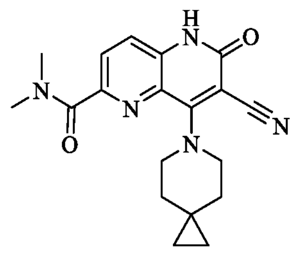 ,

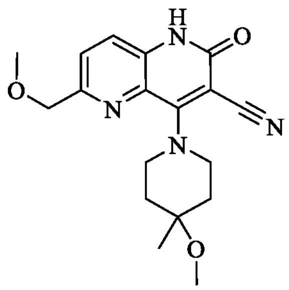 , 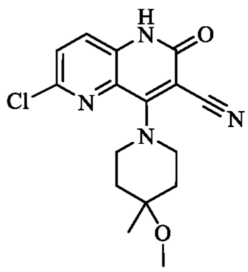 , 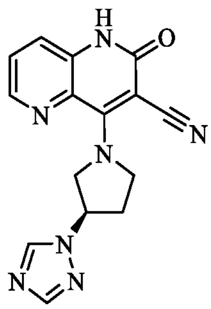 , 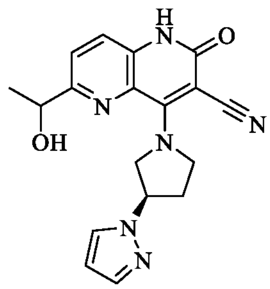 ,

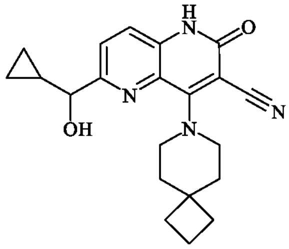 , 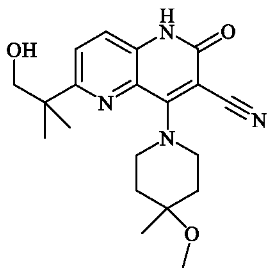 , 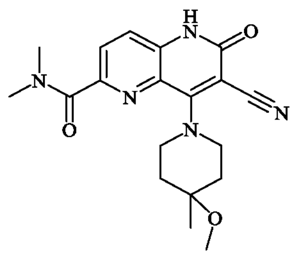 ,

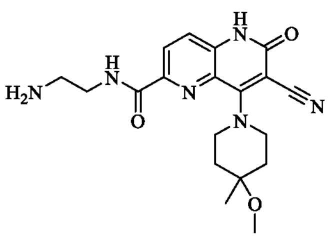 , 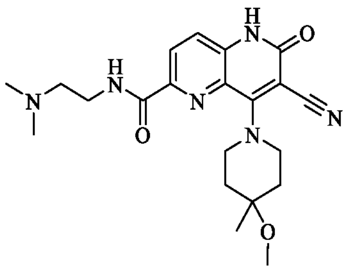 , 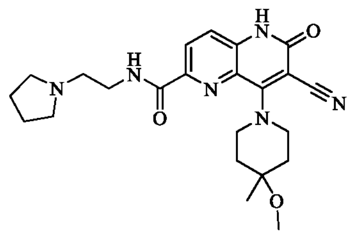 ,

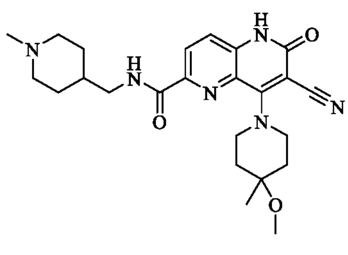 , 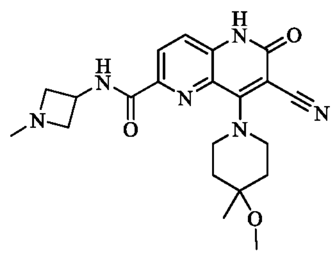 , 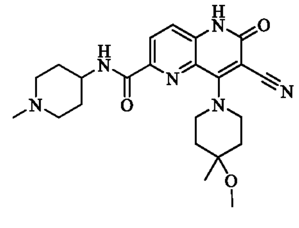 ,

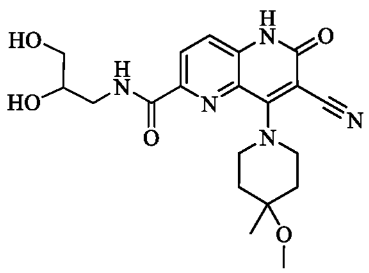 , 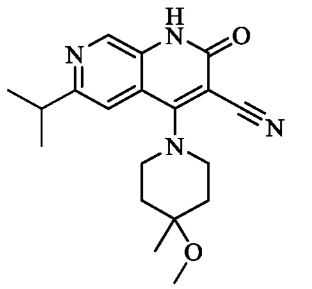 , 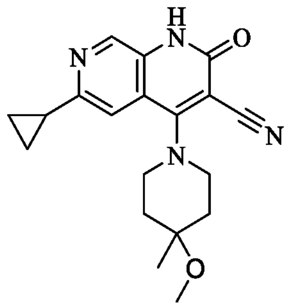 ,

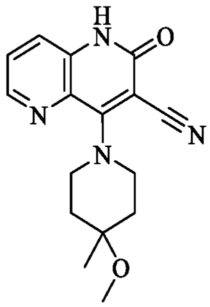 , 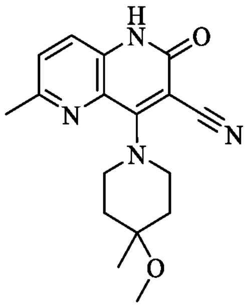 , 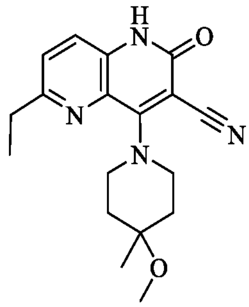 , 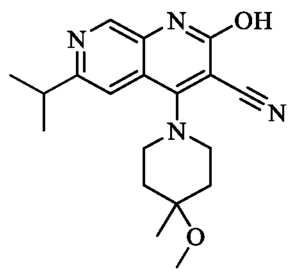 ,

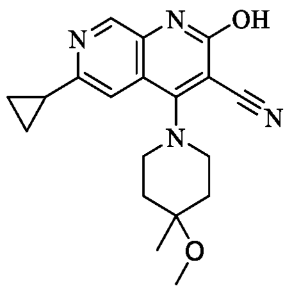 , 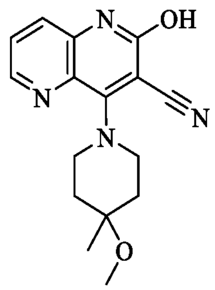 , 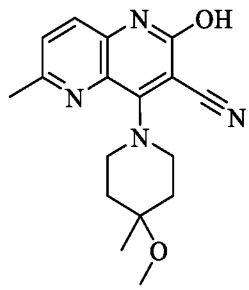 , 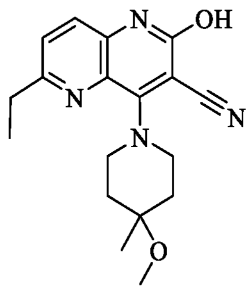 ,

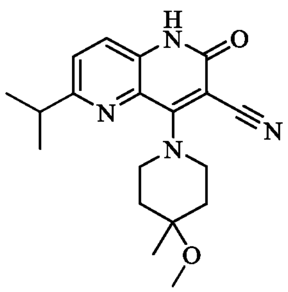 , 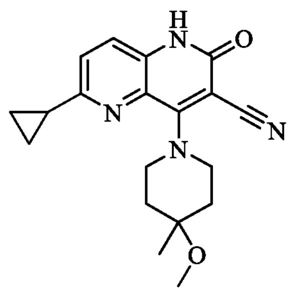 , 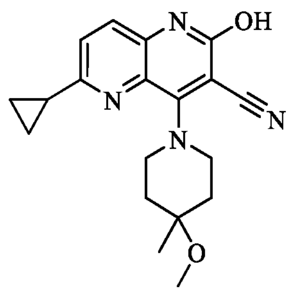 ,

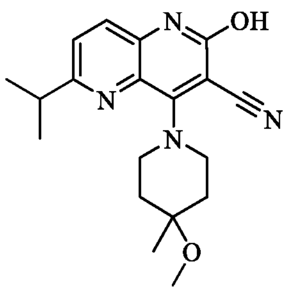 , 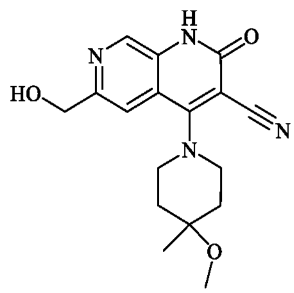 , 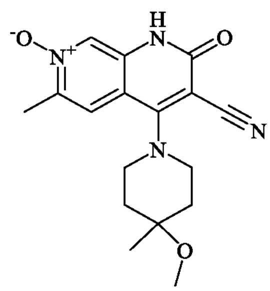 ,

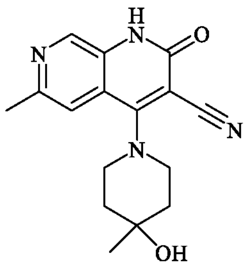 , 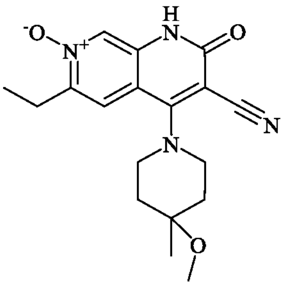 , 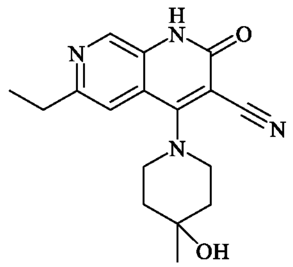 ,

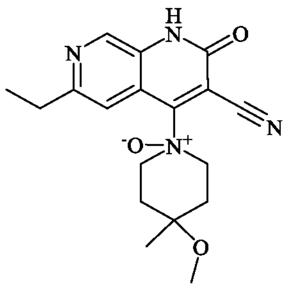 , 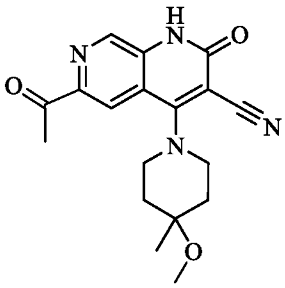 , 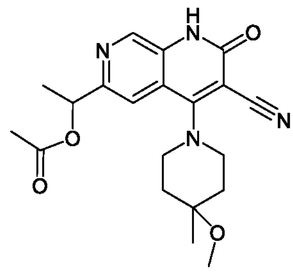 ,

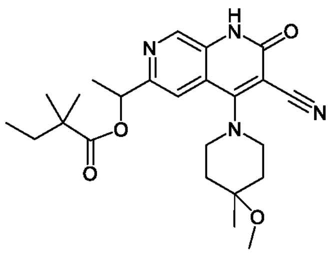 , 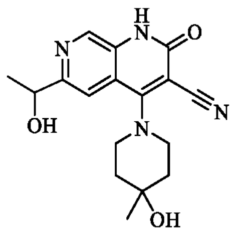 , 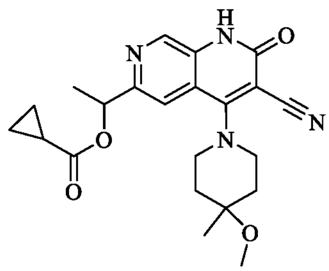 ,

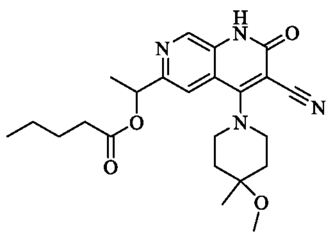 , 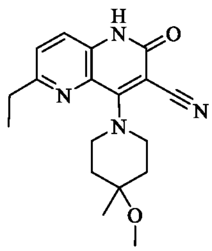 and 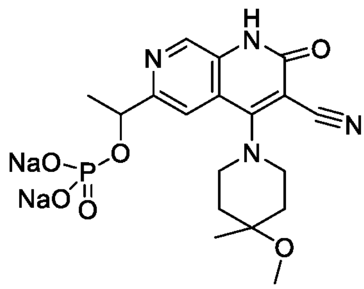 .

**9.** A deuterated compound of the compound of formula (I), wherein the hydrogen atom in the deuterated compound of the compound of formula (I) can be optionally replaced with one or more deuterium atoms.

**10.** The deuterated compound according to claim 9, selected from the following structures:

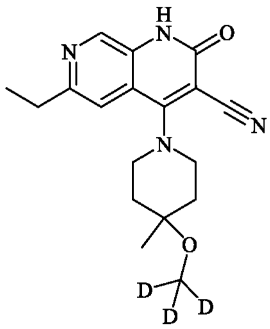,
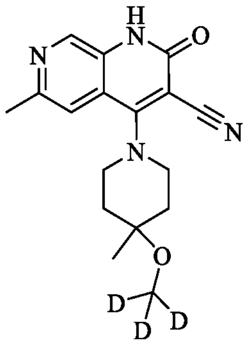,
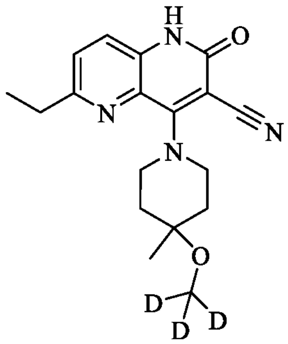,

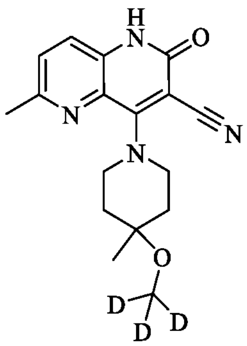,
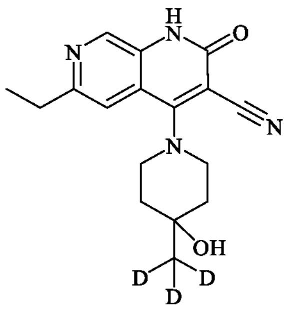,
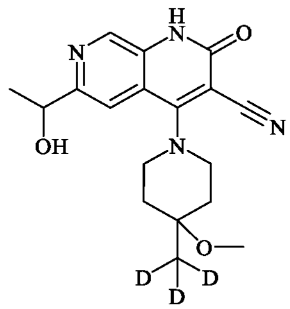,

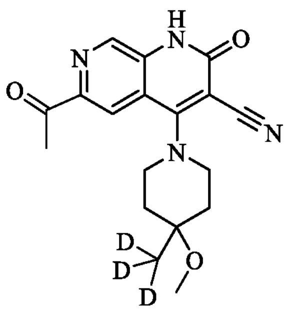,
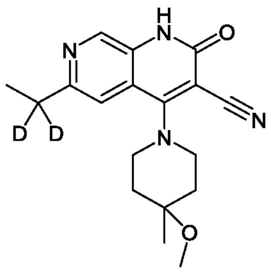,
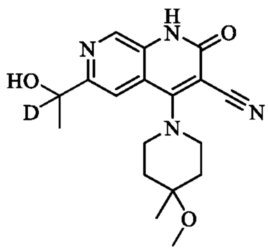,
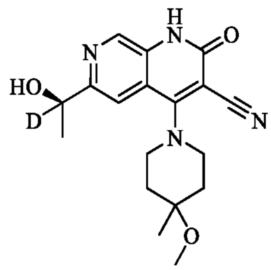

and

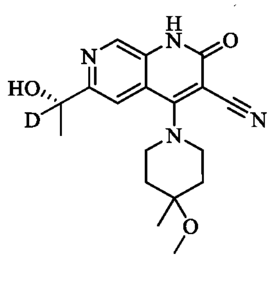.

**11.** The compound or the pharmaceutically acceptable salt, the isomer, the deuterated compound, the metabolite or the prodrug thereof according to claim 1, wherein the compound is selected from the following structures:

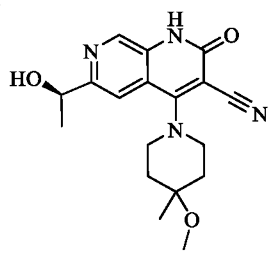 and 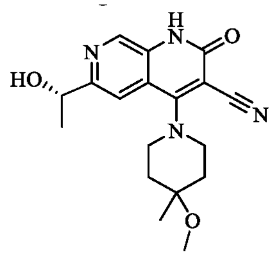 .

12. A pharmaceutical composition comprising the compound or the pharmaceutically acceptable salt, the isomer, the deuterated compound, the metabolite or the prodrug thereof according to any one of claims 1-11, and one or more second therapeutically active agents, wherein the second therapeutically active agent is selected from acetylcholinesterase inhibitors, amyloid-β (or fragments thereof), antibodies of amyloid-β (or fragments thereof), amyloid-lowering or -inhibiting agents, α-adrenoceptor antagonists, β-adrenoceptor blockers, anticholinergics, anticonvulsants, tranquilizers, calcium channel blockers, catechol-*O*-methyltransferase inhibitors, central nervous system stimulators, corticosteroids, dopamine receptor agonists, dopamine receptor antagonists, dopamine reuptake inhibitors, γ-aminobutyric acid receptor agonists, immunomodulators, immunosuppressants, interferons, levodopa, *N*-methyl-D-aspartate receptor antagonists, monoamine oxidase inhibitors, muscarinic receptor agonists, nicotinic receptor agonists, neuroprotective agents, norepinephrine reuptake inhibitors, other PDE9 inhibitors, other PDE inhibitors, β-secretase inhibitors, γ-secretase inhibitors, serotonin (5-hydroxytryptamine)1A (5-$HT_{1A}$) receptor antagonists, serotonin (5-hydroxytryptamine)6 (5-HT6) receptor antagonists, serotonin (5-HT) reuptake inhibitors and trophic factors.

13. A pharmaceutical formulation comprising the compound or the pharmaceutically acceptable salt, the isomer, the deuterated compound, the metabolite or the prodrug thereof according to any one of claims 1-11, wherein preferably, the pharmaceutical formulation comprises one or more pharmaceutical carriers.

14. Use of the compound or the pharmaceutically acceptable salt, the isomer, the deuterated compound, the metabolite or the prodrug thereof according to any one of claims 1-11, the pharmaceutical composition according to claim 12 or the pharmaceutical formulation according to claim 13 in the manufacture of a medicament for treating or preventing a PDE9-mediated related disease.

## INTERNATIONAL SEARCH REPORT

International application No. **PCT/CN2020/073128**

### A. CLASSIFICATION OF SUBJECT MATTER

C07D 487/04(2006.01)i; A61K 31/4375(2006.01)i; A61P 25/18(2006.01)i; A61P 25/28(2006.01)i

According to International Patent Classification (IPC) or to both national classification and IPC

### B. FIELDS SEARCHED

Minimum documentation searched (classification system followed by classification symbols)

C07D487/04,A61K/-,A61P25/-

Documentation searched other than minimum documentation to the extent that such documents are included in the fields searched

Electronic data base consulted during the international search (name of data base and, where practicable, search terms used)

CNABS; VEN; CNKI; REG; CAPLUS; PDE9, phosphodiesterase 9, neuro+, 根据式(I)进行了结构式检索, structural search according to formula (I)

### C. DOCUMENTS CONSIDERED TO BE RELEVANT

| Category* | Citation of document, with indication, where appropriate, of the relevant passages | Relevant to claim No. |
|---|---|---|
| PX | WO 2019062733 A1 (TRANSTHERA BIOSCIENCES CO., LTD.) 04 April 2019 (2019-04-04)<br>claims 1-15, description, pages 12-27, compounds, in particular compound 12 | 1-14 |
| PX | WO 2020006018 A1 (BRISTOL-MYERS SQUIBB COMPANY) 02 January 2020 (2020-01-02)<br>description, pages 102-139, intermediates 49, 62-64, 99, and 107 | 1-4 |
| X | WO 2017019723 A1 (MERCK SHARP & DOHME CORP.) 02 February 2017 (2017-02-02)<br>claims 1-12, description, pages 28 and 29, table 1, embodiments I-7, I-8, I-9, I-11, and I-12 | 1-14 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 March 2020** | **03 April 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/073128**

| Patent document cited in search report | Publication date (day/month/year) | Patent family member(s) | Publication date (day/month/year) |
| --- | --- | --- | --- |
| WO 2019062733 A1 | 04 April 2019 | TW 201915001 A | 16 April 2019 |
| | | CN 109575016 A | 05 April 2019 |
| | | TW I677499 B | 21 November 2019 |
| WO 2020006018 A1 | 02 January 2020 | None | |
| WO 2017019723 A1 | 02 February 2017 | US 10376504 B2 | 13 August 2019 |
| | | US 2018214439 A1 | 02 August 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008139293 A1
- WO 2009121919 A1
- WO 2017019723 A1
- WO 2017019726 A1
- WO 2017019724 A1